# EUROPÄISCHE PATENTANMELDUNG

(11) **EP 0 337 229 A2**
(43) Veröffentlichungstag der Anmeldung: **18.10.1989**
(21) Anmeldenummer: 89105793.7
(22) Anmeldetag: 03.04.1989
(51) Int. Cl.: C07D 405/04, C07D 409/04, C07D 411/04, A01N 43/54, A01N 43/40

(54) **Substituierte Pyri(mi)dine**

(30) Priorität: 15.04.1988 DE 3812521
(71) Anmelder: BAYER AG, 51368 Leverkusen (DE)
(72) Erfinder: Elbe, Hans-Ludwig, Dr., D 5600 Wuppertal 11 (DE); Brandes, Wilhelm, Dr., D 5653 Leichlingen (DE); Dutzmann, Stefan, Dr., D 4000 Düsseldorf 13 (DE); Hänssler, Gerd, Dr., D 5090 Leverkusen 3 (DE)

(57) **Zusammenfassung**

Neue substituierte Pyri(mi)dine der Formel (I) 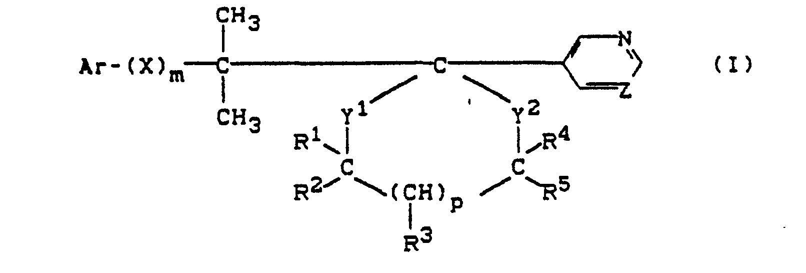 in der
Ar, X, Z, R¹-R⁵, Y¹, Y², m und p die in der Beschreibung angegebenen Bedeutungen haben, und ihre Verwendung zur Bekämpfung von Schädlingen.

Die neuen substituierten Pyri(mi)dine sind durch die Formel (I) allgemein definiert und können nach Analogieverfahren hergestellt werden, z.B. indem man geeignete Pyri(mi)dylketone mit geeigneten Di(thio)-alkoholen umsetzt.

## Beschreibung

Die Erfindung betrifft neue substituierte Pyri(mi)dine, ein Verfahren zu ihrer Herstellung sowie ihre Verwendung in Schädlingsbekämpfungsmitteln.

Es ist bekannt, daß bestimmte substituierte Pyri(mi)dine, wie beispielsweise die Verbindung 1-(4-Chlor-2-methylphenoxy)-3,3-dimethyl-2-(3-pyridyl)-butan-2-ol oder die Verbindung 1-(3,4-Dichlorphenoxy)-3,3-dimethyl-2-(5-pyrimidyl)-butan-2-ol oder die Verbindungen 3,3-Dimethyl-1-(2-methylphenoxy)-2-(3-pyridyl)-butan-2-ol oder die Verbindung 1-(4-Chlorphenoxy)-3,3-dimethyl-2-(5-pyrimidyl)-butan-2-ol oder die Verbindung 3,3-Dimethyl-1-(4-methylphenoxy)-2-(5-pyrimidyl)-butan-2-ol oder deren Säureadditionssalze, wie beispielsweise die Verbindung 2-(4-Chlorphenoxy)-1-(2,4-dichlorphenoxy)-1-(3-pyridyl)-ethanol Naphthalindisulfonat fungizide Eigenschaften besitzen (vergl. z.B. EP 1399).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Pyri(mi)dine der allgemeinen Formel (I) 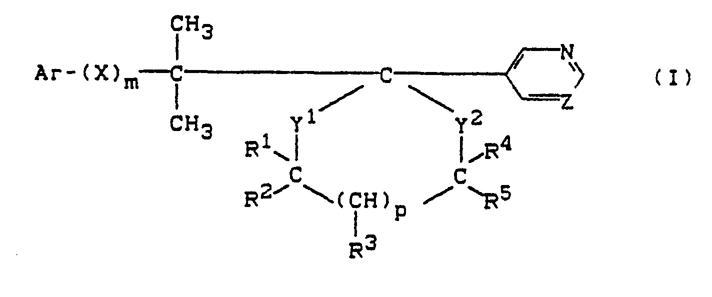 in welcher
Ar für gegebenenfalls substituiertes Aryl steht,
X für Sauerstoff, Schwefel, Sulfinyl, Sulfonyl oder für eine der Gruppen -CHε-; -O-CH₂-; -CH₂-0-; -0-CH₂-CH₂-; -S(O)ₙ CH₂-; -CH₂-S(O)ₙ- oder -S(O)ₙ-CH₂-CH₂- steht,
Y' und Y² unabhängig voneinander jeweils für Sauerstoff oder Schwefel stehen,
Z für Stickstoff oder eine CH-Gruppe steht,
R', R², R³ und R⁴ unabhängig voneinander jeweils für Wasserstoff oder Alkyl stehen oder
R' und R⁴ gemeinsam für einen zweifach verknüpften Alkandiylrest stehen,
R⁵ für Wasserstoff oder für jeweils gegebenenfalls substituiertes Alkyl oder Aryl steht,
m für eine Zahl 0 oder 1 steht,
n jeweils für eine Zahl 0, 1 oder 2 steht und
p für eine Zahl 0 oder 1 steht,
sowie deren Säureadditionssalze und Metallsalzkomplexe gefunden.

Die Verbindungen der Formel (I) können gegebenenfalls als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Pyri(mi)dine der allgemeinen Formel (I) 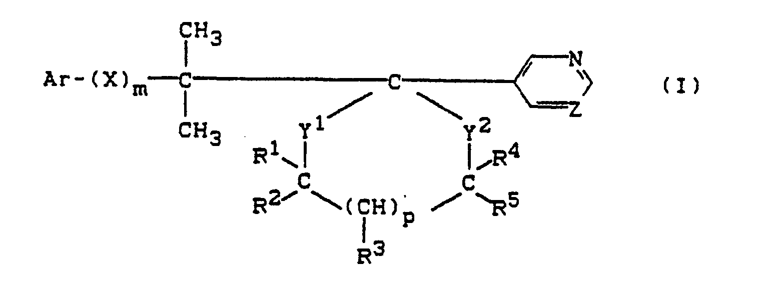 in welcher
Ar für gegebenenfalls substituiertes Aryl steht,
X für Sauerstoff, Schwefel, Sulfinyl, Sulfonyl oder für eine der Gruppen -CH₂-; -O-CH₂-; -CH₂-0-; -O-CH₂-CH₂-; -S(O)ₙ-CH₂-; -CH₂-S(O)ₙ₋ oder -S(O)ₙ-CH₂-CH₂- steht,
Y' und Y² unabhängig voneinander jeweils für Sauerstoff oder Schwefel stehen,
Z für Stickstoff oder eine CH-Gruppe steht,
R', R², R³ und R⁴ unabhängig voneinander jeweils für Wasserstoff oder Alkyl stehen oder
R' und R⁴ gemeinsam für einen zweifach verknüpften Alkandiylrest stehen,
R⁵ für Wasserstoff oder für jeweils gegebenenfalls substituiertes Alkyl oder Aryl steht,
m für eine Zahl 0 oder 1 steht,
n jeweils für eine Zahl 0, 1 oder 2 steht und
p für eine Zahl 0 oder 1 steht,
   sowie deren Säureadditionssalze und Metallsalzkomplexe erhält, wenn man Pyri(mi)dylketone der Formel (11) 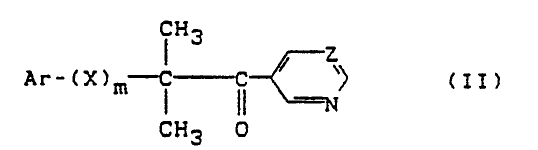 in welcher
   Ar, X, Z und m die oben angegebene Bedeutung haben,
   mit Di(thio)alkoholen der Formel (III) 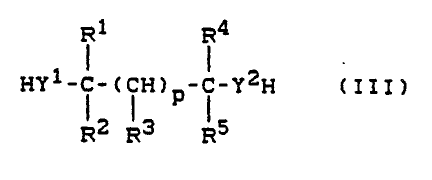 in welcher
   Y¹, _{Y}2, _{R}¹, _{R}2, R³, R⁴, R⁵ und p die oben angegebene Bedeutung haben,
   gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

Weiterhin wurde gefunden, daß die neuen substituierten Pyri(mi)dine der allgemeinen Formel (I) eine gute Wirksamkeit gegen Schädlinge besitzen.

Überraschenderweise zeigen die erfindungsgemäßen substituierten Pyri(mi)dine der Formel (I) eine erheblich bessere fungizide Wirksamkeit als die aus dem Stand der Technik bekannten substituierten Pyri-(mi)dine, wie beispielsweise die Verbindung 1-(4-Chlor-2-methylphenoxy)-3,3-dimethyl-2-(3-pyridyl)-butan-2-ol oder die Verbindung 1-(3,4-Dichlorphenoxy)-3,3-dimethyl-2-(5-pyrimidyl)-butan-2-ol oder die Verbindung 3,3-Dimethyl-1-(2-methylphenoxy)-2-(3-pyridyl)-butan-2-ol oder die Verbindung 1-(4-Chlorphenoxy)-3,3-dimethyl-2-(5-pyrimidyl)-butan-2-ol oder die Verbindung 3,3-Dimethyl-1-(4-methylphenoxy)-2-(5-pyrimidyl)-butan-2-ol oder deren Säureadditionssalze, wie beispielsweise die Verbindung 2-(4-Chlorphenoxy)-1-(2,4-dichlorphenyl)-1-(3-pyridyl)-ethanol Naphthalindisulfonat, welche chemisch und wirkungsmäßig naheliegende Verbindungen sind.

Die erfindungsgemäßen substituierten Pyri(mi)dine sind durch die Formel (I) allgemein definiert. Bevorzugt sind Verbindungen der Formel (I),
bei welchen

Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, geradkettiges oder verzweigtes, zweifach verknüpftes Alkandiyl mit 1 bis 10 Kohlenstoffatomen, 'Cycloalkyl mit 3 bis 7 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen mit 1 bis 6 Kohlenstoffatomen oder jeweils gegebenenfalls einfach bis mehrfach, gleich oder verschieden durch Halogen substituiertes Phenyl oder Phenoxy,
oder für gegebenenfalls einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;

X für Sauerstoff, Schwefel, Sulfinyl, Sulfonyl oder für eine der Gruppen -CH₂-; -O-CH₂-; -CH₂-0-; -O-CH₂-CH₂-; -S(O)ₙ-CH₂-; -CH2-S(O)R oder -S(O)ₙ-CH₂-CH₂- steht,
wobei
n jeweils für eine Zahl 0, 1 oder 2 steht,
Y¹ und Y² unabhängig voneinander jeweils für Sauerstoff oder Schwefel stehen,
Z für Stickstoff oder eine CH-Gruppe steht,
R', R², R³ und R⁴ unabhängig voneinander jeweils für Wasserstoff oder für geradkettiges oder verzweigtes Alkyl mit 1 bis 6 Kohlenstoffatomen stehen oder
R' und R⁴ gemeinsam für einen geradkettigen oder verzweigten Alkandiylrest mit 2 bis 10 Kohlenstoffatomen stehen,
R⁵ für Wasserstoff, für jeweils geradkettiges oder verzweigtes Alkyl, Hydroxyalkyl, Alkoxyalkyl, Alkylcarbonyloxyalkyl, Alkylsulfonyloxyalkyl, Dialkylaminoalkyl oder N,N-Dialkylcarbamoylalkyl mit jeweils 1 bis 4 Kohlenstoffatomen in den einzelnen Alkylteilen steht; außerdem für jeweils im Arylteil gegebenenfalls einfach oder mehrfach, gleich oder verschieden substituiertes Aralkyloxyalkyl, Aryloxyalkyl, Aralkylcarbonyloxyalkyl, Arylcarbonyloxyalkyl, Arylsulfonyloxyalkyl oder Aralkyl mit jeweils 1 bis 4 Kohlenstoffatomen in den einzelnen Alkylteilen und jeweils 6 bis 10 Kohlenstoffatomen im Arylteil steht oder für gegebenenfalls einfach oder mehrfach, gleich oder verschieden substituiertes Aryl mit 6 bis 10 Kohlenstoffatomen steht, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino, Alkanoylamino oder
N-Alkyl-alkanoylamino mit jeweils 1 bis 4 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 4 Kohlenstoffatomen substituiertes Phenyl und gegebenenfalls durch jeweils geradkettiges
oder verzweigtes Alkyl oder Alkanoyl mit jeweils 1 bis 4 Kohlenstoffatomen substituiertes 1,4-Piperazinyl, m für eine Zahl 0 oder 1 steht und
p für eine Zahl 0 oder 1 steht.

Besonders bevorzugt sind substituierte Pyri(mi)dine der allgemeinen Formel (I),
bei welchen
Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s-oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethylbutan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, . Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoxyiminomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils gegebenenfalls ein- bis dreifach durch Fluor oder Chlor substituiertes Phenyl oder Phenoxy;
oder für gegebenenfalls ein- bis dreifach, gleich oder verschieden substituiertes ci-Naphthyl oder ß-Naphthyl steht, wobei als Substituenten jeweils infrage kommen: Fluor, Chlor, Brom, Cyano, Methyl, Ethyl, n- oder i-Propyl;
X für Sauerstoff, Schwefel, Sulfinyl, Sulfonyl oder für eine der Gruppen -CH2₋; -O-CH₂-; -CH₂0-; -O-CH₂-CH₂-; -S(O)ₙ-CH₂-; -CH₂-S(O)ₙ- oder -S(O)ₙ-CH₂-CH₂- steht,
   wobei
n jeweils für eine Zahl 0, 1 oder 2 steht,
Y' und Y² unabhängig voneinander jeweils für Sauerstoff oder Schwefel stehen,
Z für Stickstoff oder eine CH-Gruppe steht
R¹, R², R³ und R⁴ unabhängig voneinander jeweils für Wasserstoff, Methyl oder Ethyl stehen oder
R¹ und R⁴ gemeinsam für einen zweifach verknüpften Butan-1,4-diylrest stehen,
R⁵ für Wasserstoff, Methyl, Ethyl, n- oder i-Propyl, n-, i-, s- oder t-Butyl, für Hydroxymethyl, Hydroxyethyl, Methoxymethyl, Methoxyethyl, Ethoxymethyl, Ethoxyethyl, n- oder i-Propoxymethyl, Methylcarbonyloxymethyl, Ethylcarbonyloxymethyl, Methylsulfonyloxymethyl, Dimethylaminomethyl, Diethylaminomethyl, Dimethylaminoethyl, Diethylaminoethyl, Dimethylaminocarbamoylmethyl, Diethylaminocarbamoylmethyl und außerdem für jeweils gegebenenfalls im Phenylteil ein- bis dreifach, gleich oder verschieden substituiertes Benzyloxymethyl, Phenoxymethyl, Phenoxyethyl, Benzylcarbonyloxymethyl, Benzyloxymethyl, Phenylsulfonyloxymethyl, Benzyl, Phenylethyl oder Phenyl steht, wobei als Phenylsubstituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n-oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl, Dimethylamino, Diethylamino, Acetamido, N-Methylacetamido, gegebenenfalls ein- bis dreifach, gleich oder verschieden durch Fluor, Chlor, Brom, Methyl und/oder Ethyl substituiertes Phenyl oder gegebenenfalls durch Methyl oder Acetyl substituiertes 1,4-Piperazinyl,
m für eine Zahl 0 oder 1 steht und
p für eine Zahl 0 oder 1 steht.

Bevorzugte erfindungsgemäße Verbindungen sind auch Additionsprodukte aus Säuren und denjenigen substituierten Pyri(mi)dinen der Formel (I), in denen die Substituenten und Indices Ar, X, Y', Y², Z, R¹, R², R³, R⁴, R⁵, m und p die Bedeutungen haben, die bereits vorzugsweise für diese Substituenten und Indices genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Pyri(mi)dinen der Formel (I), in denen die Substituenten und Indices Ar, X, Y', Y², Z, R', R², R³, R⁴, R^{S}, m und p die Bedeutungen haben, die bereits vorzugsweise für diese Substituenten und Indices genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Ganz besonders bevorzugt sind Verbindungen der Formel (I),
bei welchen
Ar für gegebenenfalls einfach oder zweifach, gleich oder verschieden substituiertes Phenyl steht, wobei als Substituenten infrage kommen: Fluor, Chlor, Brom, Cyano, Nitro, Methyl, Ethyl, i-Propyl, t-Butyl, Methoxy, Methylthio, Trifluormethyl, Difluormethyl, Trifluormethoxy, Difluormethoxy, Trifluormethylthio oder jeweils gegebenenfalls einfach oder zweifach durch Fluor oder Chlor substituiertes Phenyl oder Phenoxy; und außerdem für a-Naphthyl oder β-Naphthyl steht,
X für Sauerstoff, Schwefel oder für eine der Gruppen -CH₂-, -0-CH₂, oder -S-CH₂- steht,
Y' und Y² unabhängig voneinander jeweils für Sauerstoff oder Schwefel stehen,
Z für Stickstoff oder für eine CH-Gruppe steht,
R', R², R³ und R⁴ jeweils für Wasserstoff stehen oder
R¹ und R⁴ gemeinsam für einen zweifach verknüpften Butan-1,4-diylrest stehen,
R⁵ für Wasserstoff, Methyl, Ethyl, n- oder i-Propyl, n-, i-, s- oder t-Butyl, Hydroxymethyl, Methoxymethyl oder für jeweils gegebenenfalls einfach oder zweifach, gleich oder verschieden durch Fluor, Chlor oder
Methyl substituiertes Phenoxymethyl, Benzyloxymethyl, Benzyl oder Phenyl steht,
m für eine Zahl 0 oder 1 steht und
p für eine Zahl 0 oder 1 steht.

Im einzelnen seien außer den bei den Herstellungsbeispielen genannten Verbindungen die folgenden substituierten Pyri(mi)dine der allgemeinen Formel (I) genannt:

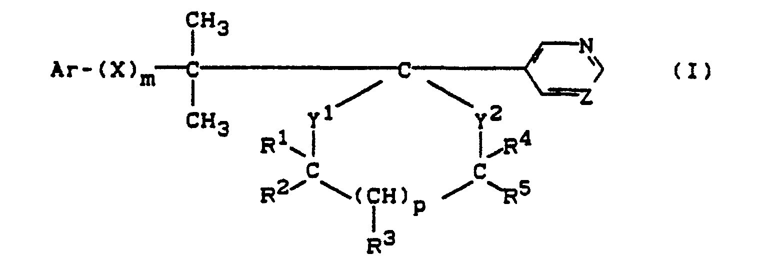

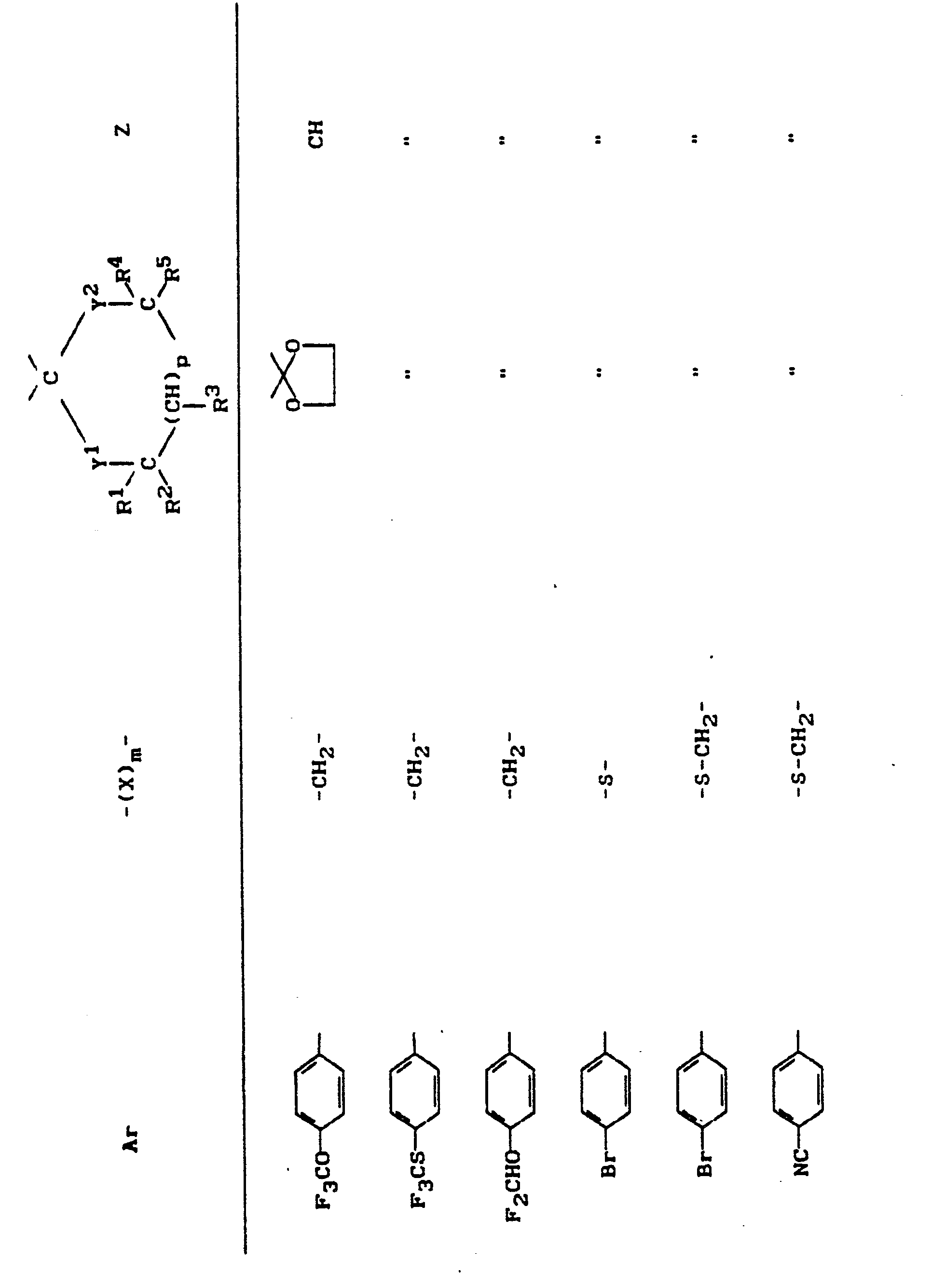

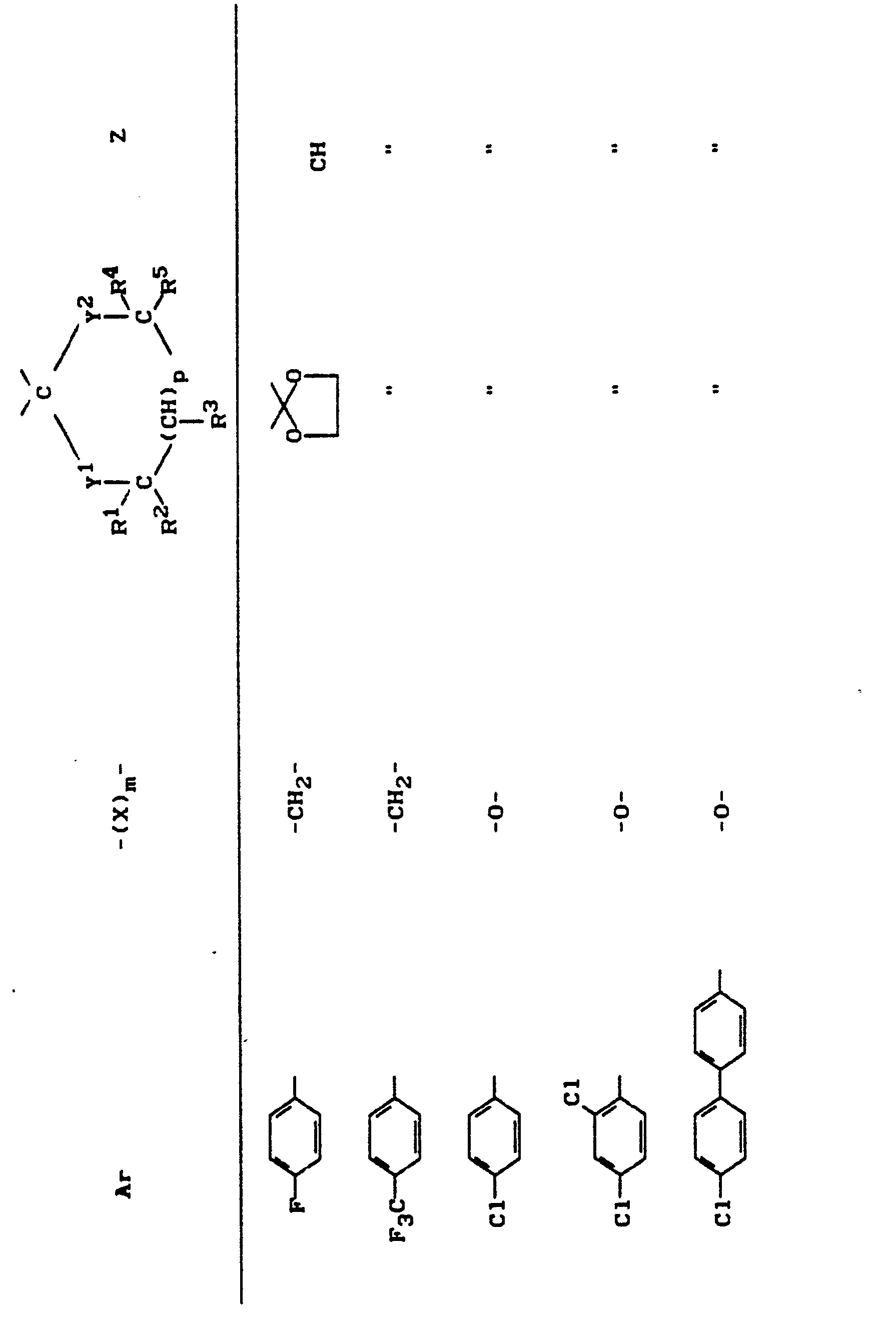

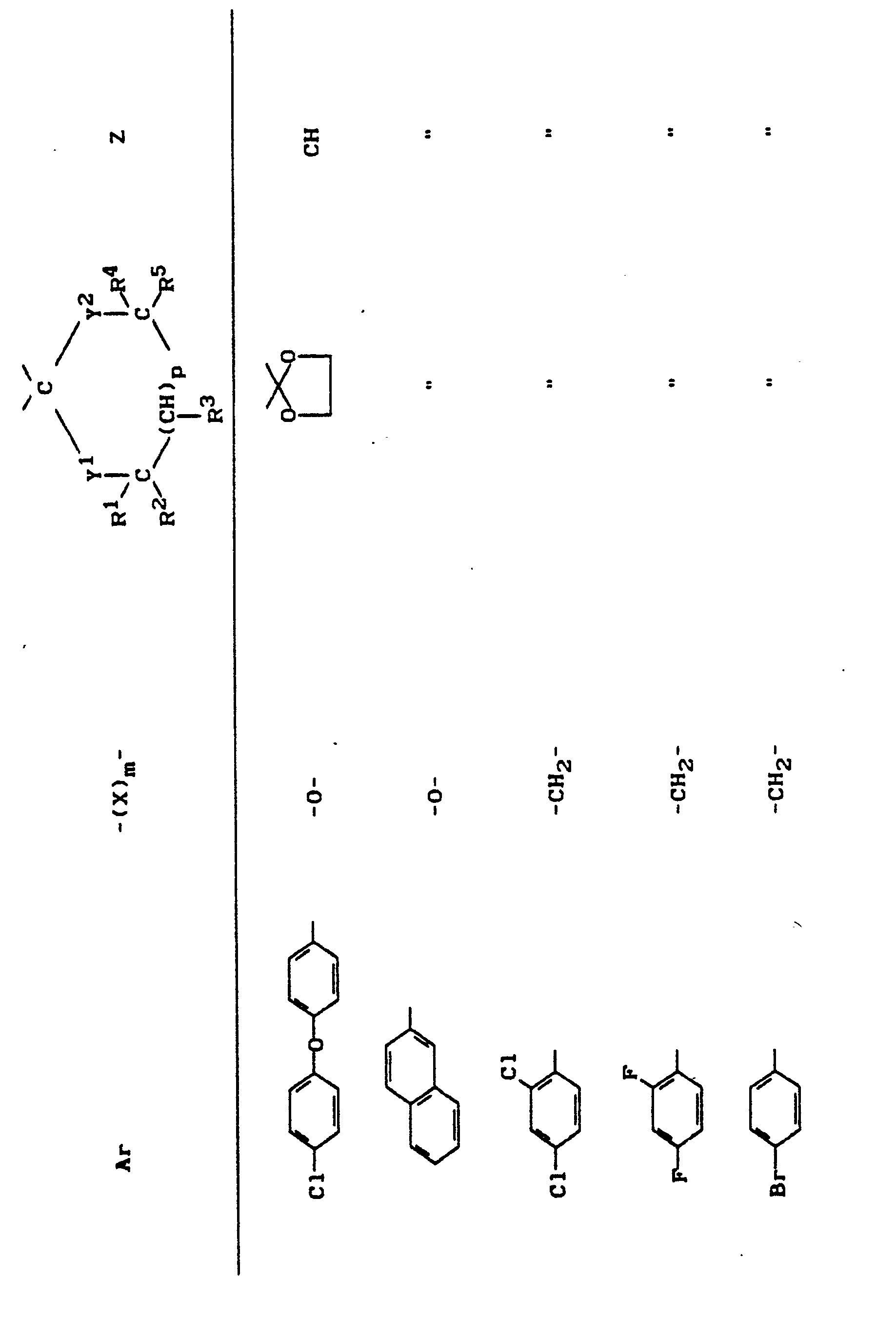

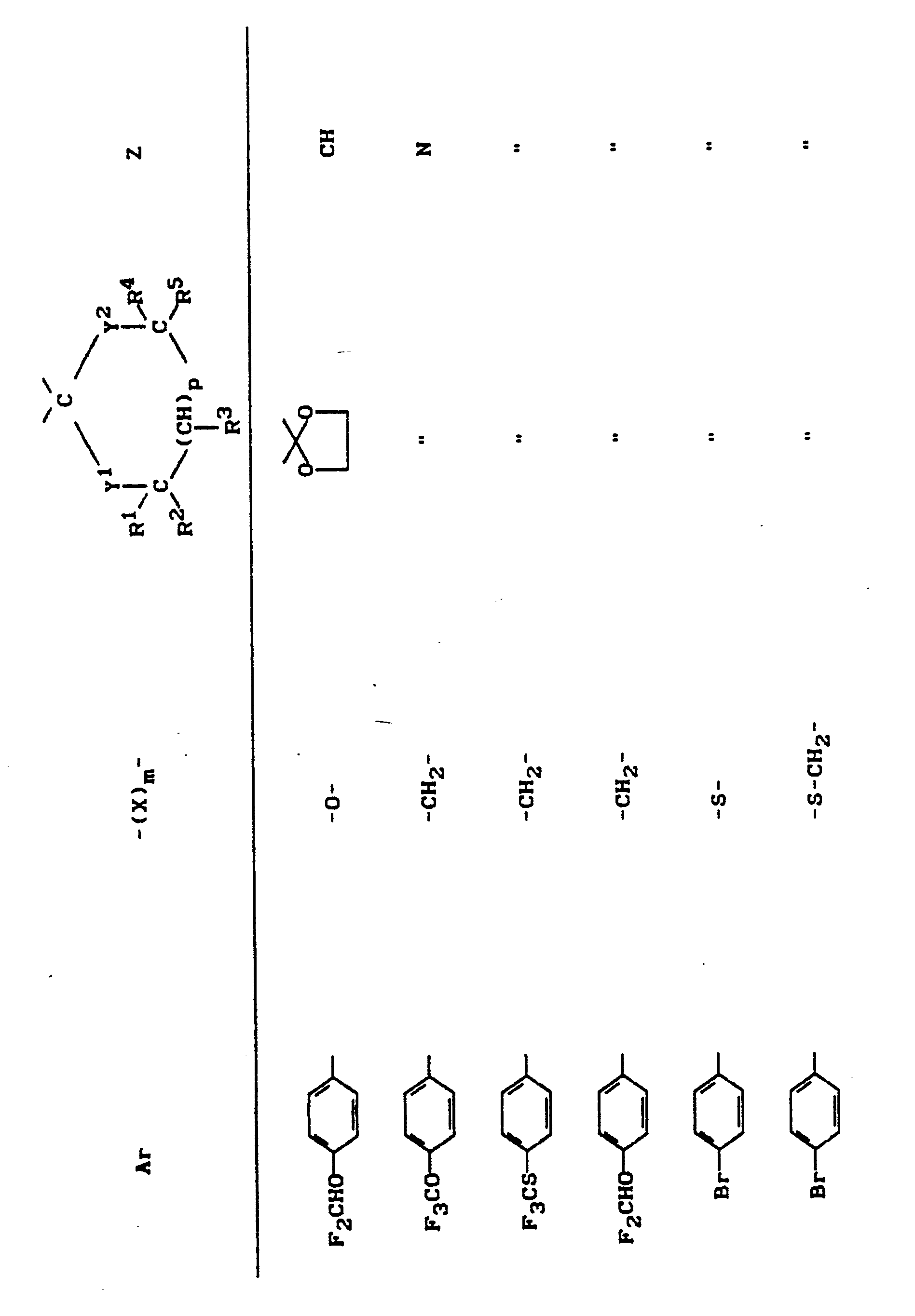

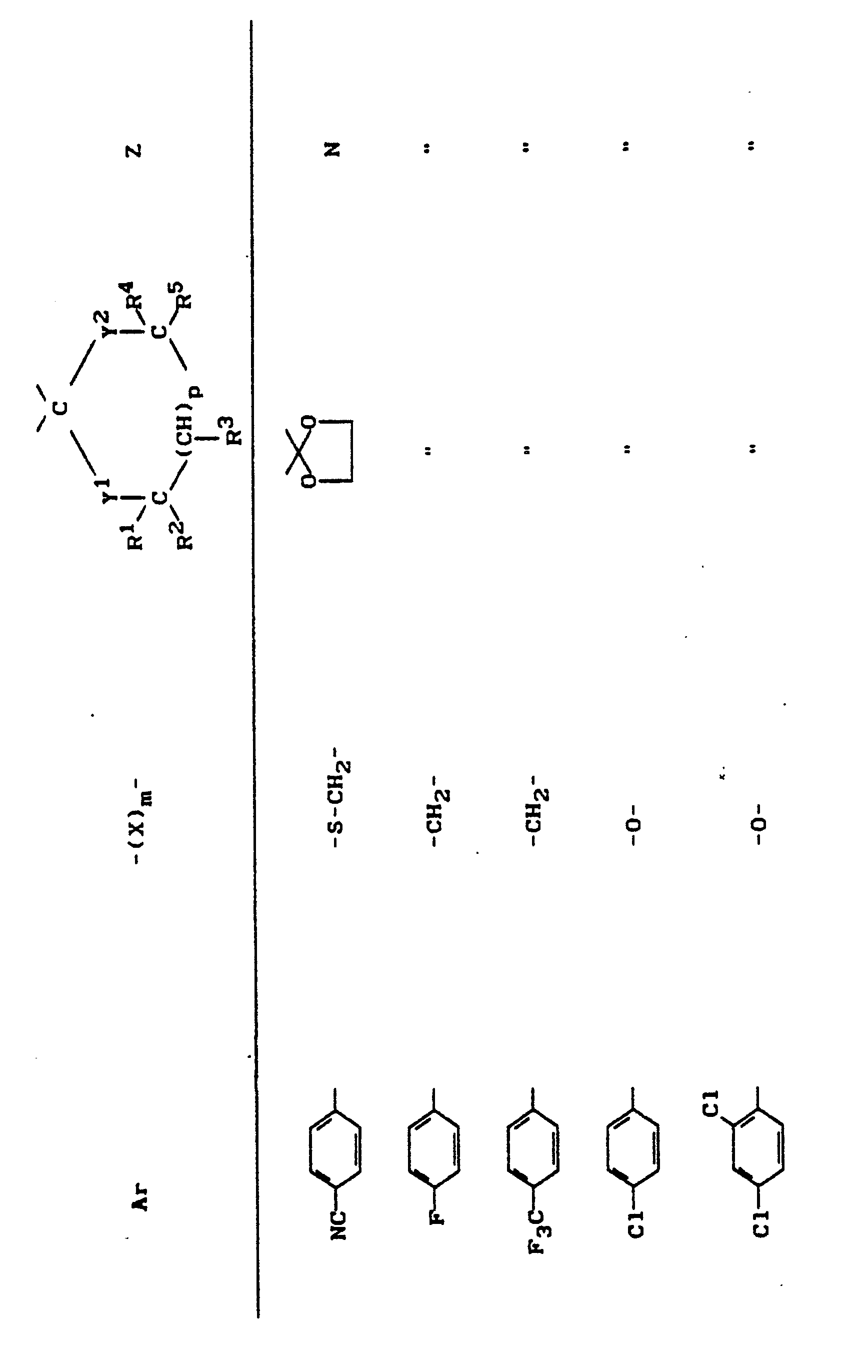

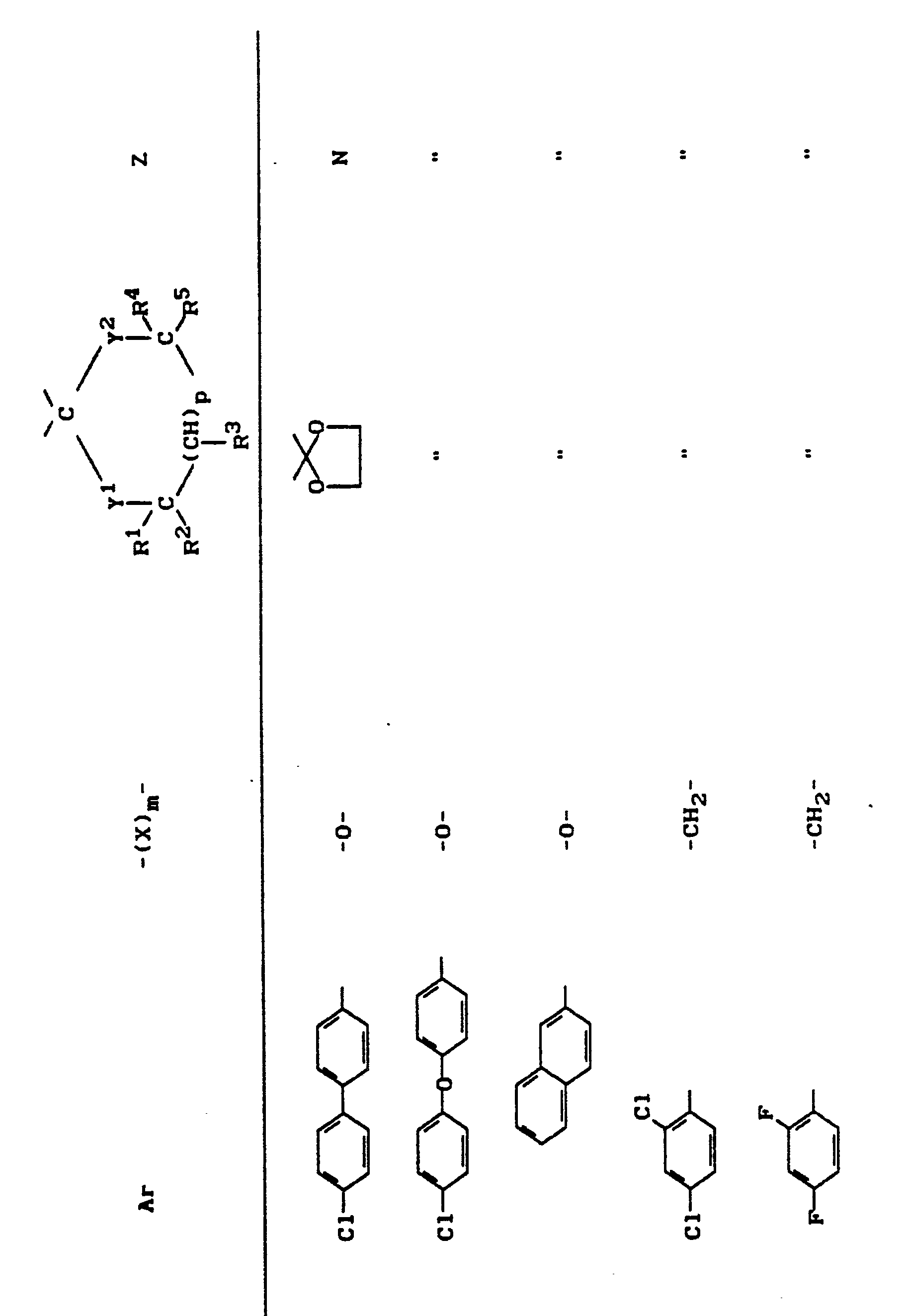

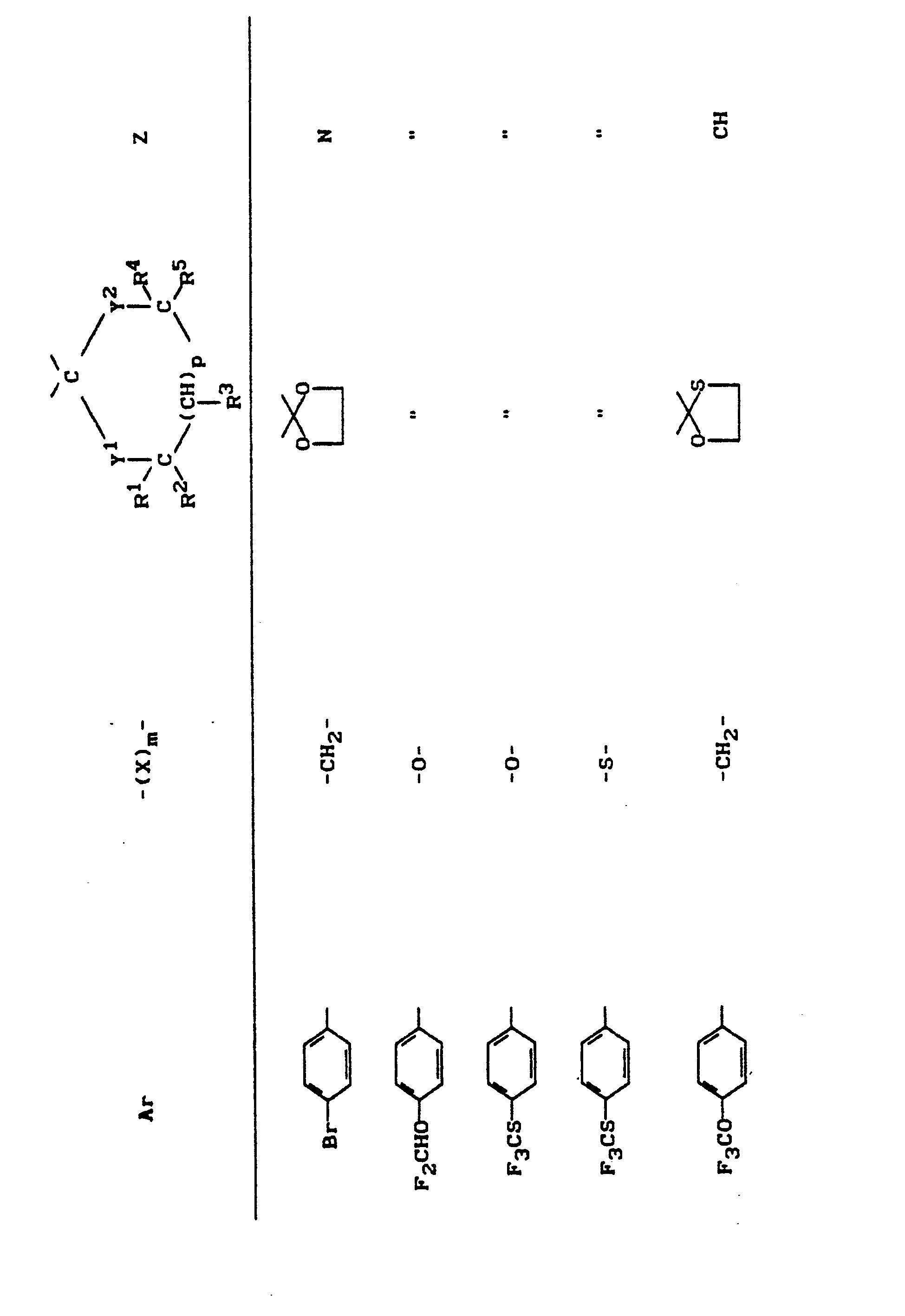

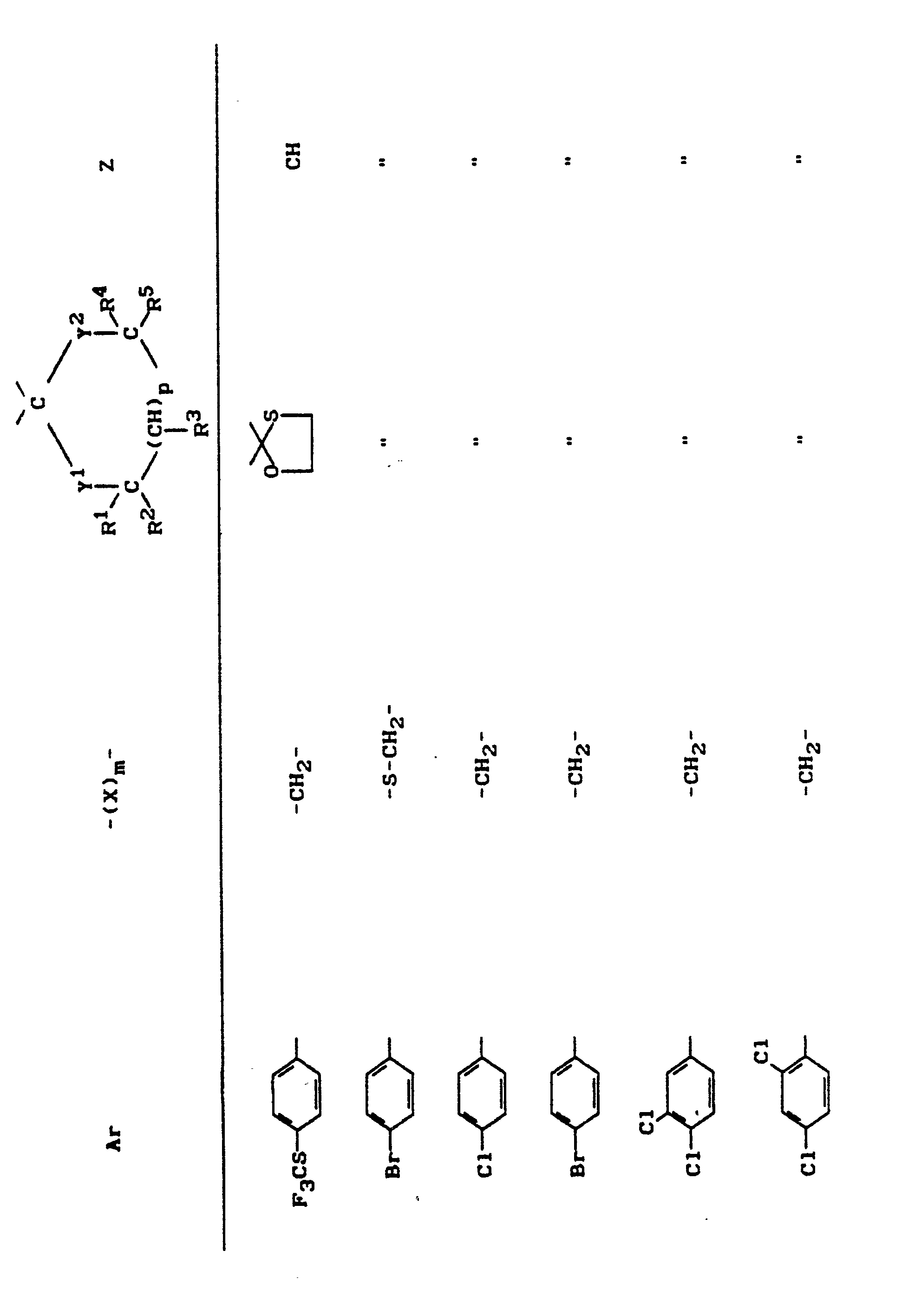

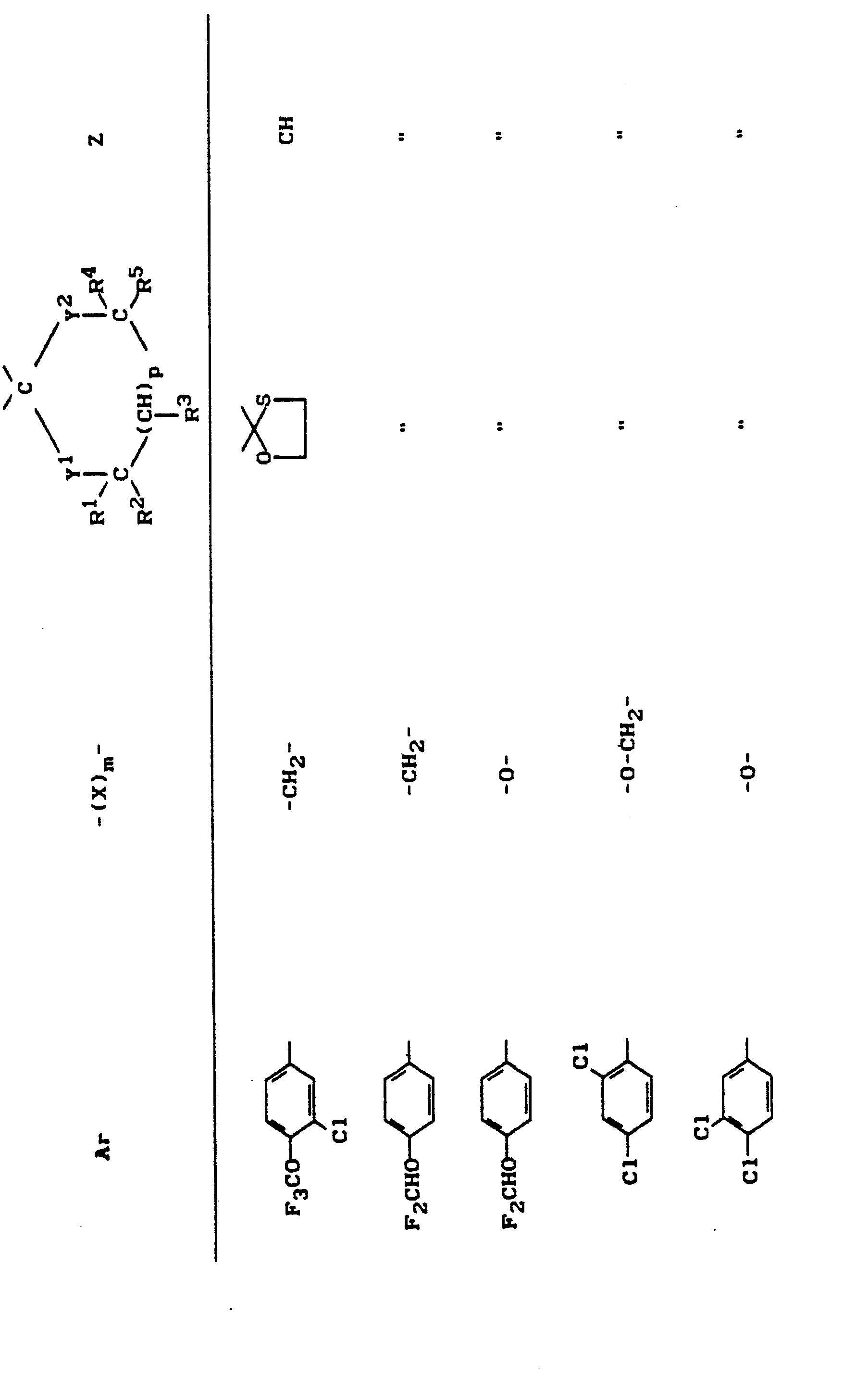

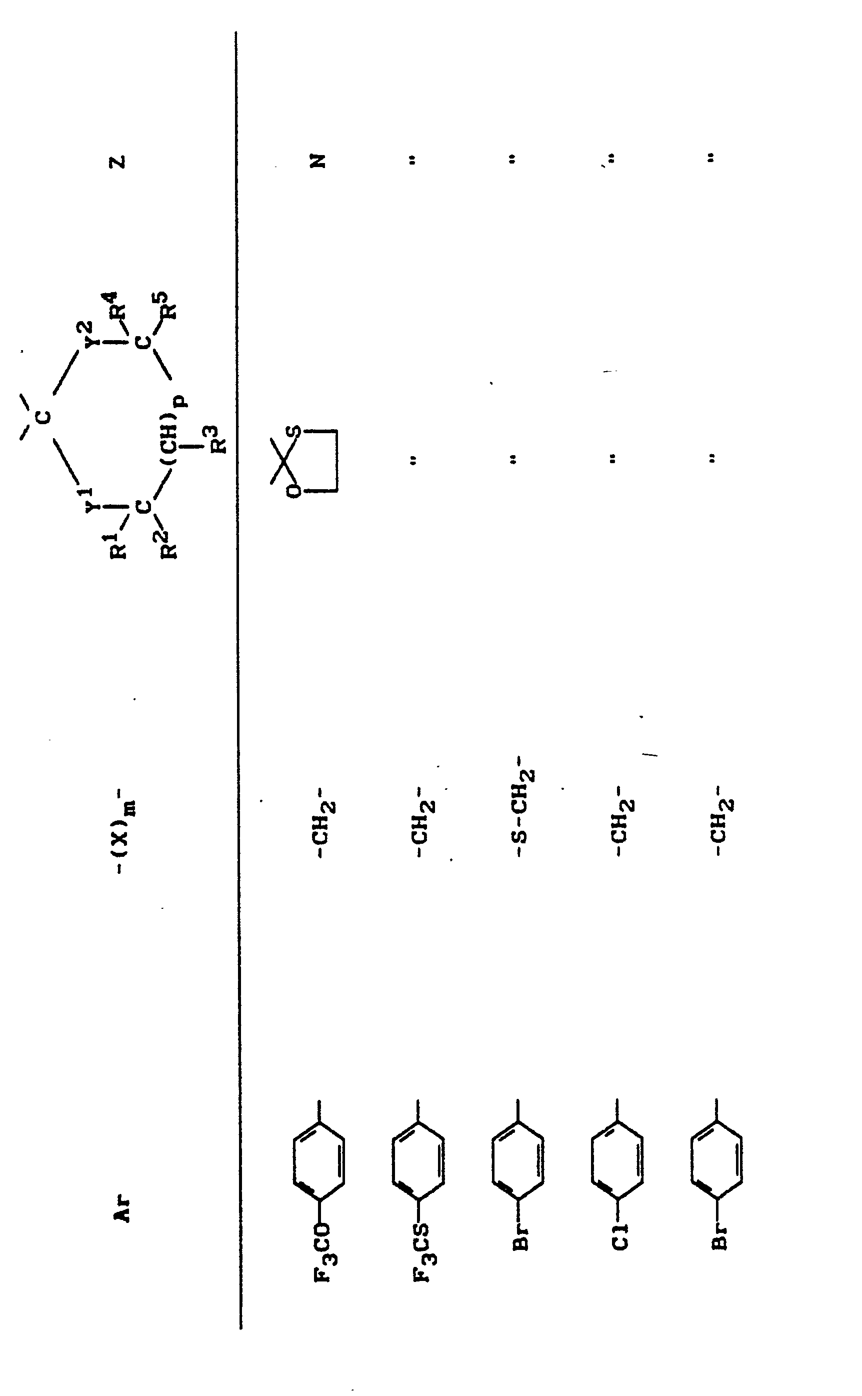

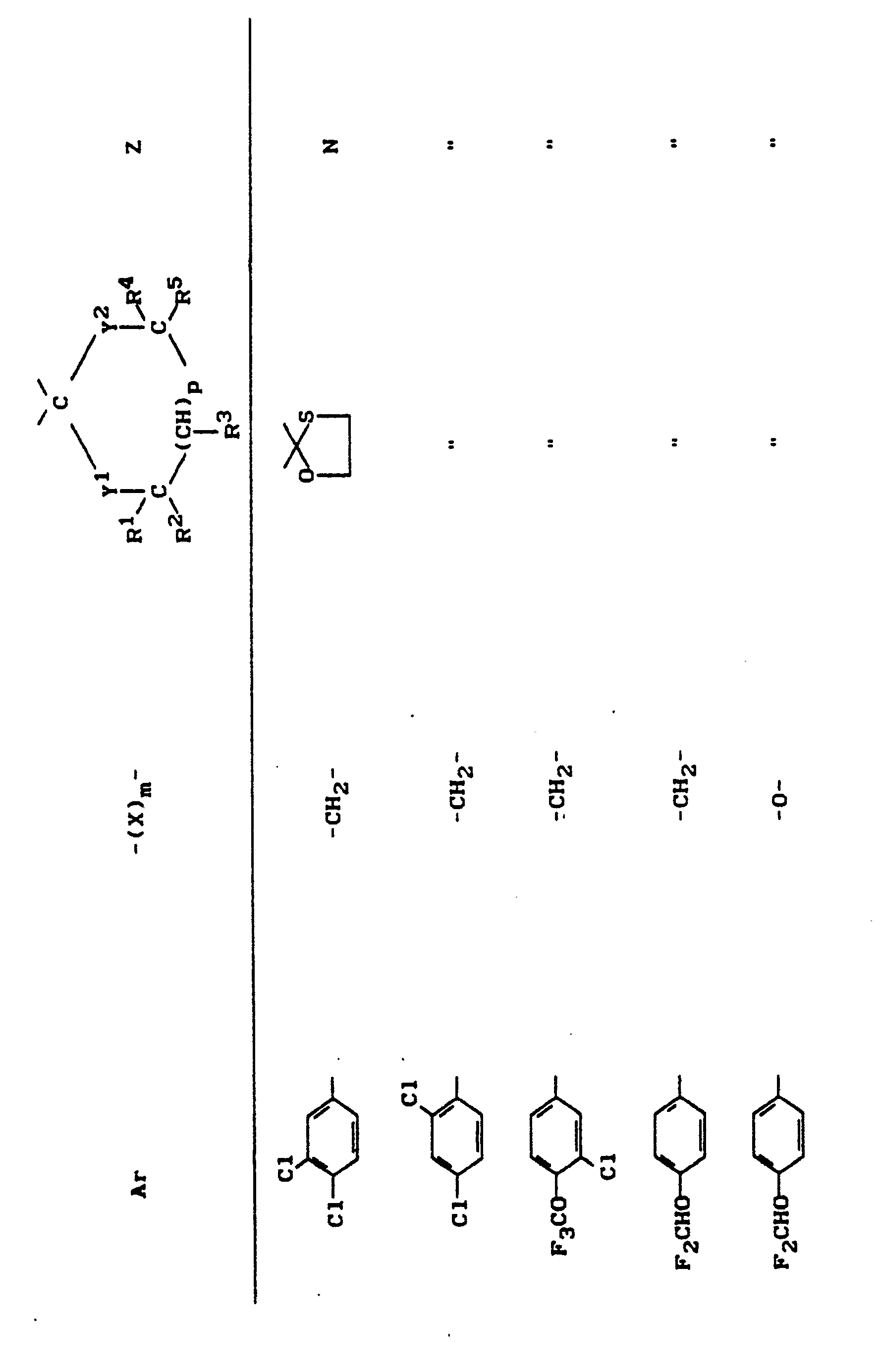

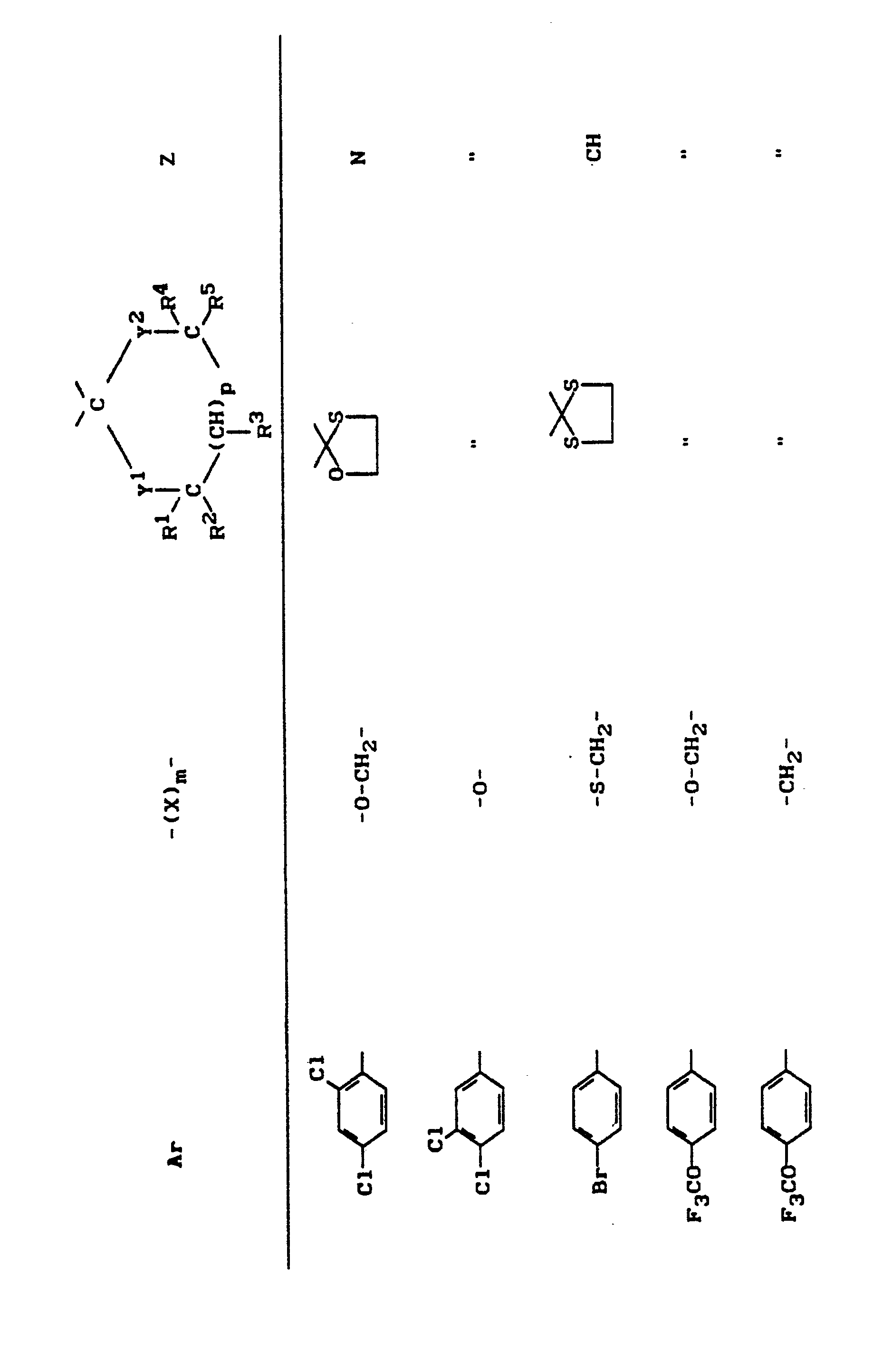

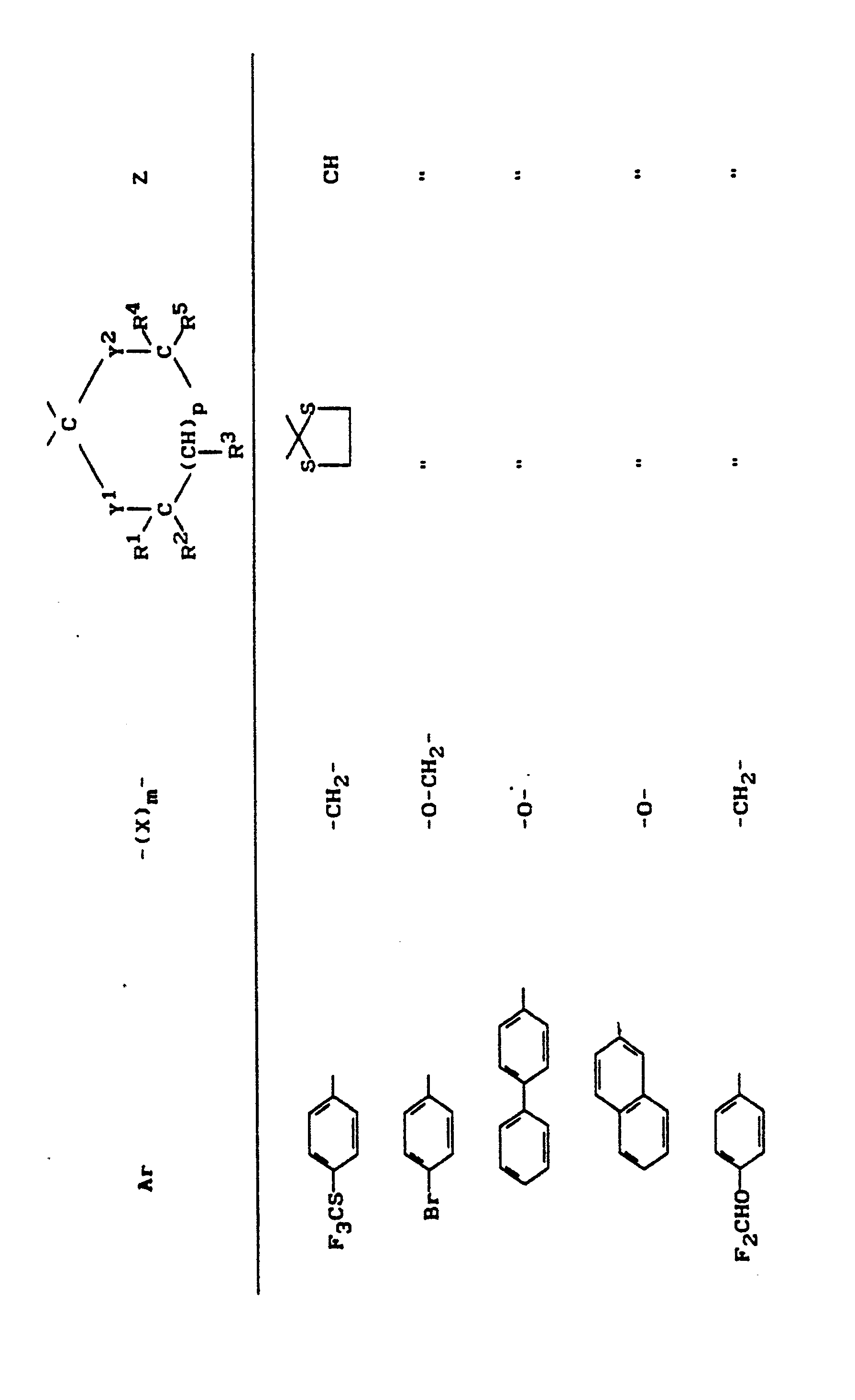

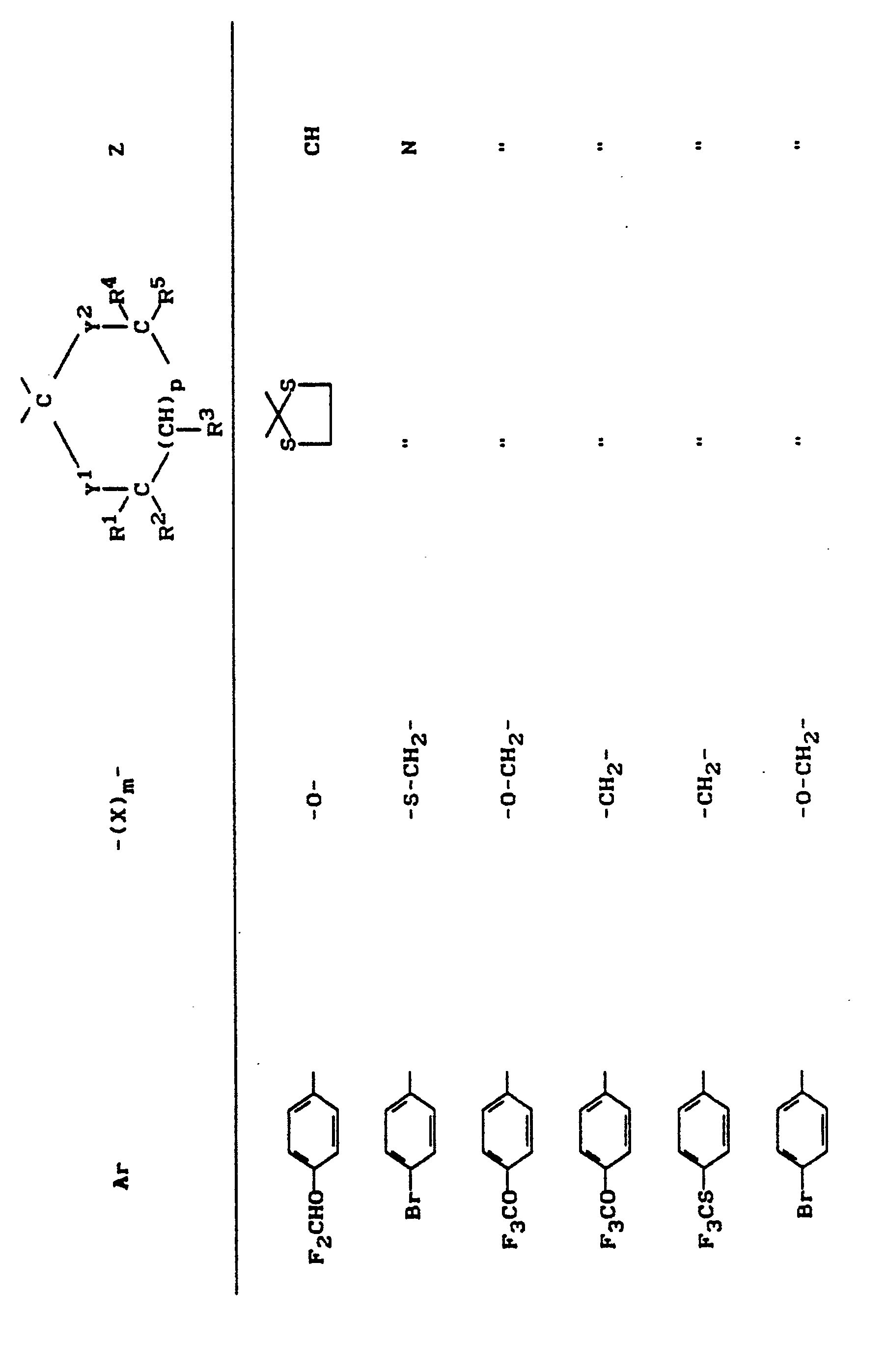

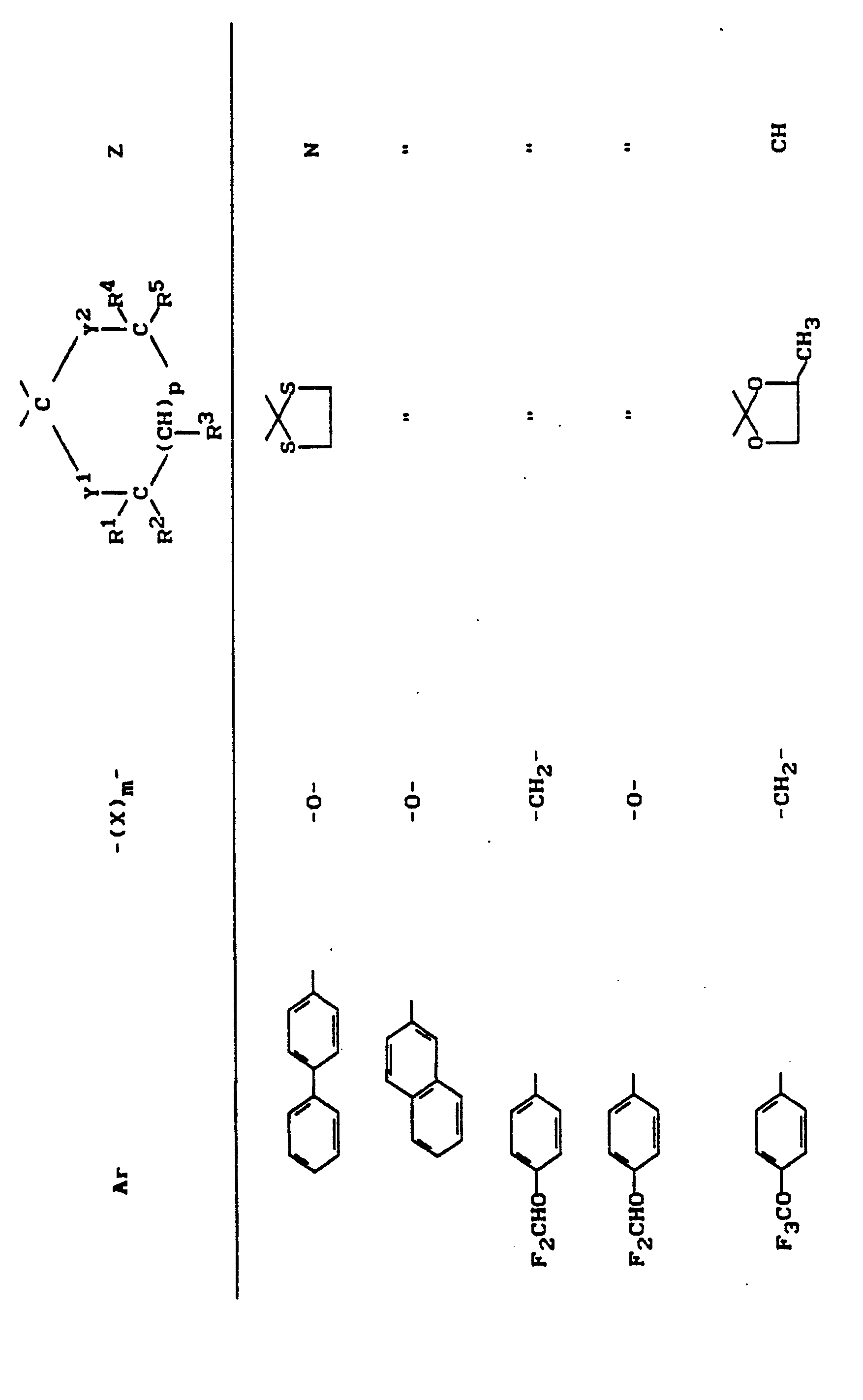

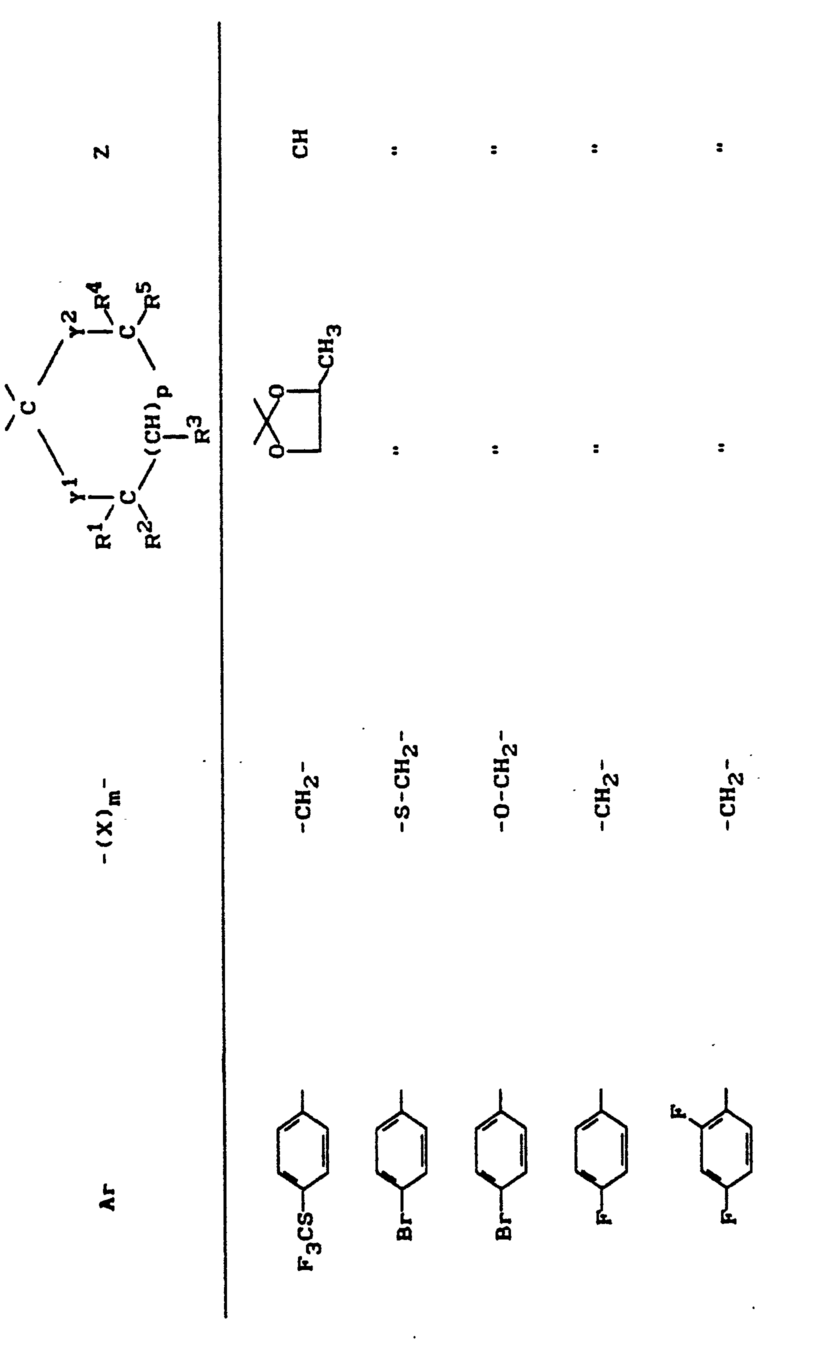

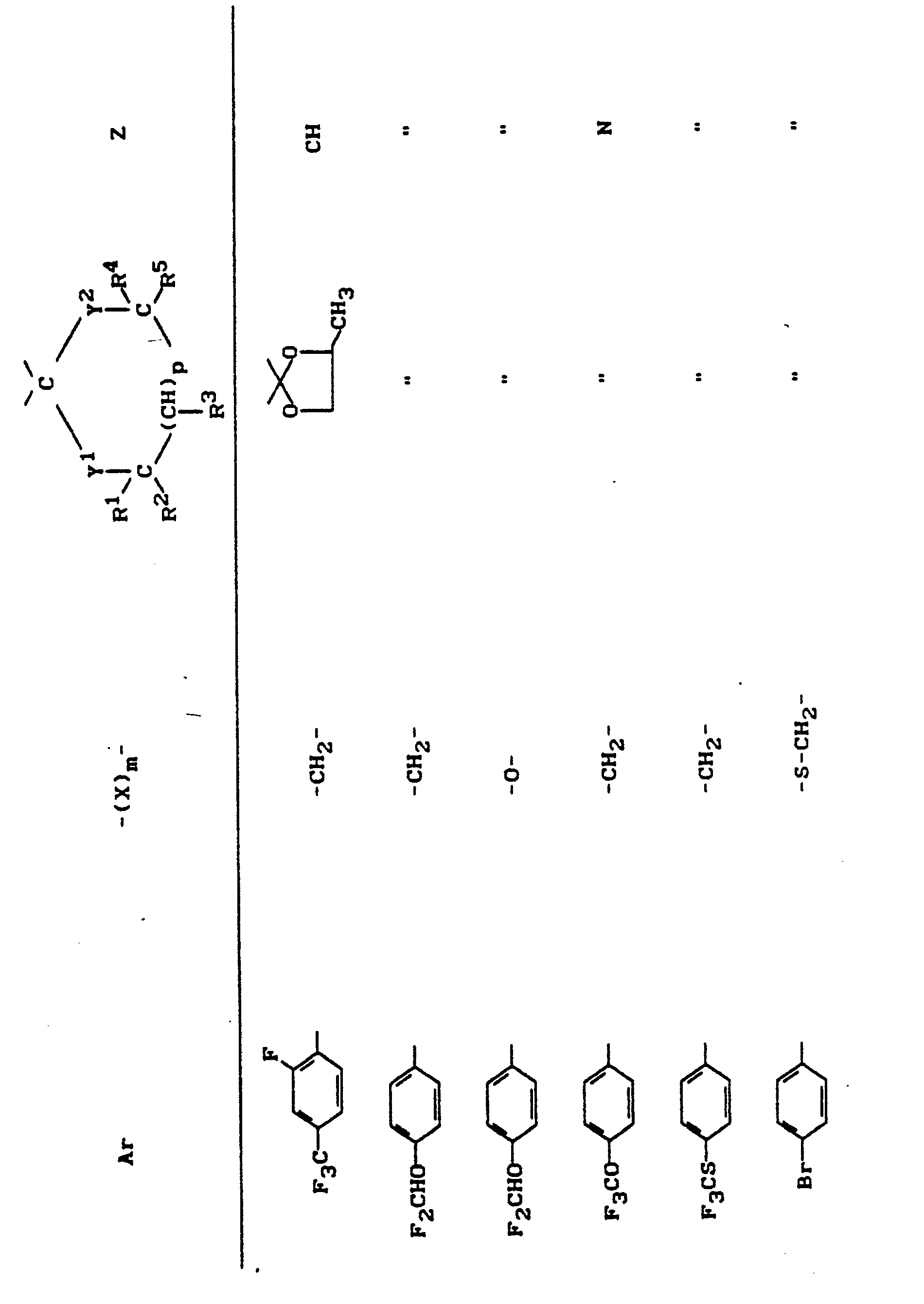

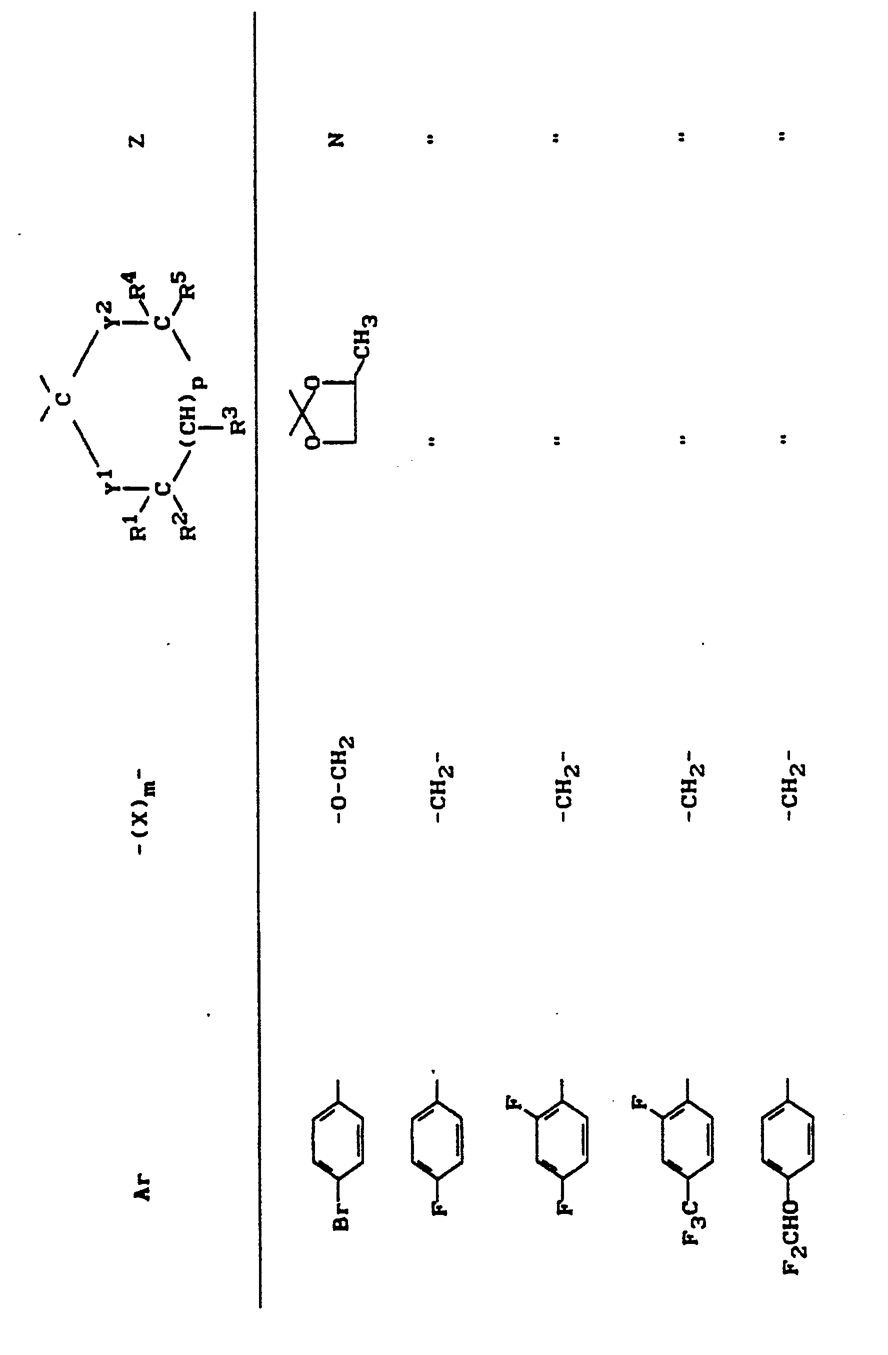

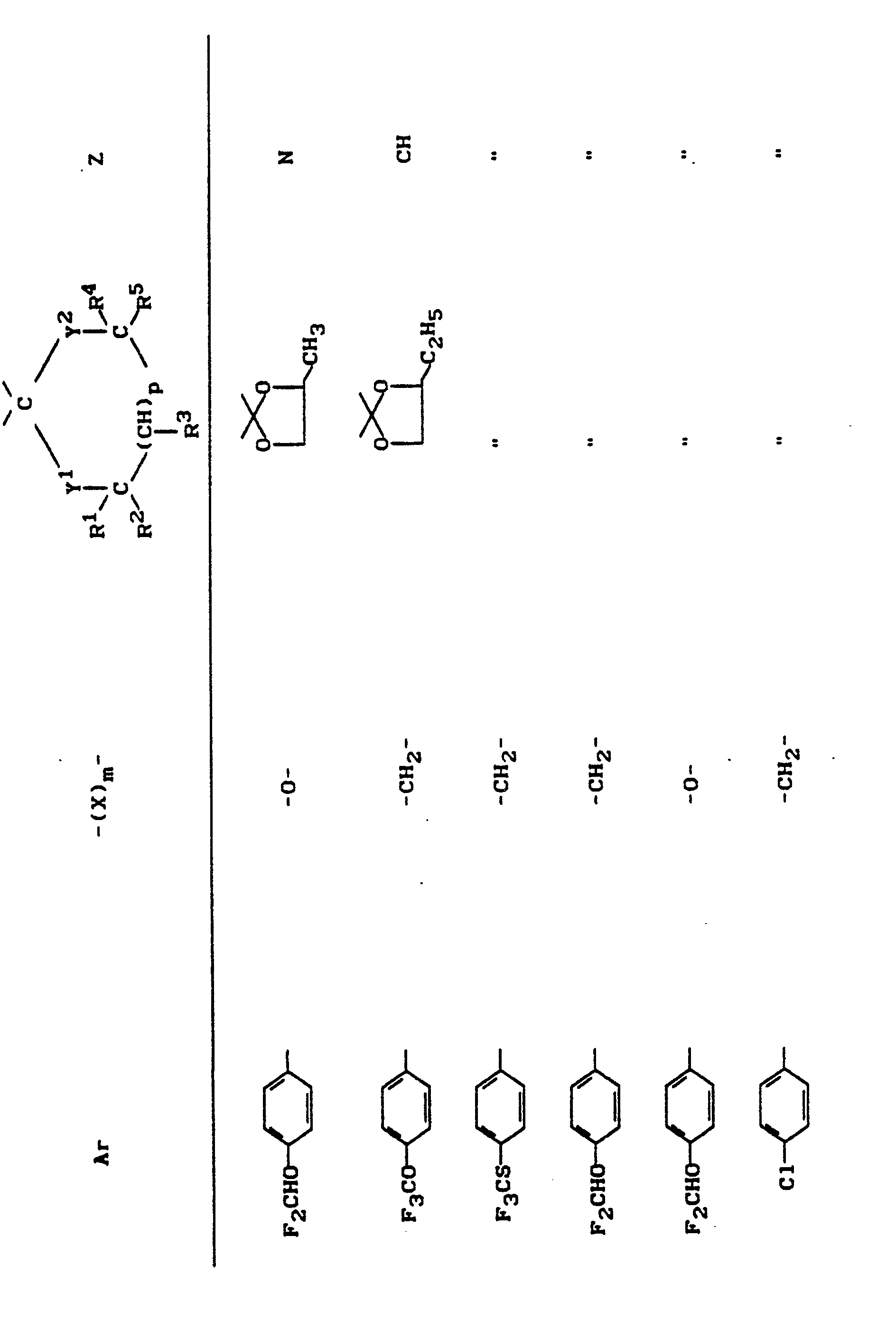

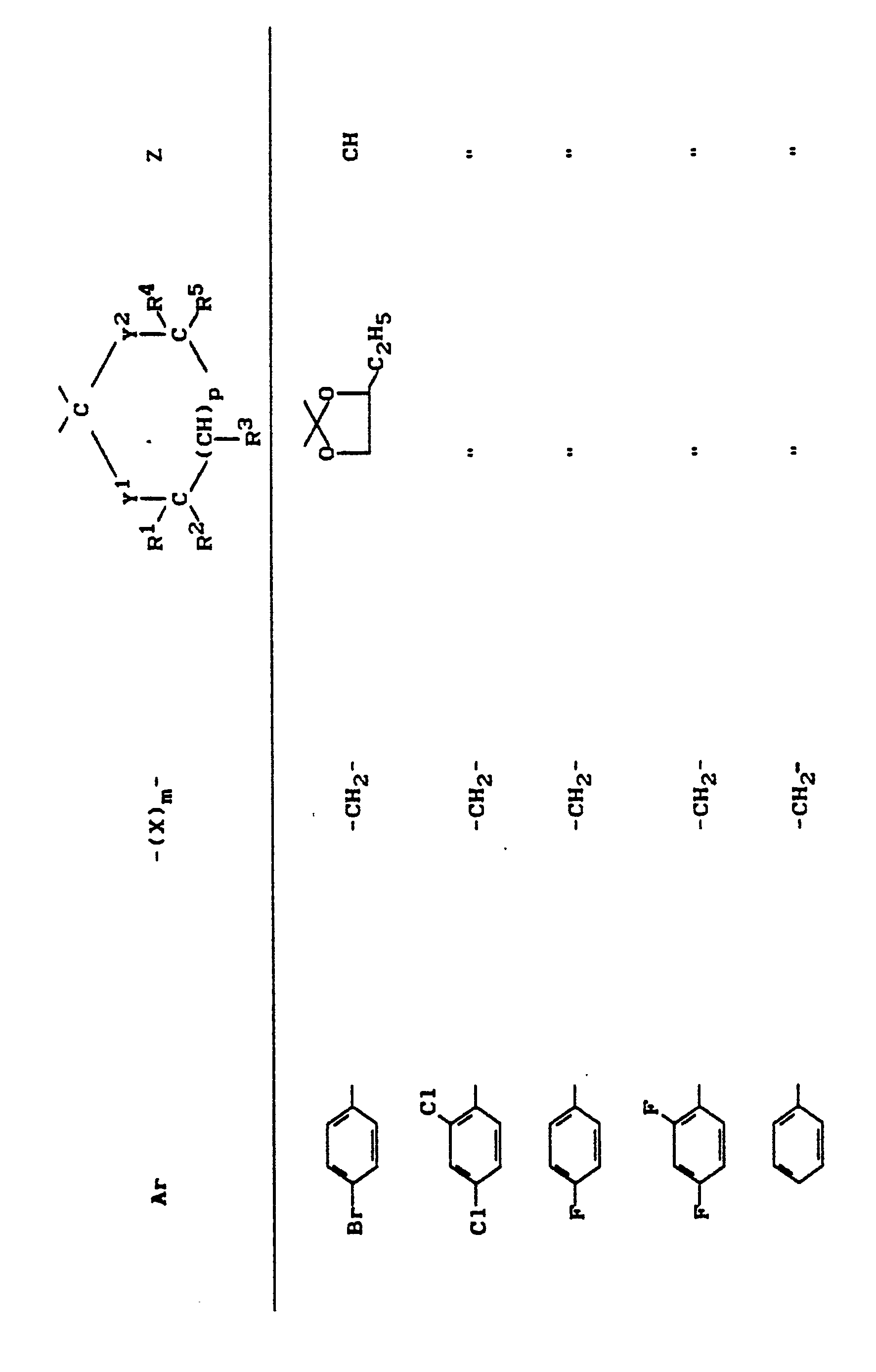

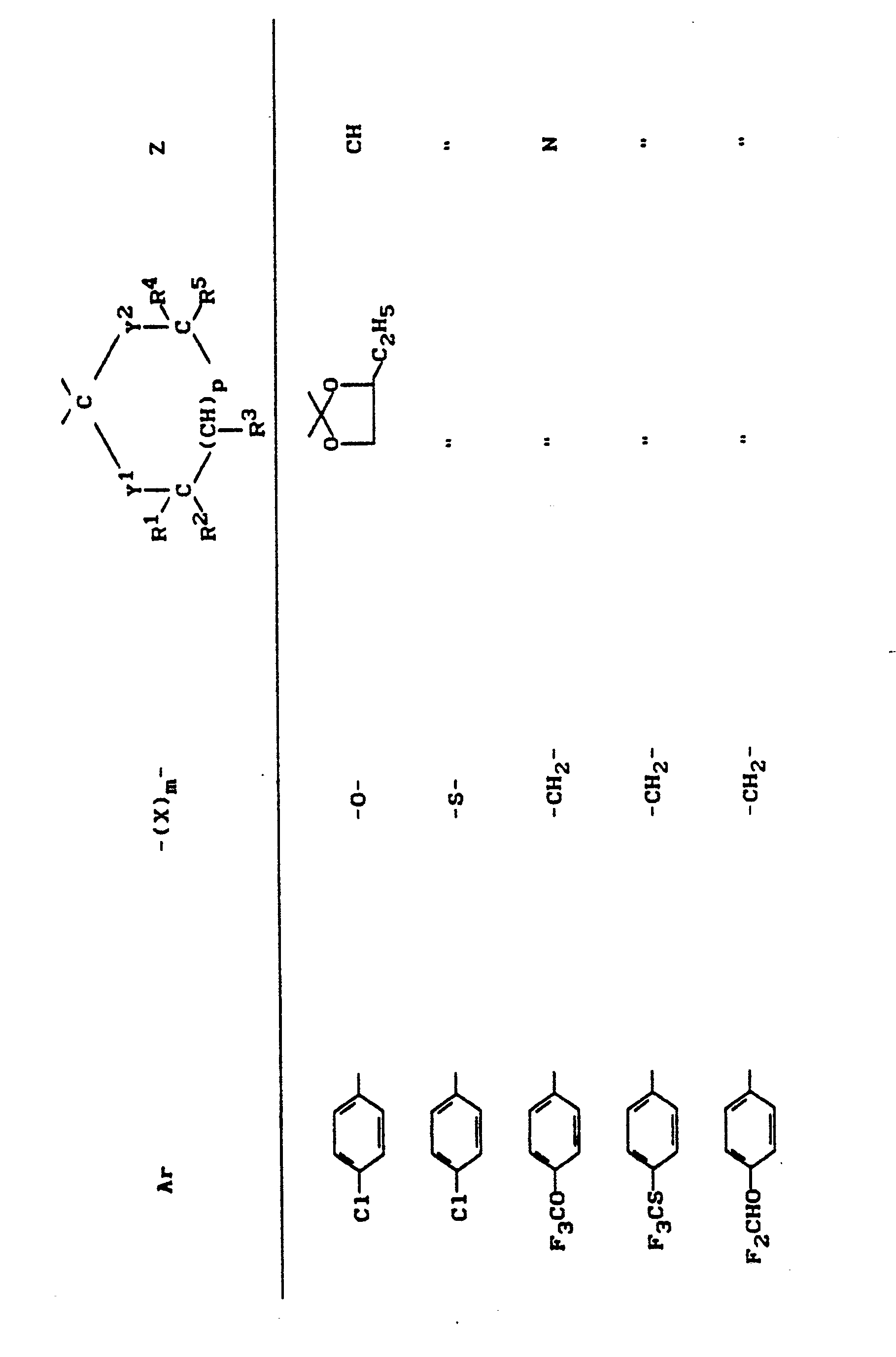

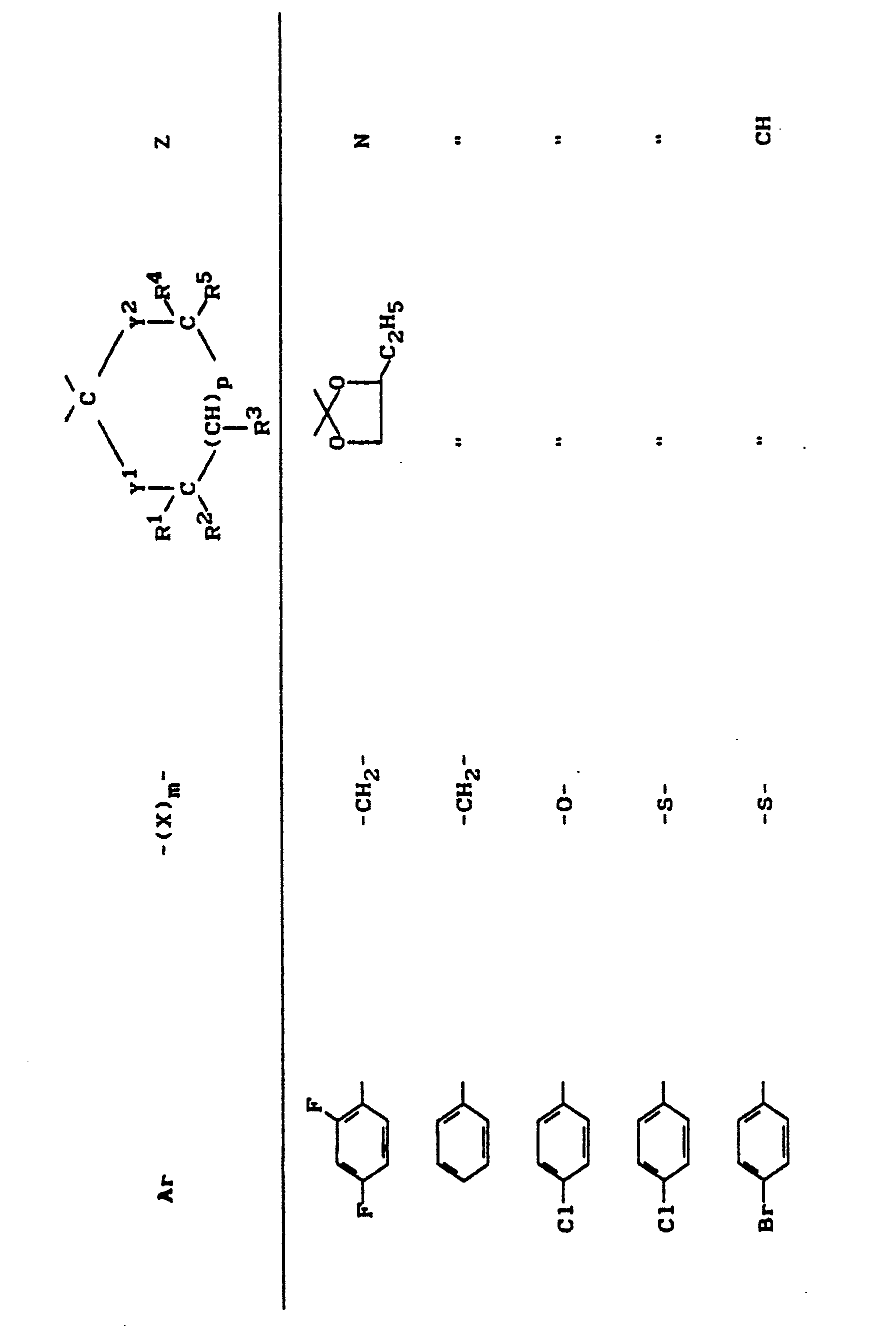

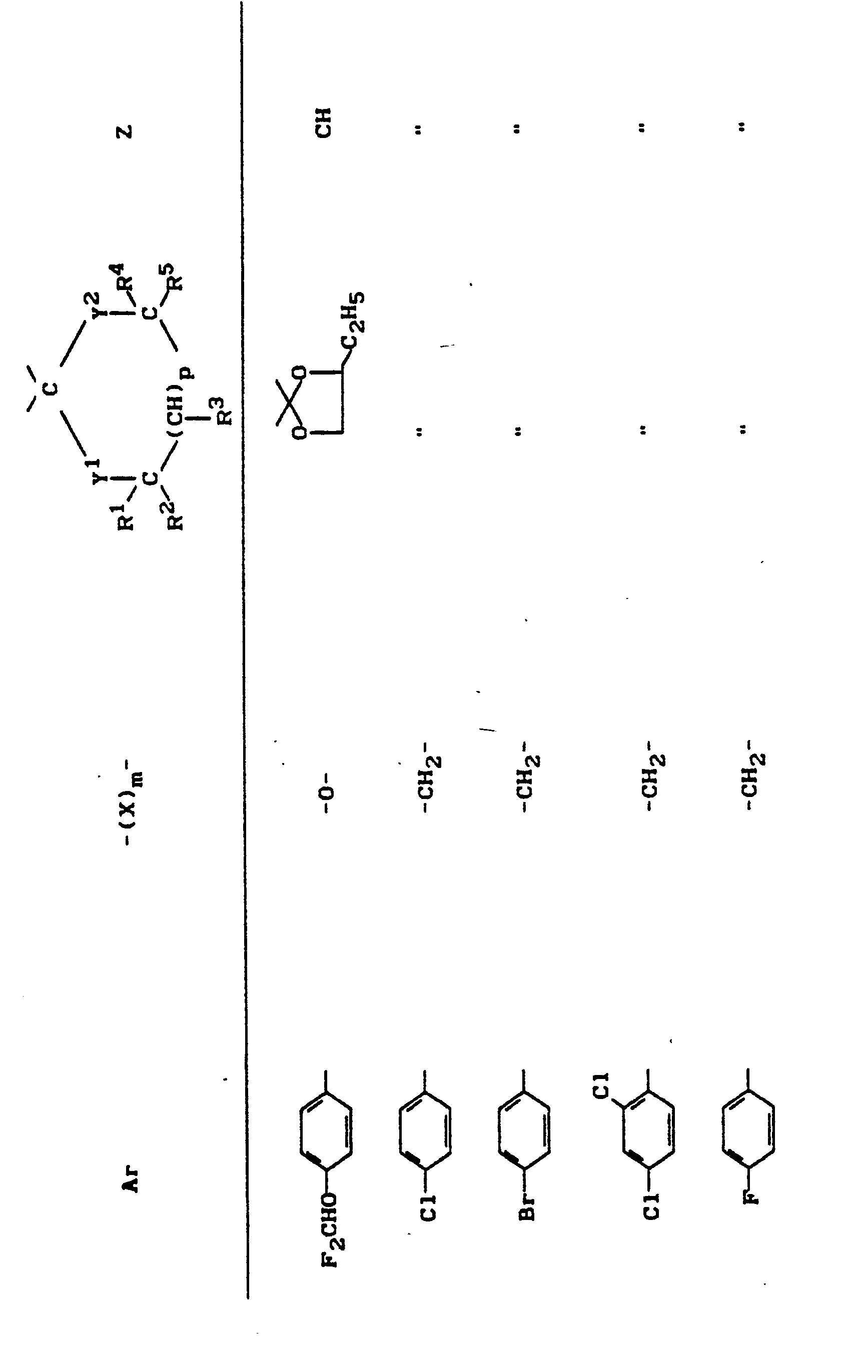

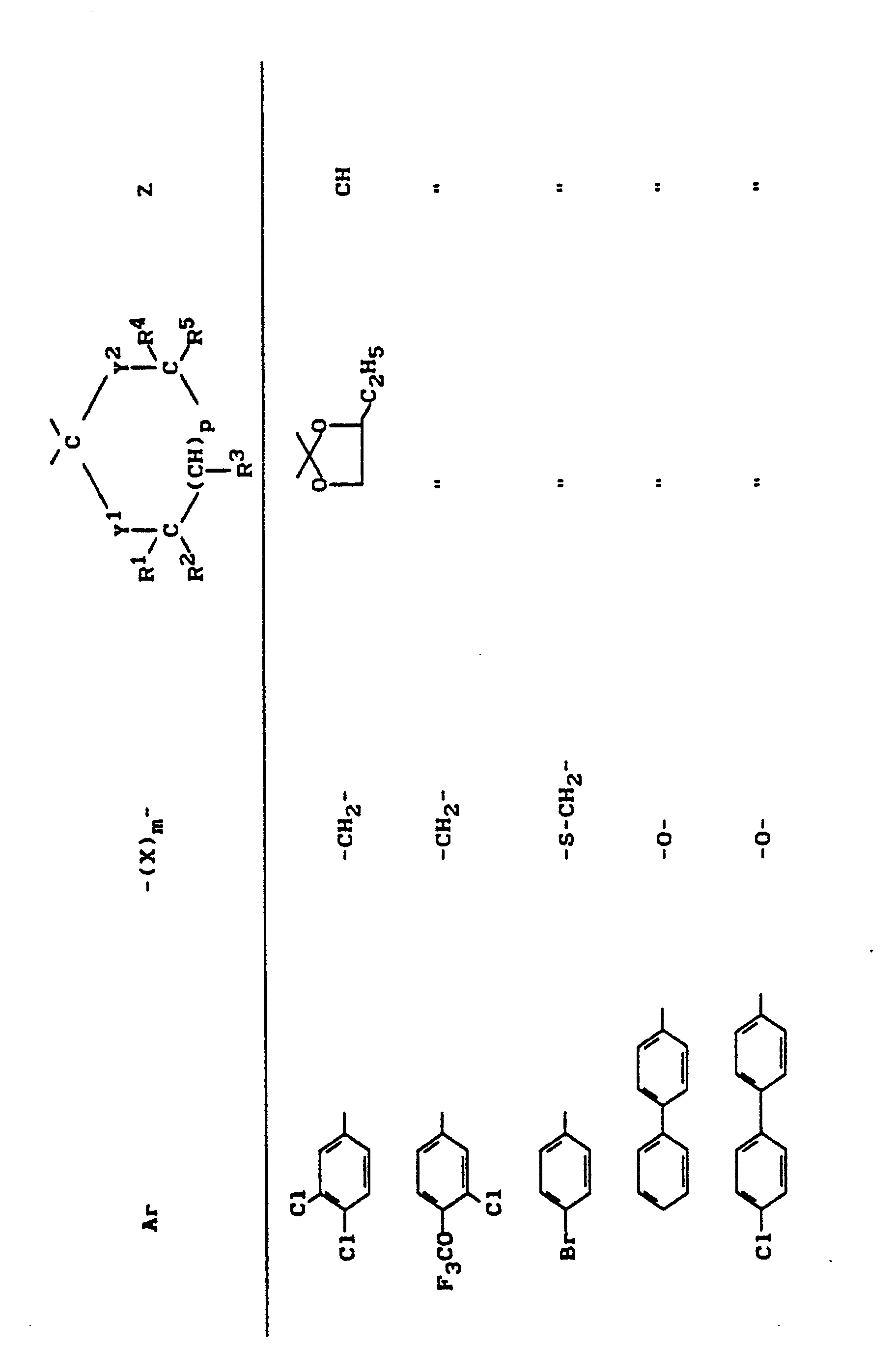

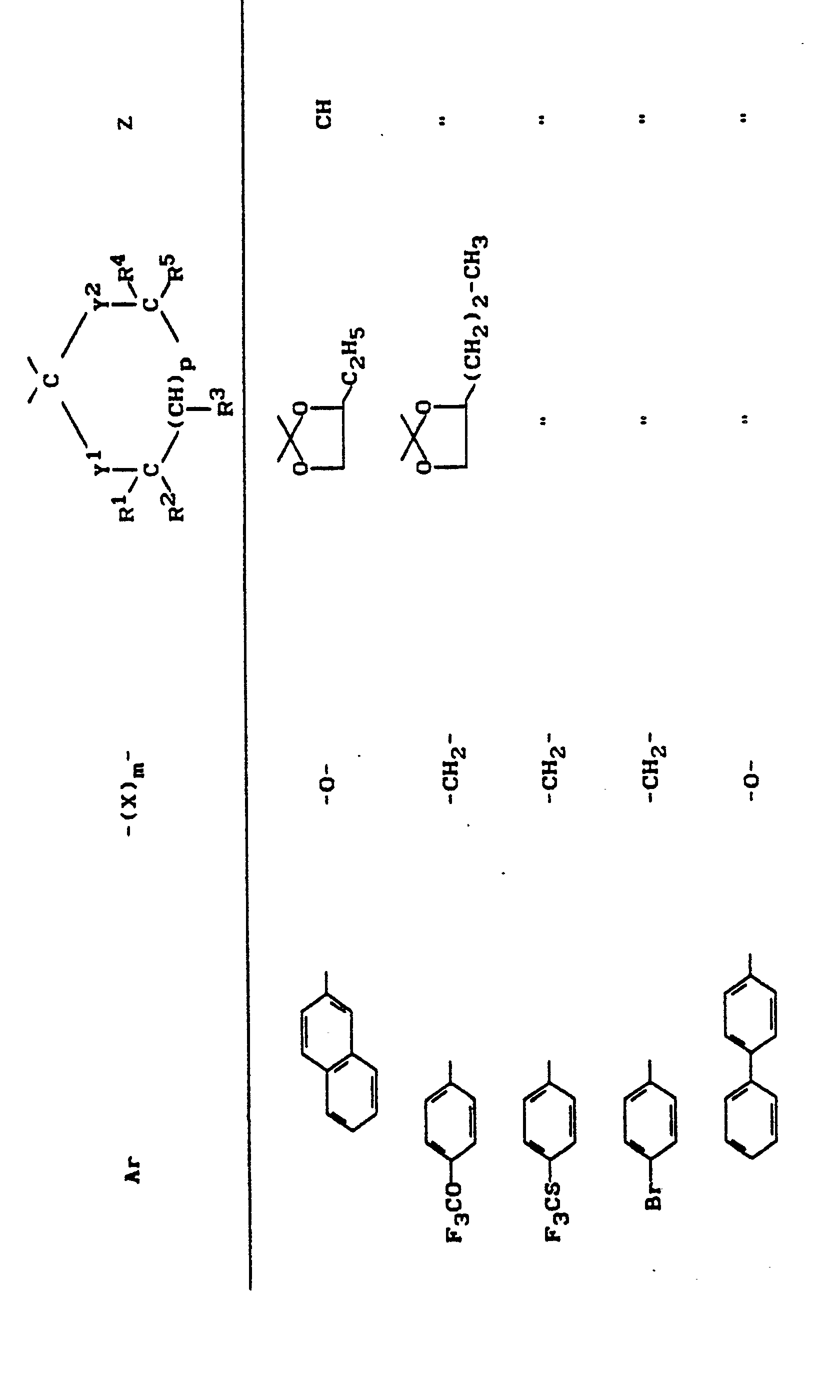

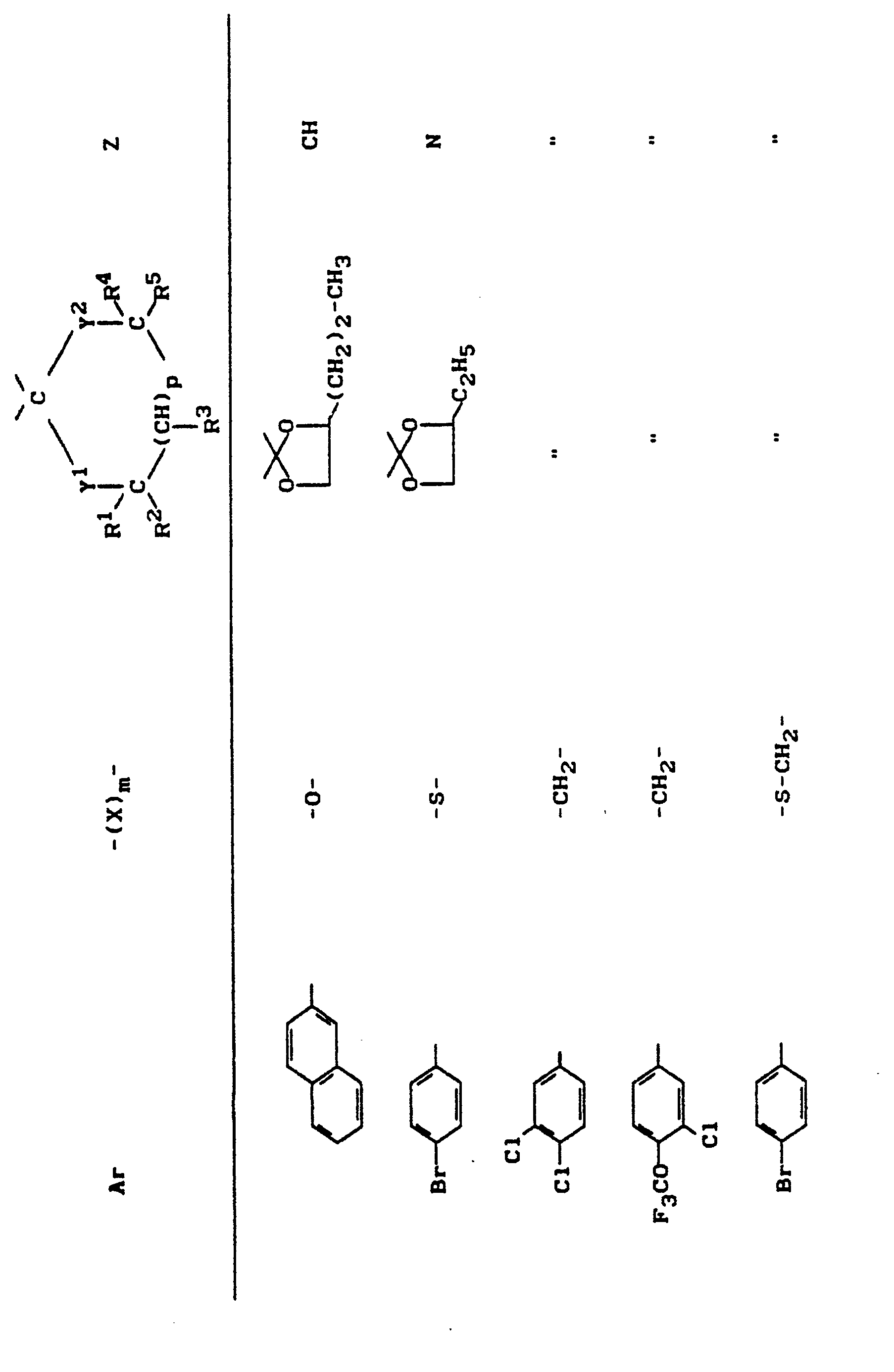

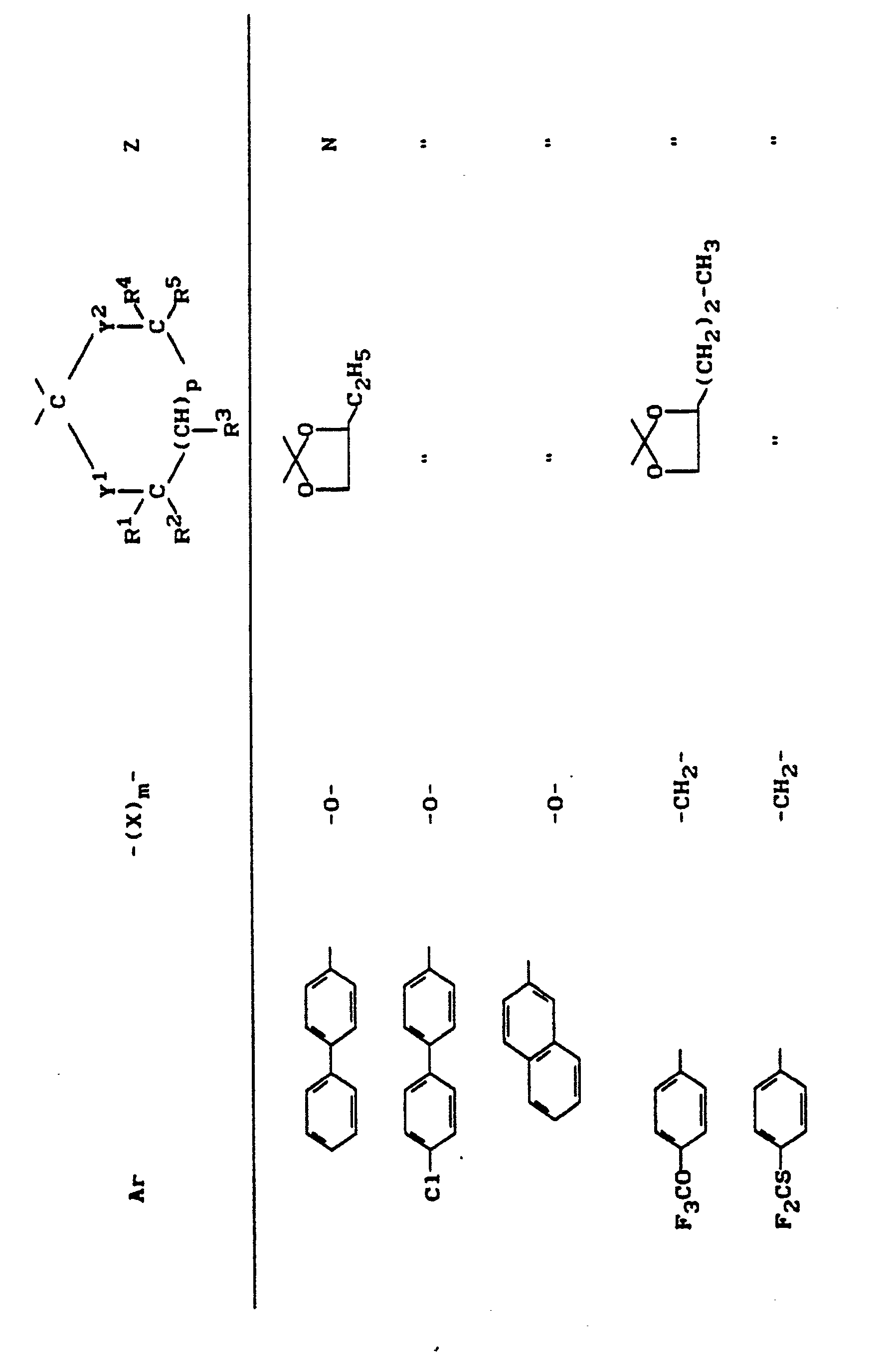

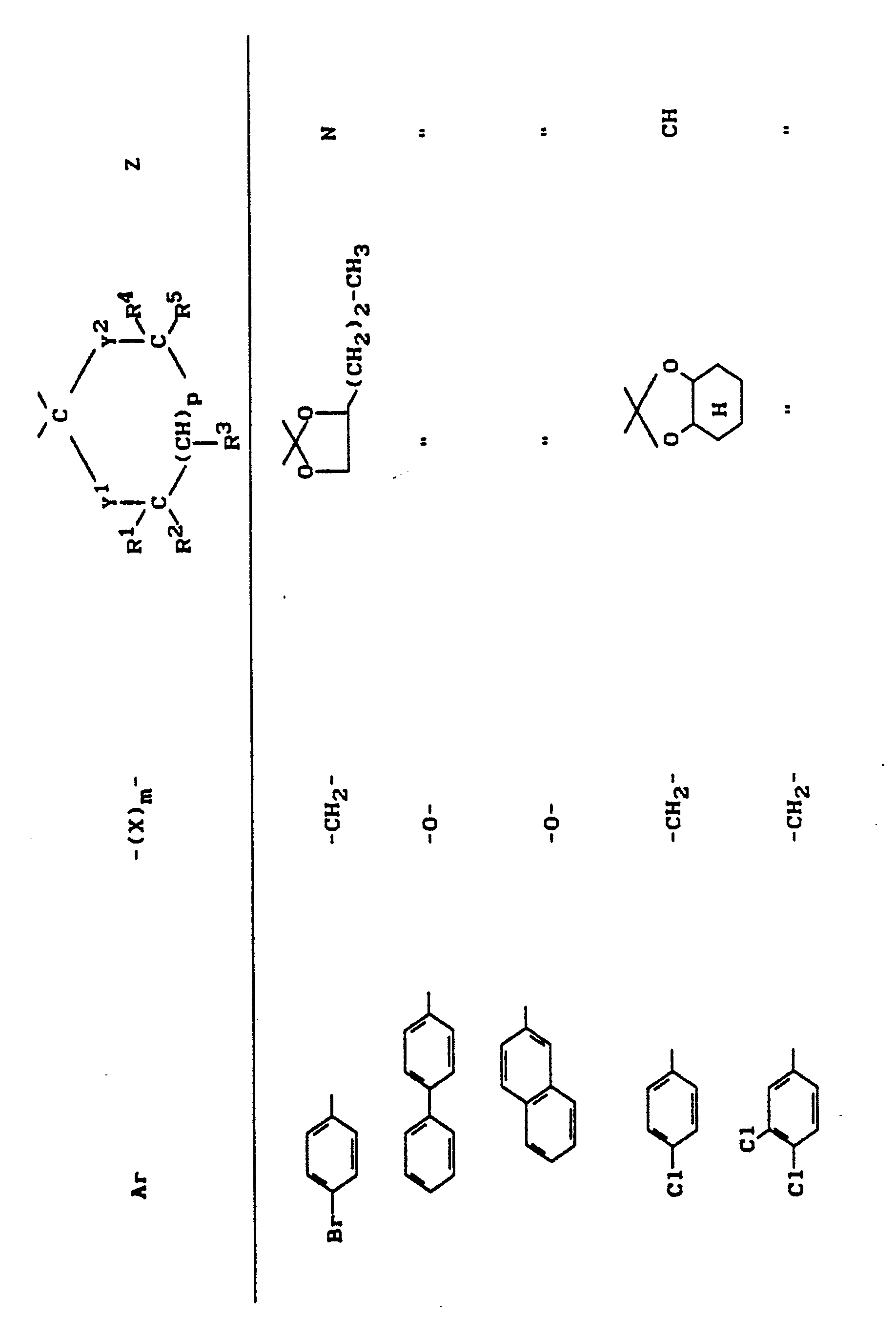

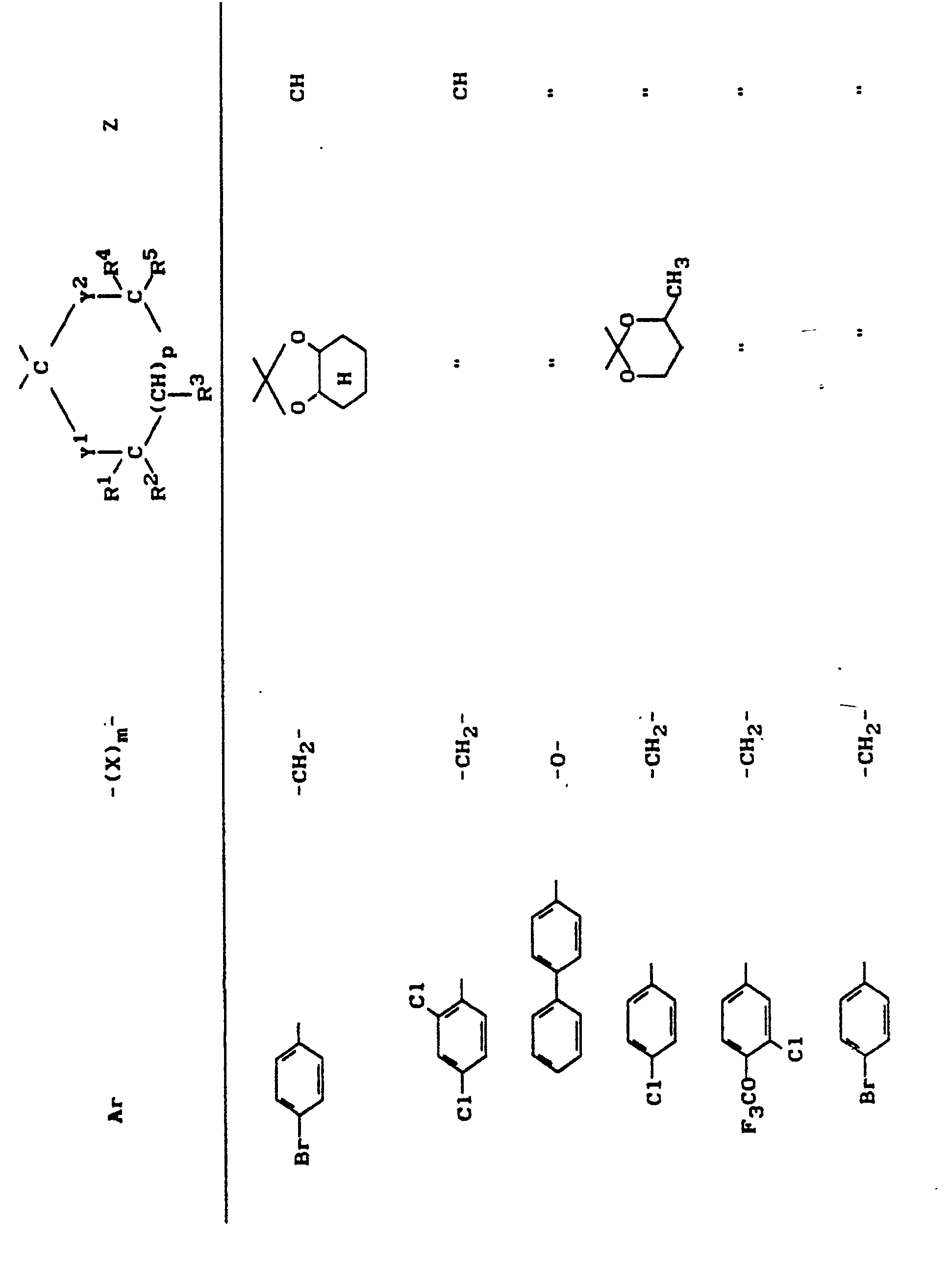

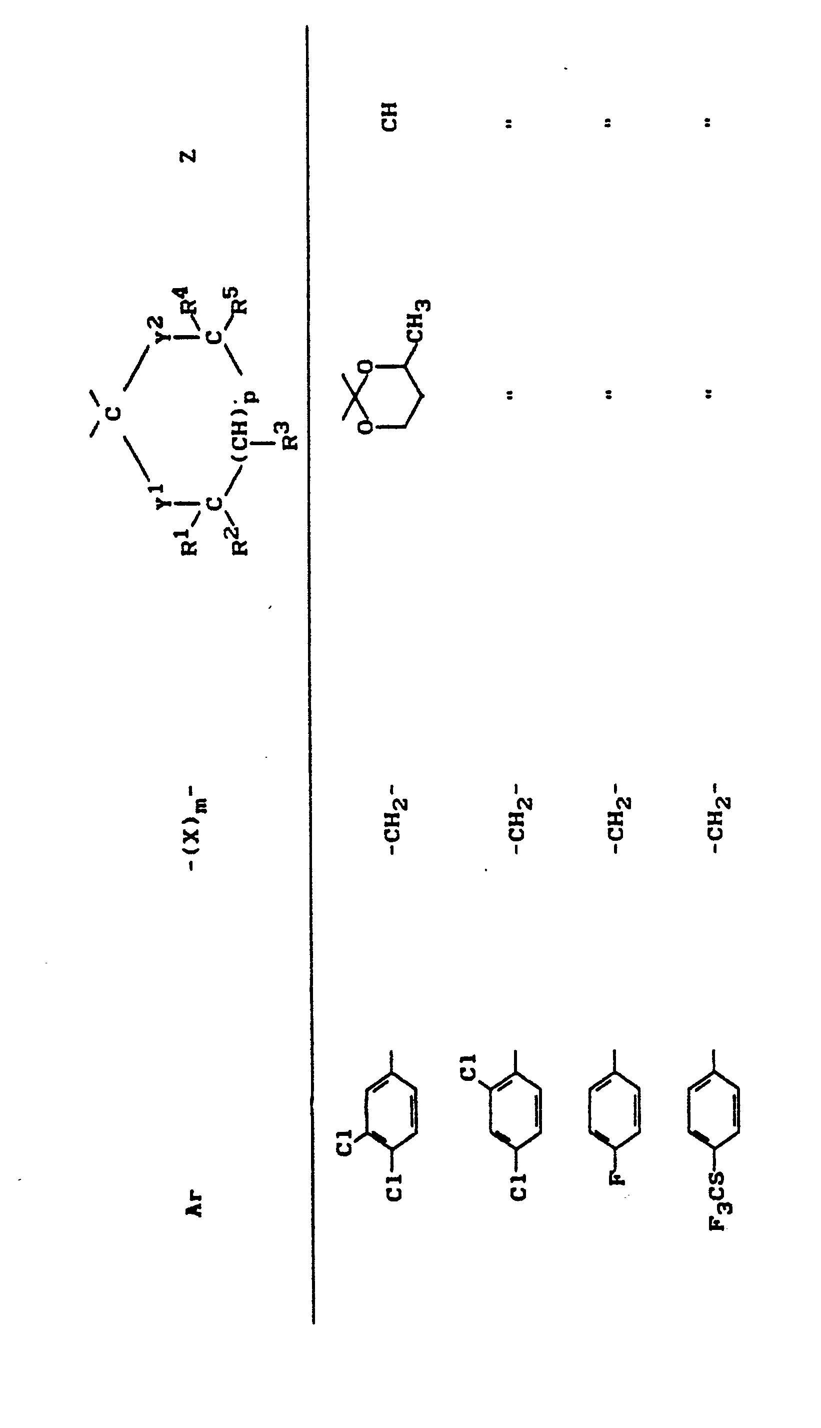

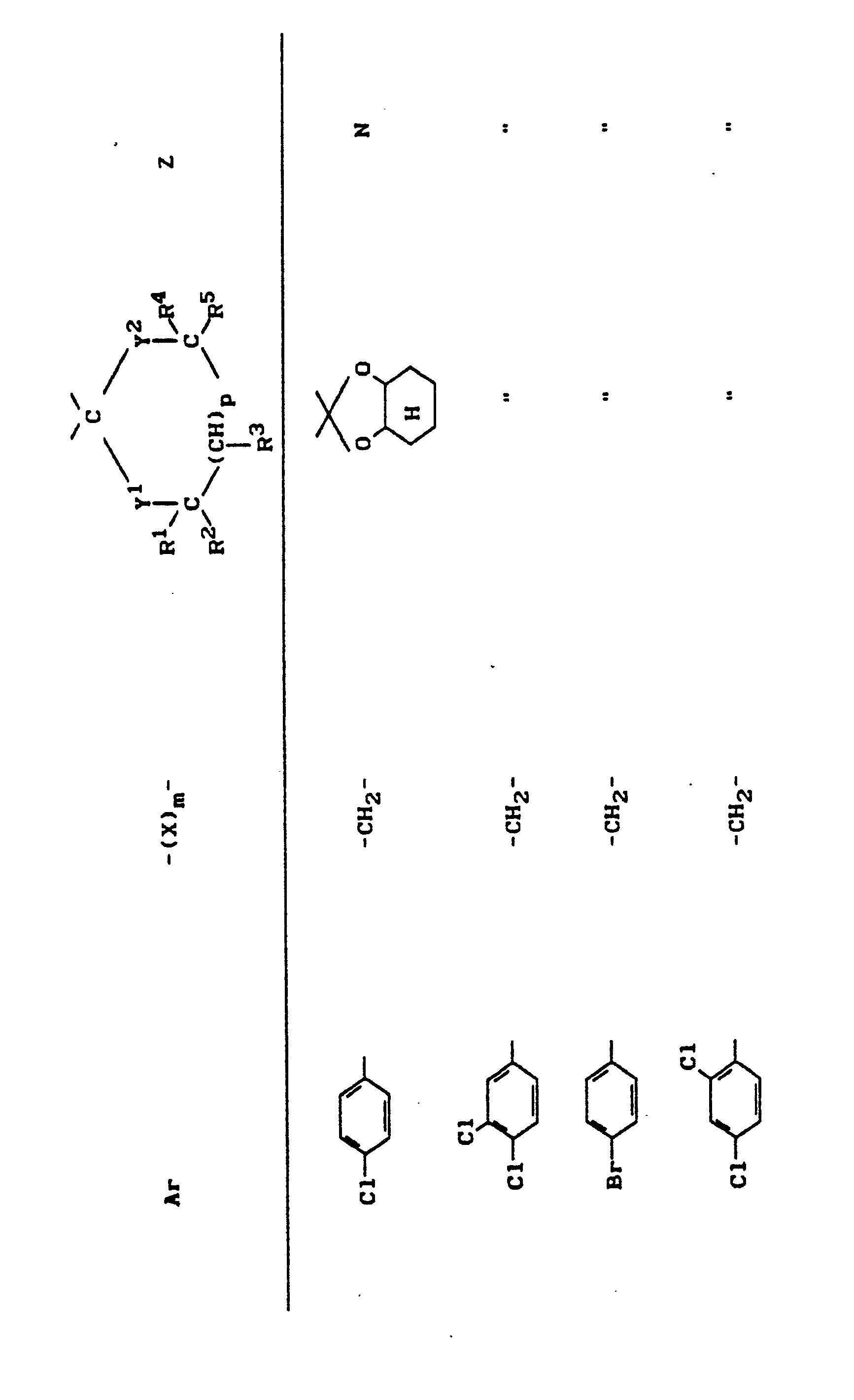

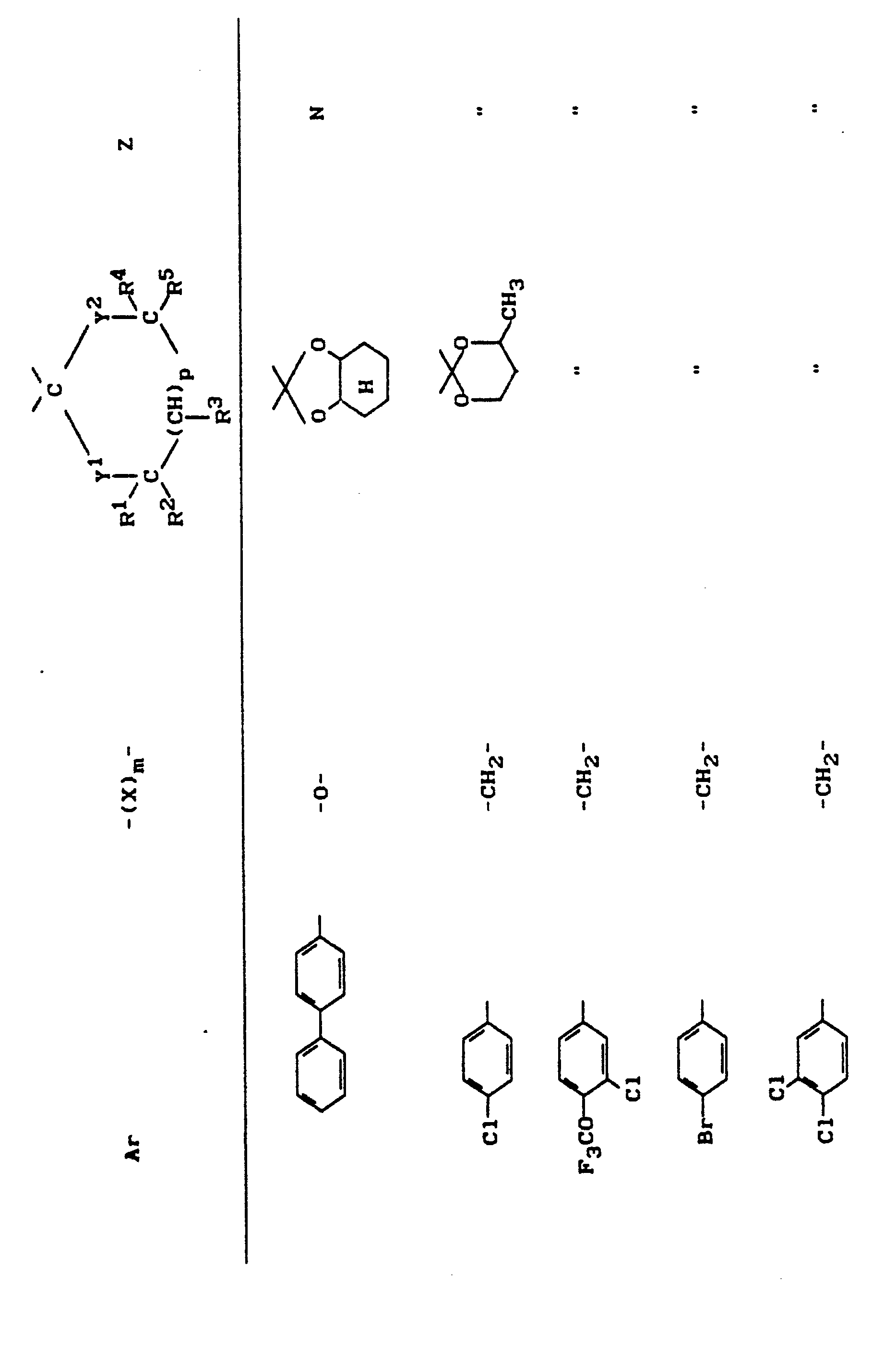

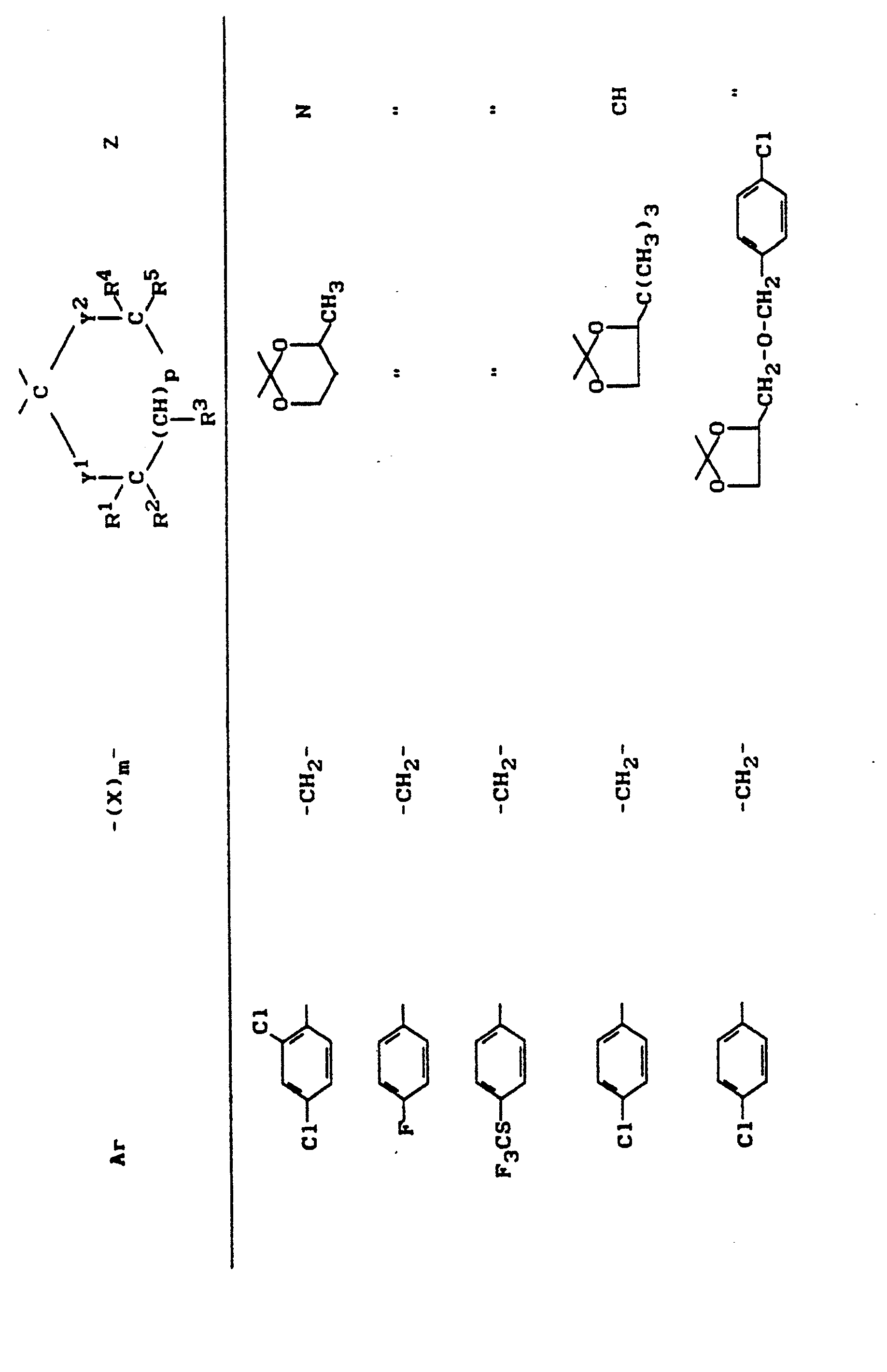

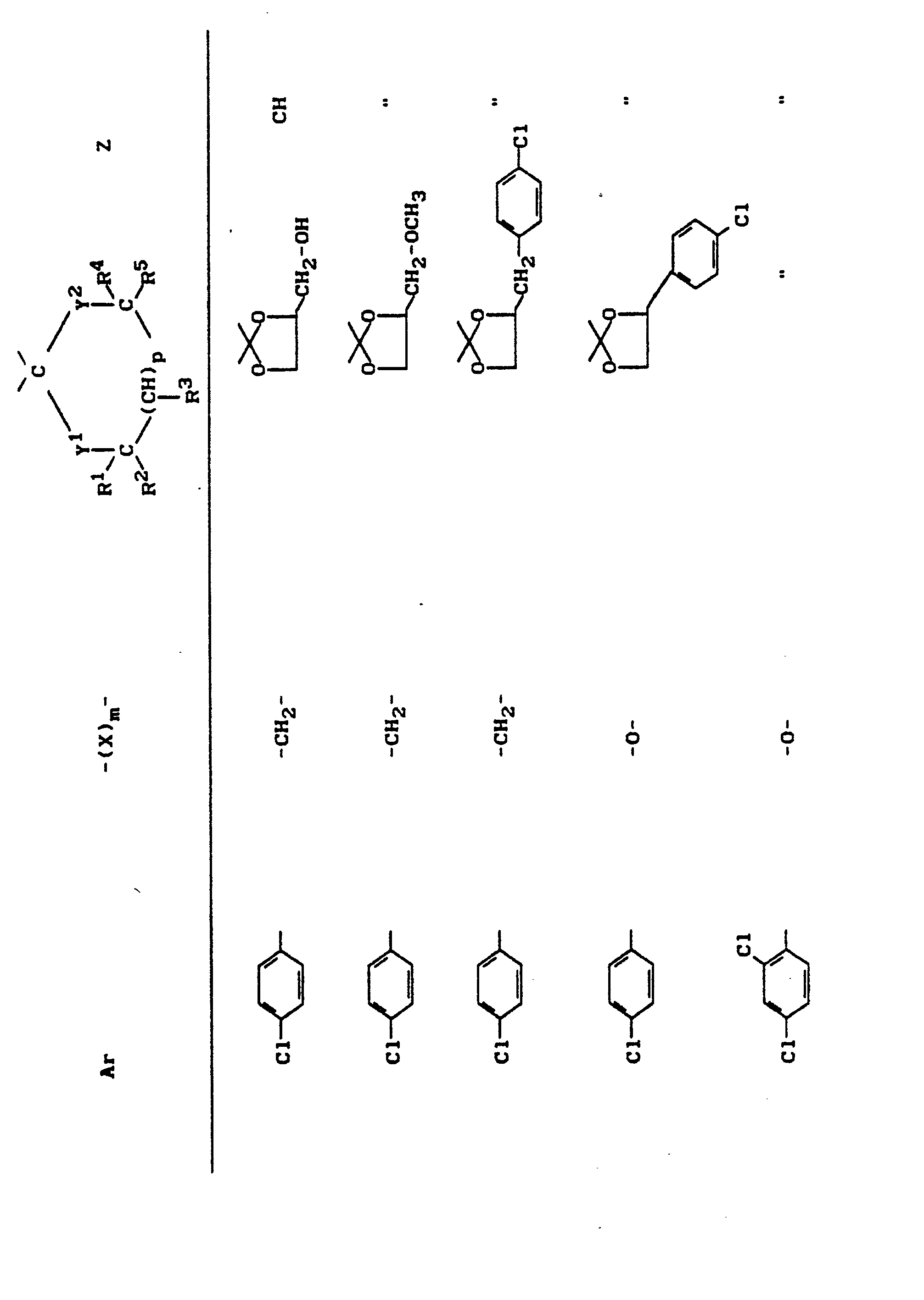

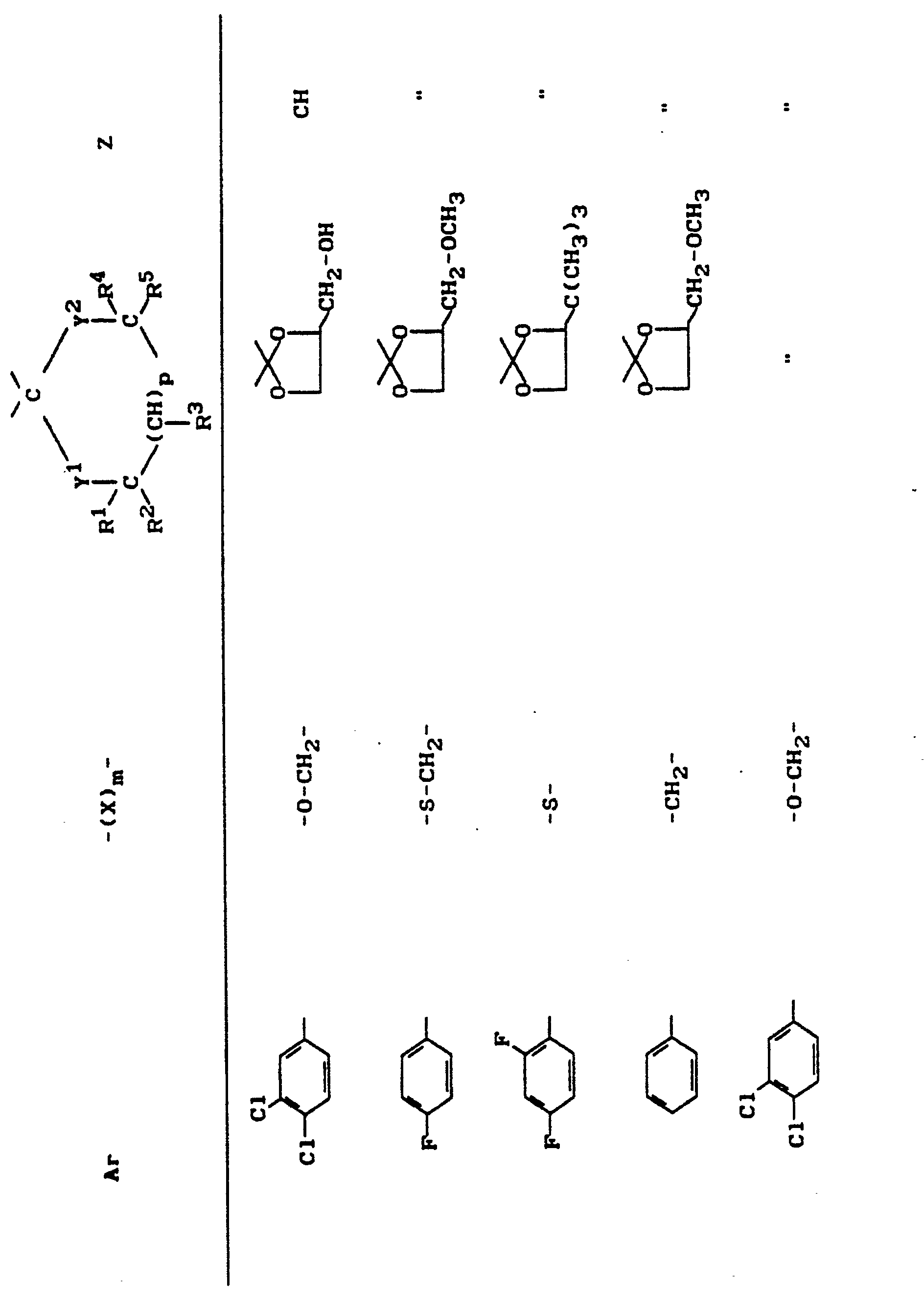

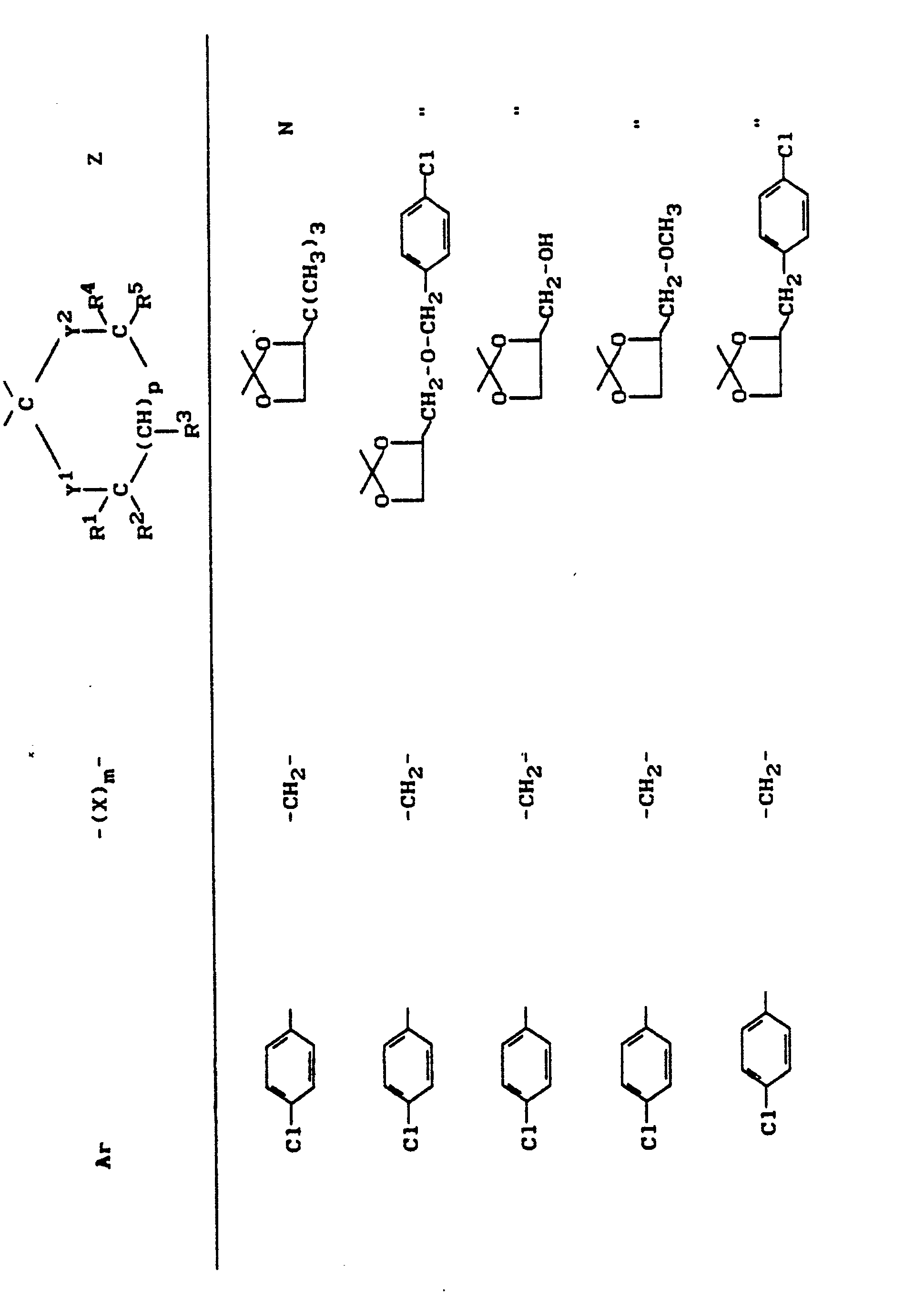

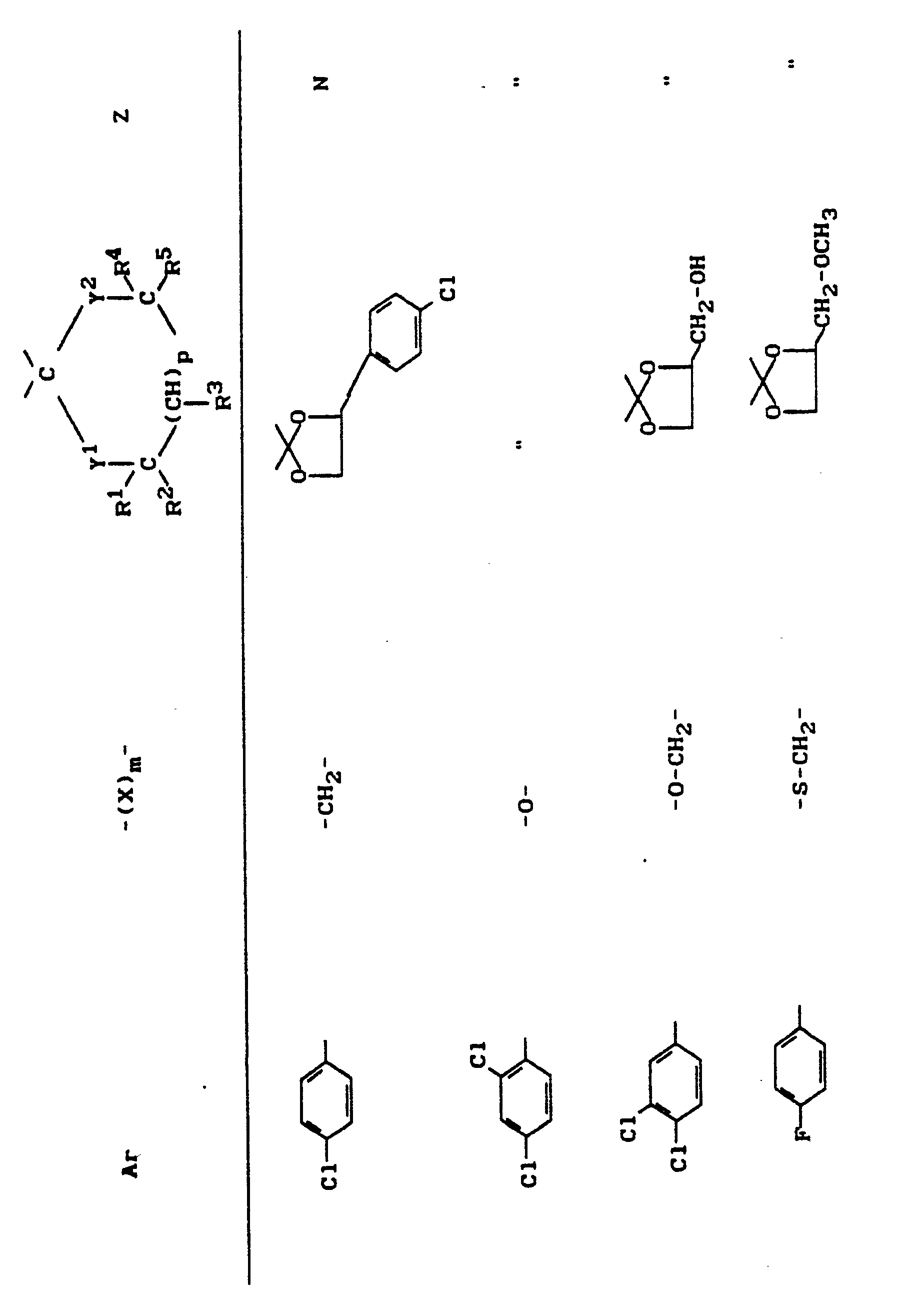

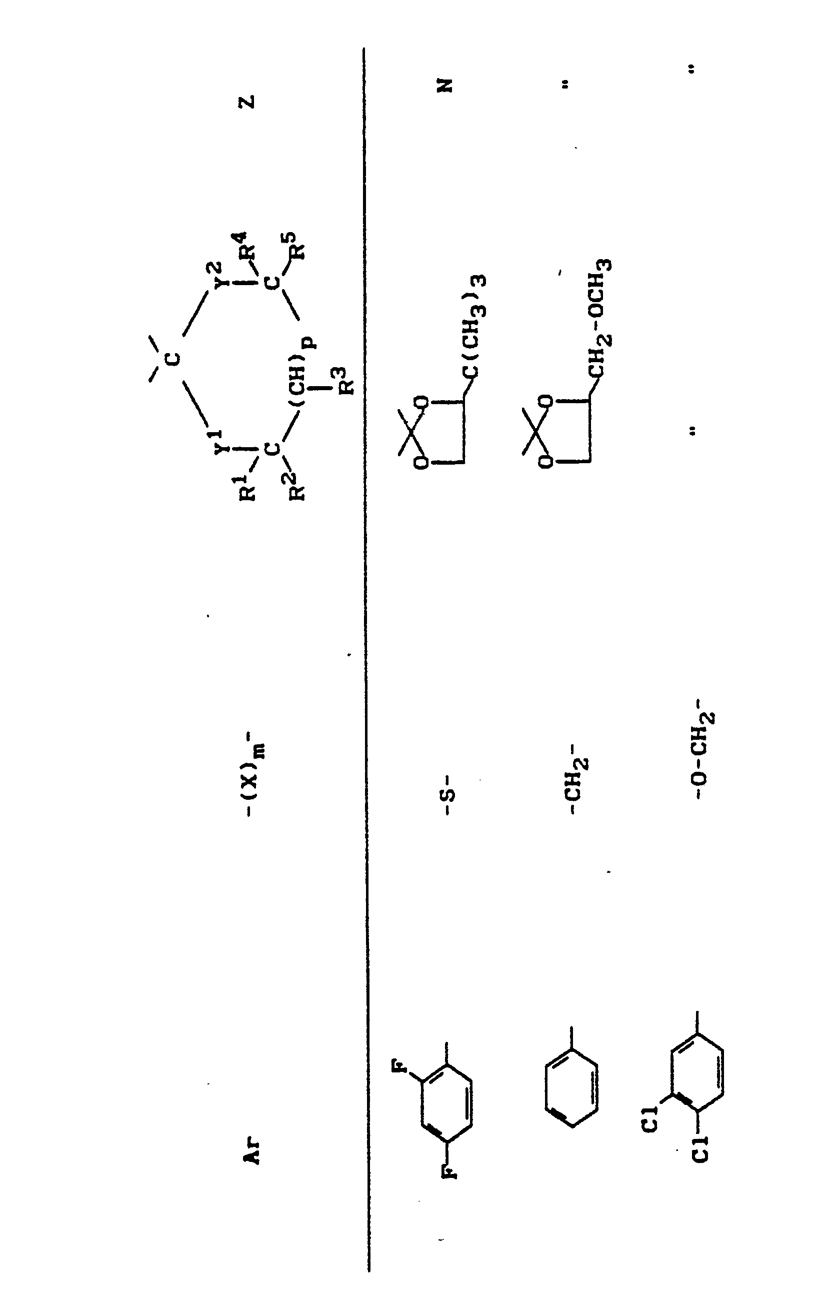

Verwendet man beispielsweise 3-(2,4-Dichlorphenoxy)-2,2-dimethyl-1-(3-pyridyl)-propan-1-on und Ethylenglykol als Ausgangsstoffe, so läßt sich der Reaktionsablauf des erfindungsgemäßen Verfahrens durch das folgende Formelschema darstellen:

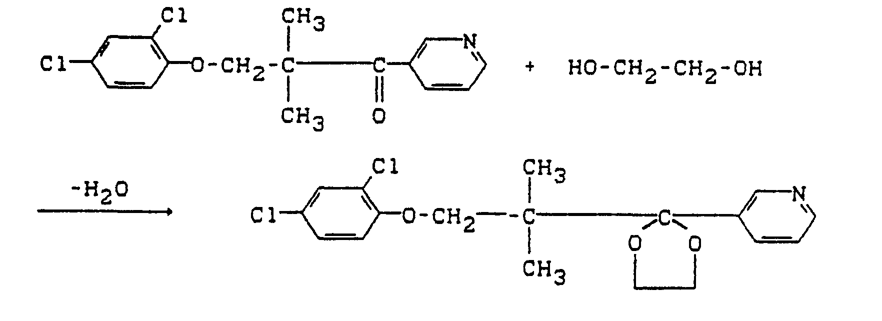

Die zur Durchführung des erfindungsgemäßen Verfahrens als Ausgangsstoffe benötigten Pyri(mi)-dylketone sind durch die Formel (11) allgemein definiert. In dieser Formel (11) stehen Ar, X, Z und m vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Pyri(mi)dylketone der Formel (II) sind bekannt (vergl. z.B. EP 173 190, EP 221 844 oder EP 221 014) oder Gegenstand von eigenen, vorgängigen, noch nicht publizierten Patentanmeldungen (vergl. Deutsche Patentanmeldung P 3 702 962 vom 31.01.1987 oder Deutsche Patentanmeldung P 3 725 968 vom 05.08.1987) und erhältlich in Analogie zu bekannten Verfahren, beispielsweise wenn man Ketone der Formel (IV), 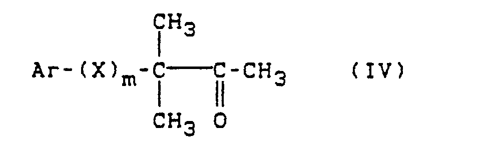 in welcher
Ar, X und m die oben angegebene Bedeutung haben,
zunächst in einer 1. Stufe mit Ameisensäureethylester gegebenenfalls in Gegenwart einer Base, wie beispielsweise Natriumethylat und gegebenenfalls in Gegenwart eines Verdünnungsmittels, wie beispielsweise Diethylether bei Temperaturen zwischen -20° und +60°C umsetzt und dann die so erhältlichen Acylenole der Formel (V) 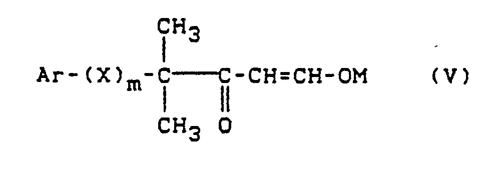 in welcher
M für Wasserstoff oder für ein Alkalimetallkation steht und
Ar, X und m die oben angegebene Bedeutung haben,
nacheinander zunächst mit Formamidin oder einem Säureadditionssalz des Formamidins, wie beispielsweise Formamidinhydroacetat und dann mit Formaldehyddimethylacetal gegebenenfalls in Gegenwart eines Verdünnungsmittels, wie beispielsweise Tetrahydrofuran bei Temperaturen zwischen 0° und 80 C umsetzt; oder wenn man Ester der Formel (VI) 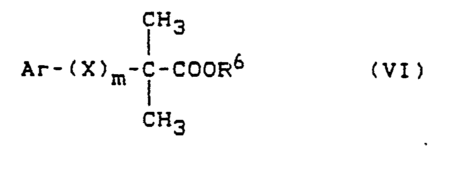
in welcher
R⁶ für Alkyl, insbesondere für Methyl oder Ethyl steht und
Ar, X und m die oben angegebene Bedeutung haben,
mit Pyridyl-Grignard-Verbindungen der Formel (VII) 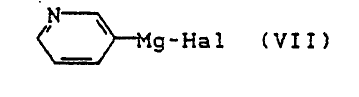 in welcher
Hal für Halogen, insbesondere für Brom steht,
gegebenenfalls in Gegenwart eines Verdünnungsmittels, wie beispielsweise Tetrahydrofuran bei Temperaturen zwischen 0 ° und 80° C umetzt;
oder wenn man Aldehyde der Formel (VIII) 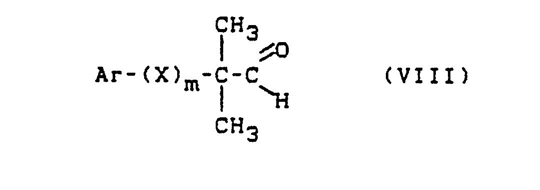 in welcher
Ar, X und m die oben angegebene Bedeutung haben,
zunächst in einer 1. Stufe mit Pyridyl-Grignard-Verbindungen der Formel (VII) 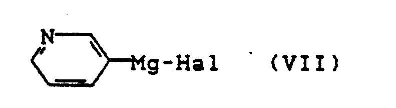 in welcher
Hal für Halogen, insbesondere für Brom steht,
gegebenenfalls in Gegenwart eines Verdünnungsmittels, wie beispielsweise Tetrahydrofuran bei Temperaturen zwischen 0° und 80° C umsetzt und dann die so erhältlichen Pyridylcarbinole der Formel (IX) 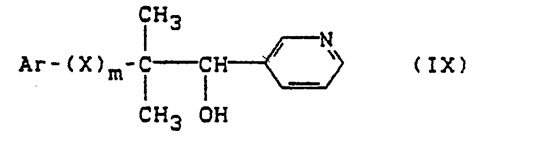 in welcher
Ar, X und m die oben angegebene Bedeutung haben,
mit einem üblichen Oxidationsmittel, wie beispielsweise Chromtrioxid gegebenenfalls in Gegenwart eines Verdünnungsmittels, wie beispielsweise Dichlormethan und gegebenenfalls in Gegenwart einer Base, wie beispielsweise Pyridin sowie gegebenenfalls in Gegenwart eines Reaktionshilfsmittels, wie Trifluoressigsäure bei Temperaturen zwischen 0° und 80' C oxidiert.

Ketone der Formel (IV) sind bekannt (vergl. z.B. DE-OS 3 210 725 oder DE-OS 3 048 266) oder lassen sich in Analogie zu bekannten Verfahren herstellen.

Ester der Formel (VI) sind bekannt (vergl. z.B. EP 221 844 oder Przem. Chem. 59, 495-498 [1980] bzw. CA94:102 975 e) oder lassen sich in Analogie zu bekannten Verfahren herstellen.

Die Aldehyde der Formel (VIII) sind ebenfalls bekannt oder erhältlich in Analogie zu bekannten Verfahren (vgl. z.B. J. Org. Chem. 40, 2282 [1975]; J. Org. Chem. 40, 3079 [1975]; Arm. Khim. Zh. 25, 861 [1972]; Brit. GB 1 140 014; Liebigs Ann. Chem. 1979, 1585; DE 3 531 585; DE 2 056 589).

Die Pyridyl-Grignard-Verbindungen der Formel (VII) sind ebenfalls bekannt (vgl. z.B. EP 221 844 oder Angew. Chem.; Int. Ed. Engl. 8, 279 [1969]).

Es ist auch möglich, die als Reaktionspartner eingesetzten Pyridyl-Grignard-Verbindungen der Formel (VII) aus geeigneten Ausgangsverbindungen, wie beispielsweise 3-Brompyridin und Isopropylmagnesiumbromid, in einer vorgelagerten Reaktion direkt im Reaktionsgefäß herzustellen und ohne Isolierung im Eintopfverfahren mit den Estern der Formel (VI) bzw. mit den Aldehyden der Formel (VIII) weiter umzusetzen.

Die zur Durchführung des erfindungsgemäßen Verfahrens weiterhin als Ausgangsstoffe benötigten Di-(thio)alkohole sind durch die Formel (III) allgemein definiert. In dieser Formel (III) stehen Y¹, Y², R', R², R³, R⁴, R⁵ und p vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Di(thio)alkohole der Formel (III) sind allgemein bekannte Verbindungen der organischen Chemie oder erhältlich in Analogie zu allgemein bekannten Verfahren.

Als Verdünnungsmittel zur Durchführung des erfindungsgemäßen Verfahrens kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether, gegebenenfalls im Gemisch mit höheren Alkoholen, wie beispielsweise Butanol.

Bei Verwendung flüssiger Di(thio)alkohole der Formel (III) als Reaktionskomponente ist es auch möglich, diese in einem entsprechenden Überschuß gleichzeitig als Verdünnungsmittel einzusetzen.

Das erfindungsgemäße Verfahren wird vorzugsweise in Gegenwart eines geeigneten Reaktionshilfsmittels durchgeführt. Als solche kommen vorzugsweise alle üblicherweise verwendbaren anorganischen oder organischen Säuren oder andere übliche Katalysatoren infrage.

Mit besonderem Vorzug verwendet man verdünnte wäßrige oder konzentrierte Mineralsäuren wie Salzsäure, Schwefelsäure oder Phosphorsäure oder organische Sulfonsäuren, wie Methansulfonsäure oder p-Toluolsulfonsäure.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200 ^{*} C, vorzugsweise bei Temperaturen zwischen 40. C und 180 C.

Gegebenenfalls kann bei der Durchführung des erfindungsgemäßen Verfahrens auch unter erhöhtem Druck gearbeitet werden.

Zur Durchführung des erfindungsgemäßen Verfahrens setzt man pro Mol an Pyri(mi)dylketon der Formel (II) im allgemeinen 1,0 bis 10,0 Mol, vorzugsweise 1,0 bis 5,0 Mol an Di(thio)alkohol der Formel (III) und gegebenenfalls 0,01 bis 3,0 Mol, vorzugsweise 0,1 bis 2,0 Mol an Reaktionshilfsmittel ein.

Die Reaktionsdurchführung, Aufarbeitung und Isolierung der Reaktionsprodukte erfolgt nach allgemein üblichen Methoden (vergl. auch die Herstellungsbeispiele);

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallisation reinigen.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide einsetzbar.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infesfans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis), zur Bekämpfung von Reiskrankheiten, wie beispielsweise zur Bekämpfung der Reisfleckenkrankheit (Pyricularia oryzae) oder zur Bekämpfung von Krankheiten im Obst- und Gemüsebau, wie beispielsweise gegen den Erreger des Bohnengrauschimmels (Botrytis cinerea) einsetzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren_{'} Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe, wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

### Herstellungsbeispiele

### Beispiele 1

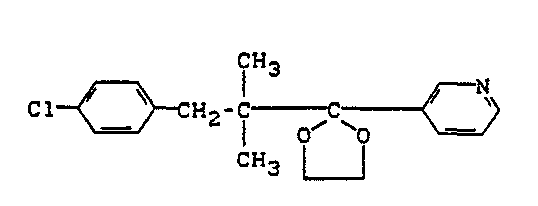

6 g (0,022 Mol) 3-(4-Chlorphenyl)-2,2-dimethyl-1-(3-pyridyl)-propan-1-on, 2,7 g (0,044 Mol) Ethylenglykol und 8,8 g (0,0462 Mol) p-Toluolsulfonsäure werden in einem Gemisch aus 145 ml Xylol und 22 ml Butanol 20 Stunden über einem Wasserabscheider refluxiert. Die abgekühlte Reaktionsmischung wird mit verdünnter wäßriger Natronlauge alkalisch gestellt, die organische Phase abgetrennt, mit Wasser gewaschen, über Natriumsulfat getrocknet, im Vakuum eingeengt, der Rückstand mit Petrolether verrührt, abgesaugt und getrocknet.

Man erhält 4,9 g (70 % der Theorie) an 2-[1-(4-Chlorphenyl)-2-methyl-prop-2-yl]-2-(3-pyridyl)-1,3-dioxolan vom Schmelzpunkt 121 -123` C.

### Herstellung der Ausgangsverbindung

### Beispiel 11-1:

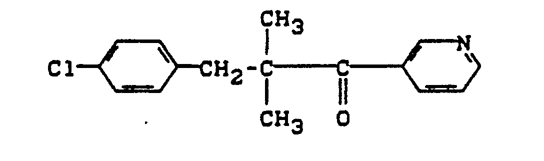

40 g (0,145 Mol) 3-(4-Chlorpenyl)-2,2-dimethyl-1-(3-pyridyl)-propan-1-ol gibt man bei Raumtemperatur in eine Mischung aus 81,8 g (0,218 Mol) Pyridindichromat und 11,2 g (0,058 Mol) Pyridintrifluoracetat in 400 ml Dichlormethan, rührt anschließend 7 Stunden bei Raumtemperatur, trennt die organische Phase ab, wäscht mit Wasser, trocknet über Natriumsulfat, engt im Vakuum ein und reinigt den Rückstand durch Chromatographie an Kieselgel (Laufmittel: Essigester/Cyclohexan 3:1).

Man erhält 28,2 g (71 % der Theorie) an 3-(4-Chlorphenyl)-2,2-dimethyl-1-(3-pyridyl)-propan-1-on als Öl vom Brechungsindex 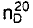 1,5711.

### Beispiel IX-1:

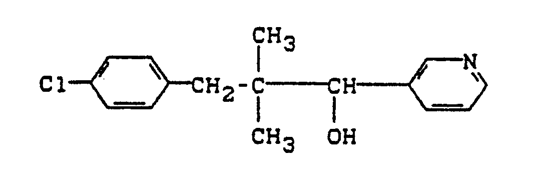

Zu 23,7 g (0,15 Mol) 3-Brompyridin in 115 ml Tetrahydrofuran gibt man bei Raumtemperatur tropfenweise unter Rühren 22,1 g (0,15 Mol) Isopropylmagnesiumbromid in 100 ml Ether, rührt nach beendeter Zugabe weitere 30 Minuten bei Raumtemperatur, gibt dann tropfenweise unter Rühren 22,6 g (0,1 Mol) 3-(4-Chlorphenyl)-2,2-dimethylpropanol (vergl. z.B. GB 1 140 014 oder Europ. J. Med. Chem. Chim. Ther. 16, 495-501 [1981]) in 60 ml Diethylether zu, rührt anschließend weitere 2 Stunden bei Rückflußtempertur, kühlt ab auf Raumtemperatur, gibt 50 ml Wasser zu, stellt mit verdünnter Salzsäure auf pH 5 bis 6 ein, trennt die organische Phase ab, wäscht mit Wasser, trocknet über Natriumsulfat und entfernt das Lösungsmittel im Vakuum.

Man erhält 17 g (62 % der Theorie) an 3-(4-Chlorphenyl)-2,2-dimethyl-1.(3-pyridyl)-propan-1-ol vom Schmelzpunkt 160°C.

### Beispiel 2

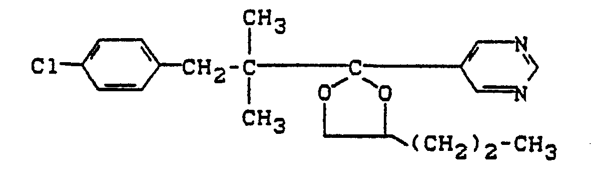

12,5 g (0,046 Mol) 3-(4-Chlorphenyl)-2,2-dimethyl-1-(5-pyrimidinyl)-propan-1-on, 9,5 g (0,091 Mol) 1,2-Pentandiol und 18,4 g (0,097 Mol) p-Toluolsulfonsäure werden in einem Gemisch aus 300 ml Xylol und 46 ml n-Butanol 20 Stunden über einem Wasserabscheider refluxiert. Die abgekühlte Reaktionsmischung wird nacheinander mit verdünnter wäßriger Natronlauge und Wasser gewaschen, über Natriumsulfat getrocknet, im Vakuum eingeengt und durch Chromatographie an Kieselgel (Laufmittel: Essigester/Cyclohexan 3:1) gereinigt.

Man erhält 8,1 g (49 % der Theorie) an 2-[i-(4-Chlorphenyl)-2-methyl-prop-2-yl]-2-(5-pyrimidyl)-4-n-propyl-1,3-dioxolan als Öl vom Brechungsindex 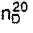 1,5359.

### Herstellung der Ausgangsverbindung:

### Beispiel 11-2:

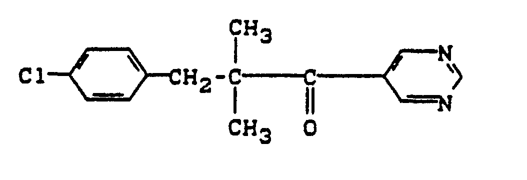

Zu 165,8 g (0,636 Mol) 5-(4-Chlorphenyl)-4,4-dimethylpent-1-en-1-ol-3-on Natriumsalz in 1000 ml Tetrahydrofuran gibt man bei 0°C 83,2 g (0,799 Mol) Formamidinhydroacetat, rührt 15 Minuten bei 0°C und gibt dann tropfenweise unter Rühren ebenfalls bei 0°C 103,1 g (0,866 Mol) Dimethylformamiddimethylacetal, rührt nach beendeter Zugabe 6 Stunden bei 50 bis 55 C, filtriert, engt das Filtrat im Vakuum ein, gibt den Rückstand in Wasser, extrahiert mit Dichlormethan, trocknet über Natriumsulfat und engt im Vakuum ein. Der Rückstand wird durch Chromatographie an Kieselgel (Laufmittel: Essigester/Cyclohexan 3:1) gereinigt.

Man erhält 30,2 g (17,2 % der Theorie) an 3-(4-Chlorphenyl)-2,2-dimethyl-1-(5-pyrimidinyl)-propan-1-on vom Schmelzpunkt 65 ° C.

### Beispiel V-1:

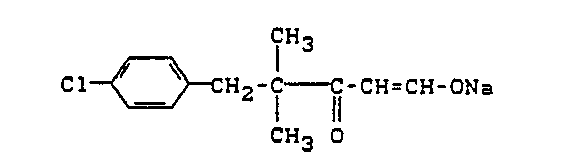

Zu 200 g (0,95 Mol) 4-(4-Chlorphenyl)-3,3-dimethylbutan-2-on (vergl. z.B. DE 3 220 730 oder DE 3 210 725) und 51,3 g (0,95 Mol) Natriummethylat in 900 ml Diethylether gibt man bei Raumtemperatur tropfenweise unter Rühren 70,3 g (0,95 Mol) Ameisensäureethylester, rührt nach beendeter Zugabe 3 Stunden bei Rückflußtemperatur, engt im Vakuum ein, nimmt den Rückstand in Wasser auf, extrahiert mit Essigester, bringt dann die wäßrige Phase mit verdünnter Salzsäure auf pH 1, extrahiert erneut mit Essigester, trocknet über Natriumsulfat, engt im Vakuum ein, nimmt den Rückstand im Methanol auf, gibt 36 g (0,666 Mol) Natriummethylat zu und entfernt das Lösungsmittel im Vakuum.

Man erhält 173,7 g (70 % der Theorie) an 5-(4-Chlorphenyl)-4,4-dimethyl-pent-1-en-1-ol-3-on Natriumsalz, welches ohne zusätzliche Reinigung sofort weiter umgesetzt wird.

In entsprechender Weise und gemäß den allgemeinen Angaben zur Herstellung erhält man die folgenden substituierten Pyri(mi)dine der allgemeinen Formel (I):

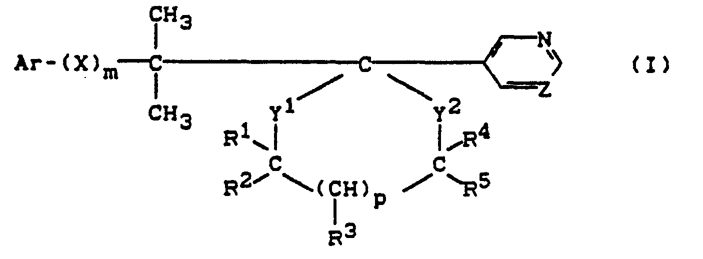

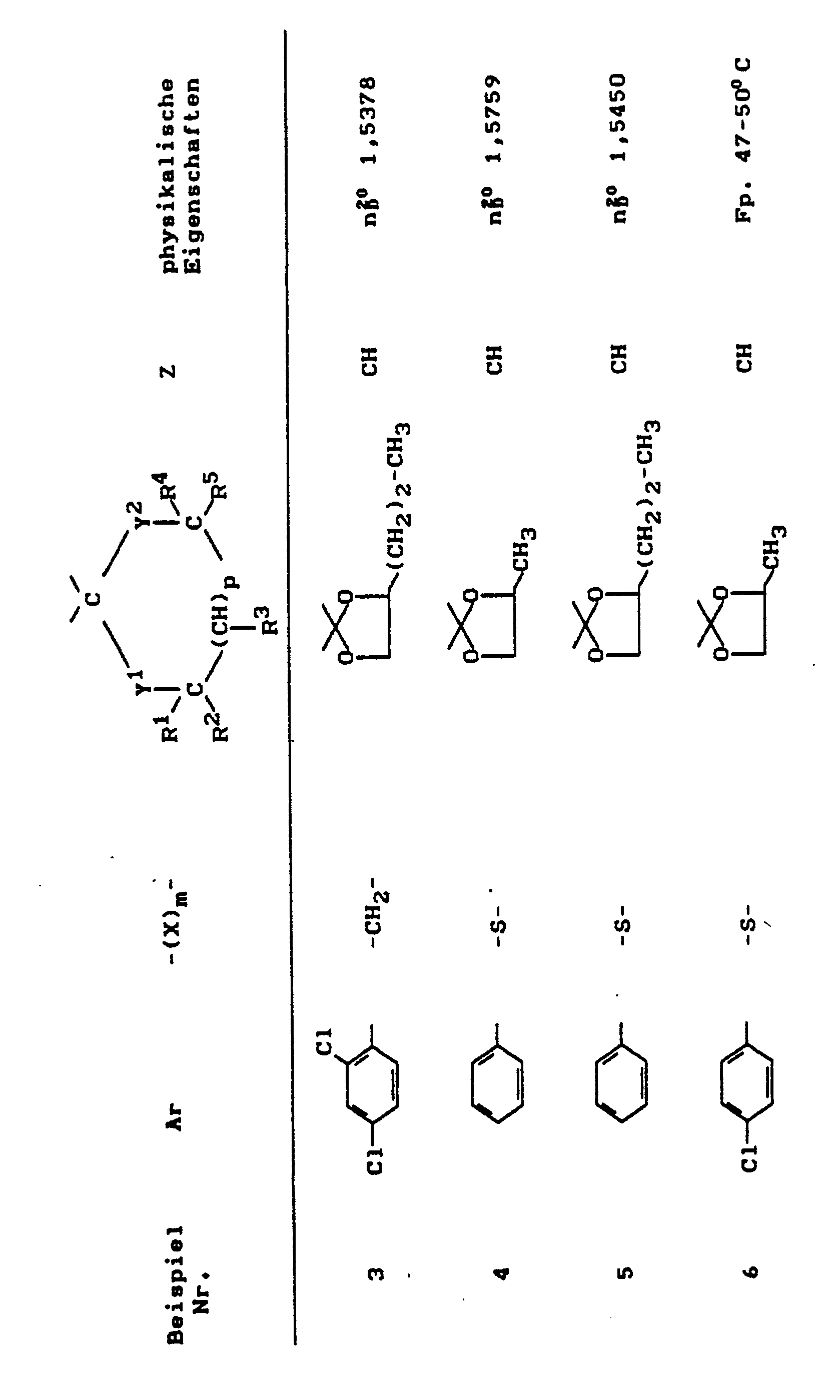

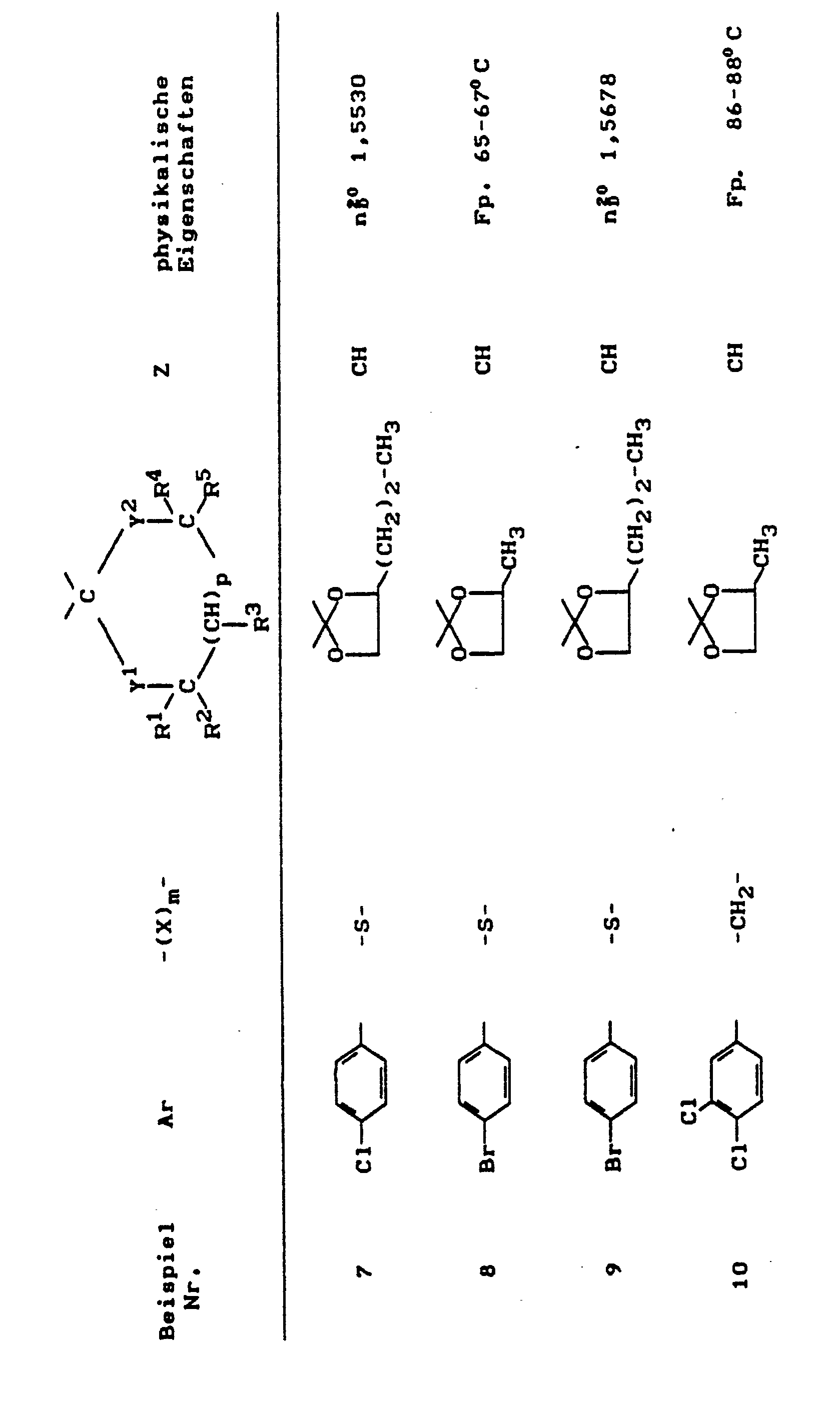

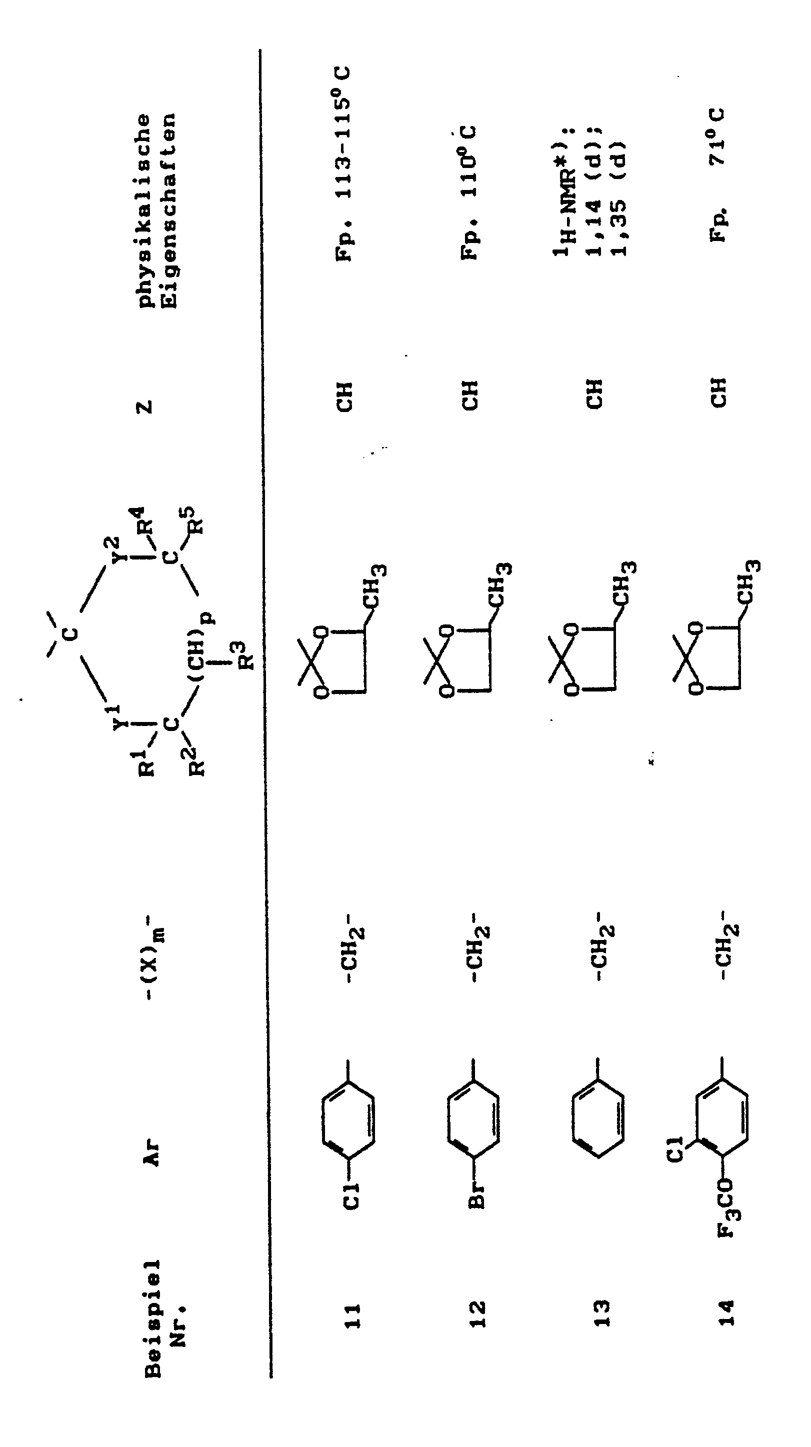

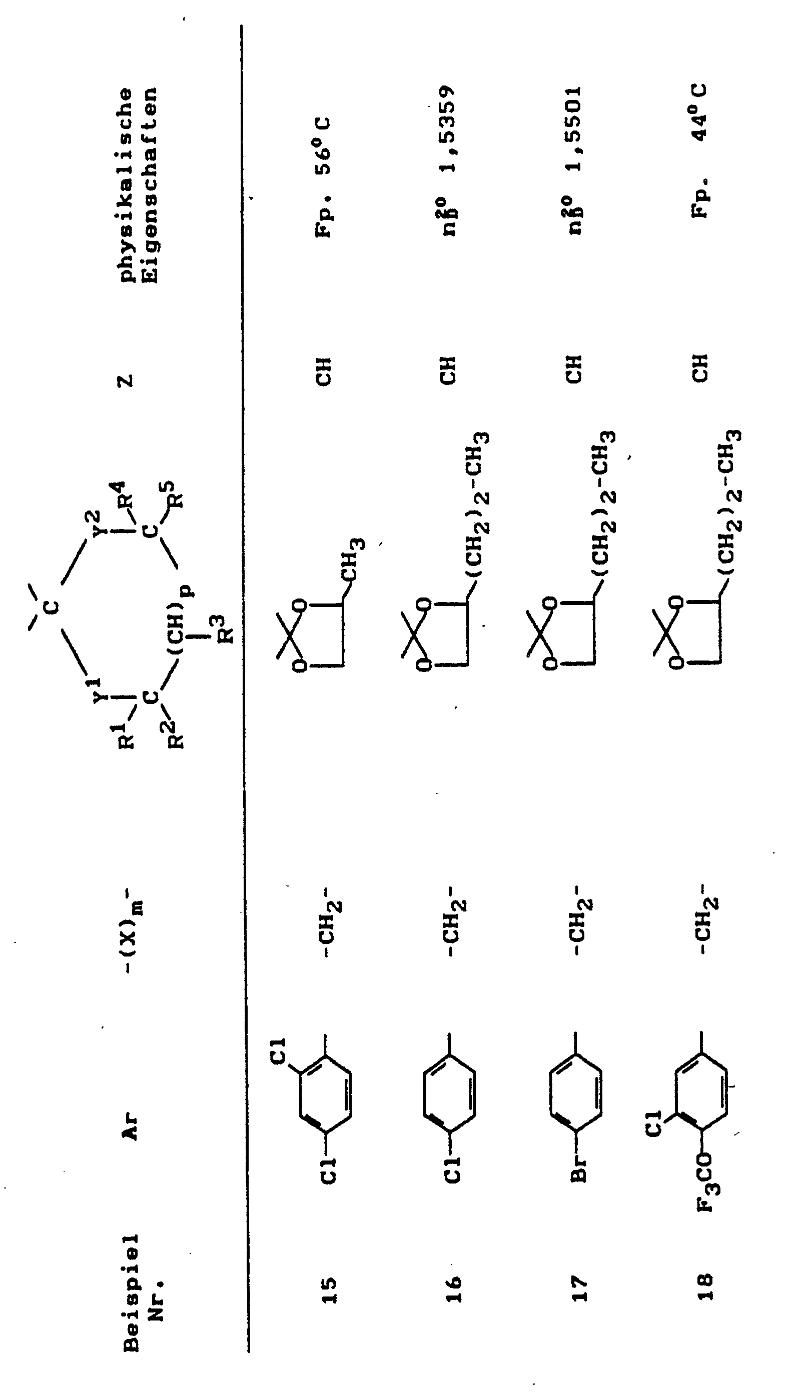

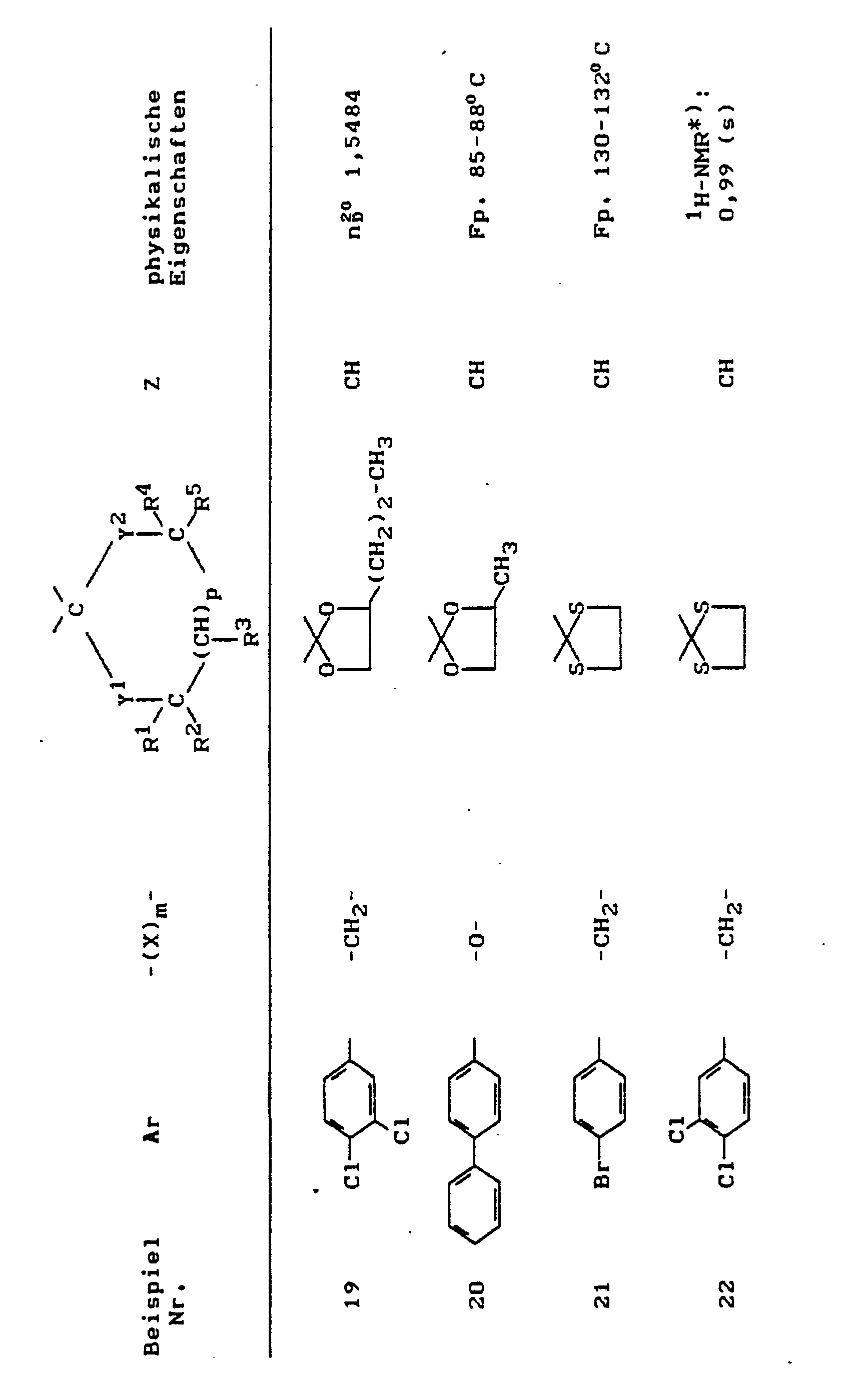

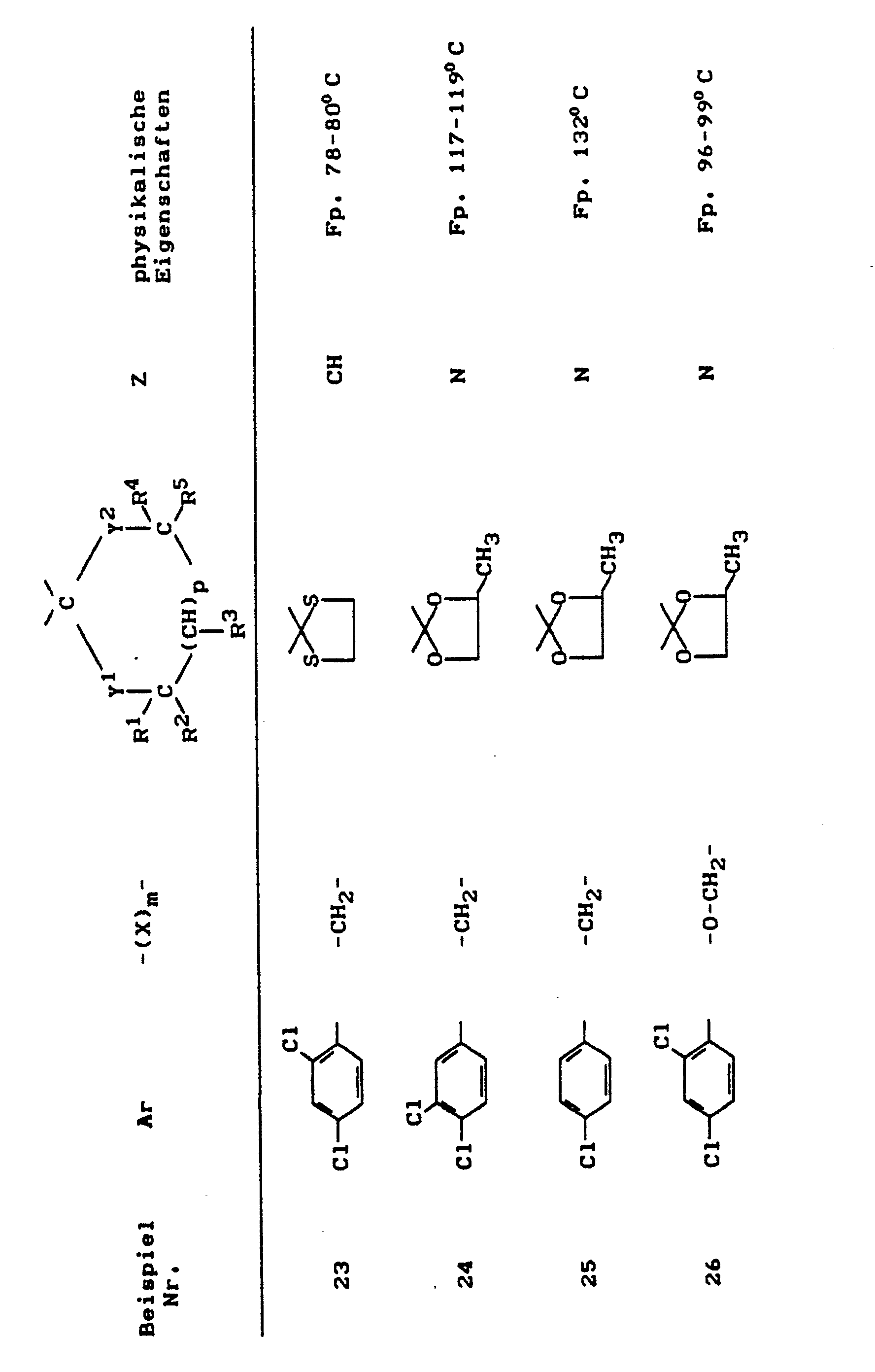

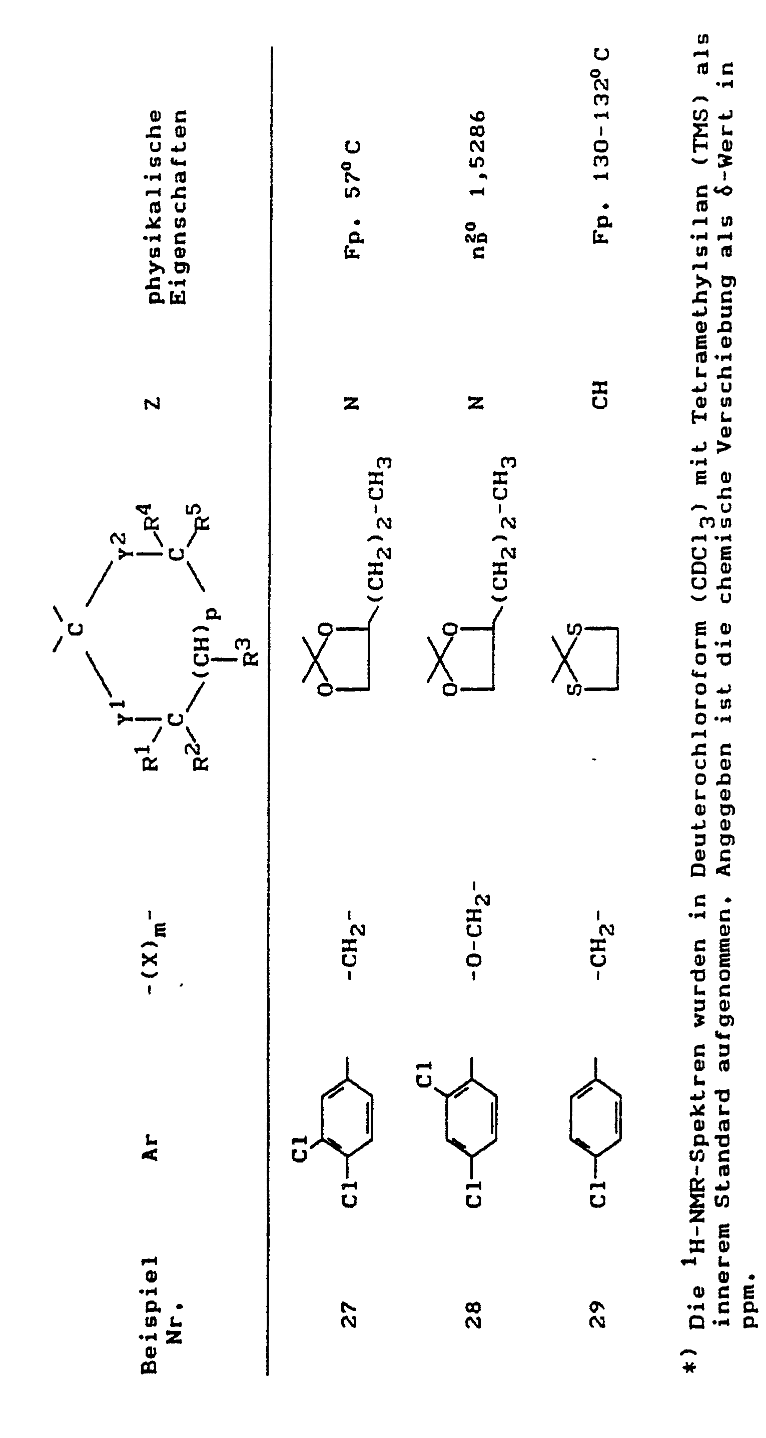

### Anwendungsbeispiele

In den folgenden Anwendungsbeispielen wurden die nachstehend aufgeführten Verbindungen als Vergleichsubstanzen eingesetzt: 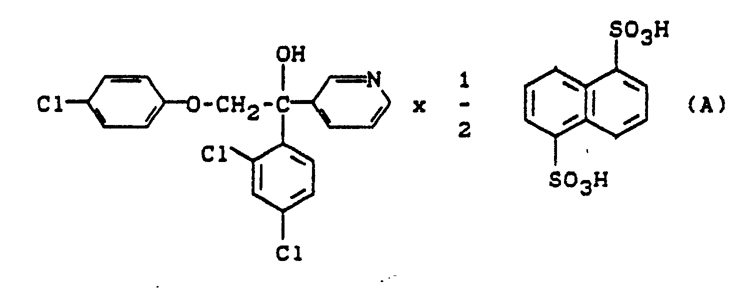 2-(4-Chlorphenoxy)-1-(2,4-dichlorphenyl)-1-(3-pyridyl)-ethanol Naphthalindisulfonat 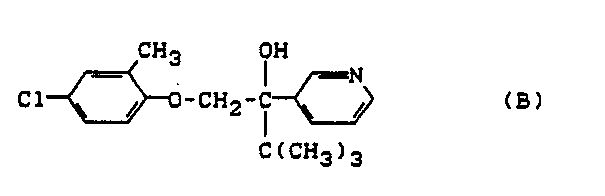 1-(4-Chlor-2-methylphenoxy)-3,3-dimethyl-2-(3-pyridyl)-2-butanol 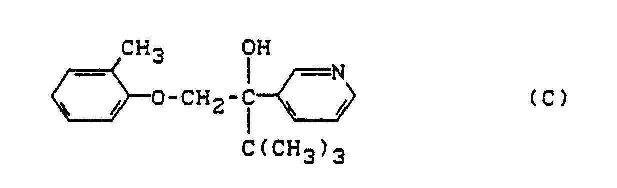 3,3-Dimethyl-1-(2-methylphenoxy)-2-(3-pyridyl)-2-butanol 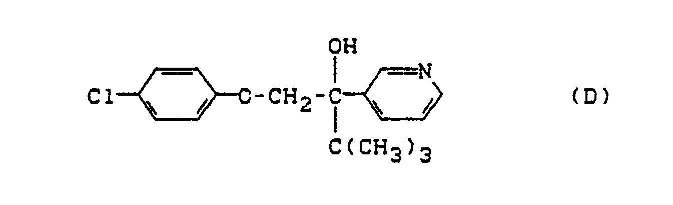 1-(4-Chlorphenoxy)-3,3-dimethyl-2-(3-pyridyl)-2-butanol 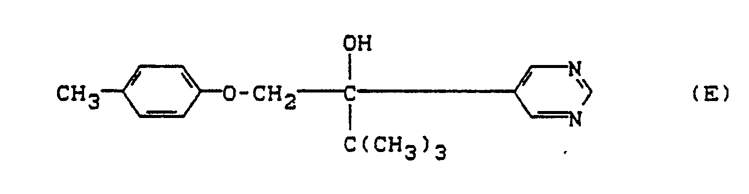 1-(4-Methylphenoxy)-3,3-dimethyl-2-(5-pyrimidinyl)-2-butanol 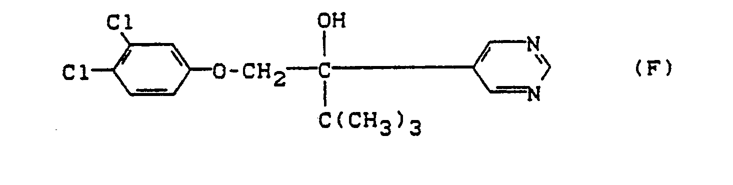 1-(3,4-Dichlorphenoxy)-3,3-dimet.hyl-2-(5-pyrimidinyl)-2-butanol
(afle bekannte aus EP 1399).

### Beispiel A

Botrytis-Test (Bohne)/protektiv

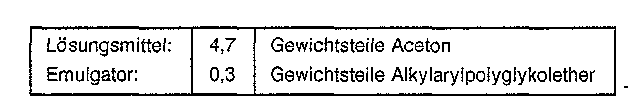

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß den Herstellungsbeispielen: 11, 12 und 13.

### Beispiel B

Erysiphe-Test (Gerste) / protektiv

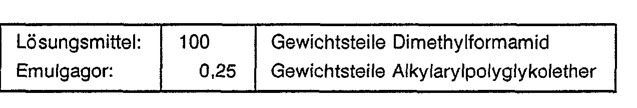

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20. C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 9, 10, 11, 12, 14 und 15.

### Beispiel C

Pyricularia-Test (Reis) /protektiv

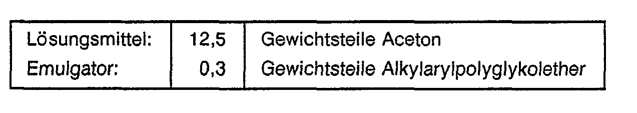

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 11, 14, 15, 16, 18, 24 und 27.

## Patentansprüche

1. Substituierte Pyri(mi)dine der allgemeinen Formel (I) 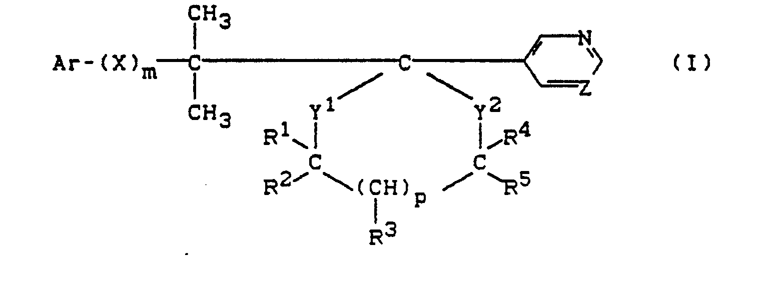 in welcher
Ar für gegebenenfalls substituiertes Aryl steht,
X für Sauerstoff, Schwefel, Sulfinyl, Sulfonyl oder für eine der Gruppen -CH₂-; -O-CH₂-; -CH₂-0-; -O-CH₂-
CH₂-; -S(O)ₙ-CH₂-; -CH₂-S(O)ₙ-oder -S(O)ₙ₋CH₂-CH₂- steht,
Y' und Y² unabhängig voneinander jeweils für Sauerstoff oder Schwefel stehen,
Z für Stickstoff oder eine CH-Gruppe steht,
R', R², R³ und R⁴ unabhängig voneinander jeweils für Wasserstoff oder Alkyl stehen oder
R' und R⁴ gemeinsam für einem zweifach verknüpften Alkandiylrest stehen,
R⁵ für Wasserstoff oder für jeweils gegebenenfalls substituiertes Alkyl oder Aryl steht,
m für eine Zahl 0 oder 1 steht,
n jeweils für eine Zahl 0, 1 oder 2 steht und
p für eine Zahl 0 oder 1 steht,
sowie deren Säureadditionssalze und Metallsalzkömplexe.

2. Substituierte Pyri(mi)dine gemäß Anspruch 1, wobei in der Formel (I)
Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, geradkettiges oder verzweigtes, zweifach verknüpftes Alkandiyl mit 1 bis 10 Kohlenstoffatomen, Cycloalkyl mit 3 bis 7 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen mit 1 bis 6 Kohlenstoffatomen oder jeweils gegebenenfalls einfach bis mehrfach, gleich oder verschieden durch Halogen substituiertes Phenyl oder Phenoxy,
oder für gegebenenfalls einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;
X für Sauerstoff, Schwefel, Sulfinyl, Sulfonyl oder für eine der Gruppen -CH₂-; -O-CH₂-; -CH₂-0-; -O-CH₂-CH₂-; -S(O)ₙ-CH₂-; -CH₂-S(O)ₙ-oder -S(O)ₙ-CH₂-CH₂- steht,
wobei
n jeweils für eine Zahl 0, 1 oder 2 steht,
Y' und Y² unabhängig voneinander jeweils für Sauerstoff oder Schwefel stehen,
Z für Stickstoff oder eine CH-Gruppe steht,
R', R², R³ und R⁴ unabhängig voneinander jeweils für Wasserstoff oder für geradkettiges oder verzweigtes Alkyl mit 1 bis 6 Kohlenstoffatomen stehen oder
R' und R⁴ gemeinsam für einen geradkettigen oder verzweigten Alkandiylrest mit 2 bis 10 Kohlenstoffatomen stehen,
R⁵ für Wasserstoff, für jeweils geradkettiges oder verzweigtes Alkyl, Hydroxyalkyl, Alkoxyalkyl, Alkylcarbonyloxyalkyl, Alkylsulfonyloxyalkyl, Dialkylaminoalkyl oder N, N-Dialkylcarbamoylalkyl mit jeweils 1 bis 4 Kohlenstoffatomen in den einzelnen Alkylteilen steht; außerdem für jeweils im Arylteil gegebenenfalls einfach oder mehrfach, gleich oder verschieden substituiertes Aralkyloxyalkyl, Aryloxyalkyl, Aralkylcarbonyloxyalkyl, Arylcarbonyloxyalkyl, Arylsulfonyloxyalkyl, oder Aralkyl mit jeweils 1 bis 4 Kohlenstoffatomen in den einzelnen Alkylteilen und jeweils 6 bis 10 Kohlenstoffatomen im Arylteil steht oder für gegebenenfalls einfach oder mehrfach, gleich oder verschieden substituiertes Aryl mit 6 bis 10 Kohlenstoffatomen steht, wobei als Arylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino, Alkanoylamino oder N-Alkyl-alkanoylamino mit jeweils 1 bis 4 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 4 Kohlenstoffatomen substituiertes Phenyl und gegebenenfalls durch jeweils geradkettiges oder verzweigtes Alkyl oder Alkanoyl mit jeweils 1 bis 4 Kohlenstoffatomen substituiertes 1,4-Piperazinyl,
m für eine Zahl 0 oder 1 steht und
p für eine Zahl 0 oder 1 steht.

3. Substituierte Pyri(mi)dine gemäß Anspruch 1, wobei in der Formel (I)
Ar für gegebenenfalls einfach bis dreifach, verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethylbutan-1,4-diyl, Tetramethylbutan-1,4-diyl, Cyclopropyl, Cyclobutyl, Cyclopentyl, Cyclohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils gegebenenfalls ein- bis dreifach durch Fluor oder Chlor substituiertes Phenyl oder Phenoxy;
oder für gegebenenfalls ein- bis dreifach, gleich oder verschieden substituiertes a-Naphthyl oder β-Naphthyl steht, wobei als Substituenten jeweils infrage kommen: Fluor, Chlor, Brom, Cyano, Methyl, Ethyl,
n- oder i-Propyl;
X für Sauerstoff, Schwefel, Sulfinyl, Sulfonyl oder für eine der Gruppen -CH₂-; -O-CH₂-; -CH₂-0-; -O-CHε-
CH₂-; -S(O)ₙ-CH₂-; -CH₂-S(O)_{"-} oder -S(O)_{"}-CH₂-CH₂- steht,
wobei
n jeweils für eine Zahl 0, 1 oder 2 steht,
Y¹ und Y² unabhängig voneinander jeweils für Sauerstoff oder Schwefel stehen,
Z für Stickstoff oder eine CH-Gruppe steht,
R', R², R³ und R⁴ unabhängig voneinander jeweils für Wasserstoff, Methyl oder Ethyl stehen oder
R¹ und R⁴ gemeinsam für einen zweifach verknüpften Butan-1,4-diylrest stehen,
R⁵ für Wasserstoff, Methyl, Ethyl, n- oder i-Propyl, n-, i-, s- oder t-Butyl, für Hydroxymethyl, Hydroxyethyl,
Methoxymethyl, Methoxyethyl, Ethoxymethyl, Ethoxyethyl, n-oder i-Propoxymethyl, Methylcarbonyloxymethyl, Ethylcarbonyloxymethyl, Methylsulfonyloxymethyl, Dimethylaminomethyl, Diethylaminomethyl, Dimethylaminoethyl, Diethylaminoethyl, Dimethylaminocarbamoylmethyl, Diethylaminocarbamoylmethyl und au-
ßerdem für jeweils gegebenenfalls im Phenylteil ein- bis dreifach, gleich oder verschieden substituiertes Benzyloxymethyl, Phenoxymethyl, Phenoxyethyl, Benzylcarbonyloxymethyl, Benzyloxymethyl, Phenylsulfonyloxymethyl, Benzyl, Phenylethyl oder Phenyl steht, wobei als Phenylsubstituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n-oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl, Dimethylamino, Diethylamino, Acetamido, N-Methylacetamido, gegebenenfalls ein- bis dreifach, gleich oder verschieden durch Fluor, Chlor, Brom, Methyl und/oder Ethyl substituiertes Phenyl oder gegebenenfalls durch Methyl oder Acetyl substituiertes 1,4-Piperazinyl,
m für eine Zahl 0 oder 1 steht und
p für eine Zahl 0 oder 1 steht.

4. Substituierte Pyri(mi)dine gemäß Anspruch 1, wobei in der Formel (I)
Ar für gegebenenfalls einfach oder zweifach, gleich oder verschieden substituiertes Phenyl steht, wobei als Substituenten infrage kommen: Fluor, Chlor, Brom, Cyano, Nitro, Methyl, Ethyl, i-Propyl, t-Butyl, Methoxy, Methylthio, Trifluormethyl, Difluormethyl, Trifluormethoxy, Difluormethoxy, Trifluormethylthio oder jeweils gegebenenfalls einfach oder zweifach durch Fluor oder Chlor substituiertes Phenyl oder Phenoxy; und außerdem für a-Naphthyl oder ß-Naphthyl steht,
X für Sauerstoff, Schwefel oder für eine der Gruppen -CH₂-, -O-CHz- oder -S-CH₂- steht,
Y' und Y² unabhängig voneinander jeweils für Sauerstoff oder Schwefel stehen,
Z für Stickstoff oder für eine CH-Gruppe steht,
R', R², R³ und R⁴ jeweils für Wasserstoff stehen oder
R¹ und R⁴ gemeinsam für einen zweifach verknüpften Butan-1,4-diylrest stehen,
R⁵ für Wasserstoff, Methyl, Ethyl, n- oder i-Propyl, n-, i-, s- oder t-Butyl, Hydroxymethyl, Methoxymethyl oder für jeweils gegebenenfalls einfach oder zweifach, gleich oder verschieden durch Fluor, Chlor oder Methyl substituiertes Phenoxymethyl, Benzyloxymethyl, Benzyl oder Phenyl steht,
m für eine Zahl 0 oder 1 steht und
p für eine Zahl 0 oder 1 steht.

5. Verfahren zur Herstellung substituierter Pyri(mi)dine der Formel (I) 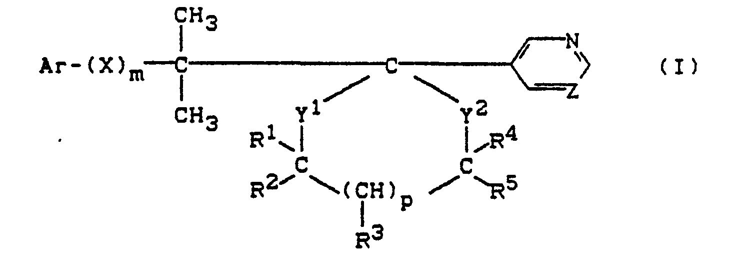 in welcher
Ar für gegebenenfalls substituiertes Aryl steht,
X für Sauerstoff, Schwefel, Sulfinyl, Sulfonyl oder für eine der Gruppen -CH₂-; -O-CH₂-; -CH₂-0-; -O-CH₂-
CH₂-; -S(O)ₙ-CH₂-; -CH₂-S(O)ₙ-oder -S(O)ₙ-CH₂-CH₂- steht,
Y' und Y² unabhängig voneinander jeweils für Sauerstoff oder Schwefel stehen,
Z für Stickstoff oder eine CH-Gruppe steht,
R¹, R², R³ und R⁴ unabhängig voneinander jeweils für Wasserstoff oder Alkyl stehen oder
R¹ und R⁴ gemeinsam für einen zweifach verknüpften Alkandiylrest stehen,
R⁵ für Wasserstoff oder für jeweils gegebenenfalls substituiertes Alkyl oder Aryl steht,
m für eine Zahl 0 oder 1 steht,
n jeweils für eine Zahl 0, 1 oder 2 steht und p für eine Zahl 0 oder 1 steht,
sowie deren Säureadditionssalze und Metallsalzkomplexe dadurch gekennzeichnet, daß man Pyri(mi)-dylketone der Formel (II) 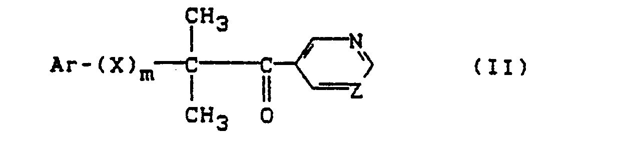 in welcher
Ar, X, Z und m die oben angegebene Bedeutung haben,

mit Di(thio)alkoholen der Formel (III) 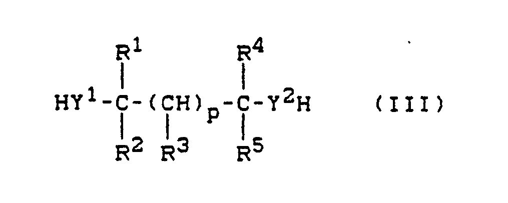 in welcher
_{Y}', _{Y}², R¹, R², R³, R⁴, R⁵ und p die oben angegebene Bedeutung haben,
gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

6. Schädlingsbekämpfungsmittel, gekennzeichnet durch einen Gehalt an mindestens einem substituierten Pyri(mi)din der Formel (I) nach Ansprüchen 1 oder 5.

7. Verwendung von substituierten Pyri(mi)dinen der Formel (I) nach den Ansprüchen 1 oder 5 zur Bekämpfung von Schädlingen.

8. Verfahren zur Bekämpfung von Schädlingen, dadurch gekennzeichnet, daß man substituierte Pyri-(mi)dine der Formel (I) nach den Ansprüchen 1 oder 5 auf Schädlinge und/oder ihren Lebensraum einwirken läßt.

9. Verfahren zur Herstellung von Schädlingsbekämpfungsmittel, dadurch gekennzeichnet, daß man substituierte Pyri(mi)dine der Formel (I) nach den Ansprüchen 1 oder 5 mit Streckmitteln und/oder oberflächenaktiven Mitteln vermischt.
